# EUROPEAN PATENT APPLICATION

(11) **EP 4 174 074 A1**
(43) Date of publication of application: **03.05.2023**
(21) Application number: 21382980.7
(22) Date of filing: 29.10.2021
(51) Int. Cl.: C07D 498/04, A61P 37/02, A61K 31/4355, C07H 15/06, C07H 15/26

(54) **MULTIANTENNARY GLYCOLIPID MIMETICS**

(71) Applicant: Consejo Superior de Investigaciones Científicas (CSIC), 41013 Sevilla (ES); Universidad de Sevilla, 41013 Sevilla (ES); Academia Sinica, Taipei 11529 (TW)
(72) Inventor: García Fernández, José Manuel, 41013 Sevilla (ES); Ortiz Mellet, Carmen, 41013 Sevilla (ES); González Cuesta, Manuel, 41013 Sevilla (ES); Herrera González, Irene, 41013 Sevilla (ES); Chang, Ya-Jen, 115 Taiwan (TW); Lai, Alan Chuan-Ying, 115 Taiwan (TW)
(74) Representative: Pons

(57) **Abstract**

The invention relates to a multiantennary glycolipid mimetics of formula I, wherein X, Y, Z and R¹ are defined in the description, or a pharmaceutical composition thereof and their use as a medicament, particularly, for the treatment and/or prevention of an immune disease cursed by Th1/Th2 imbalance. Further the invention relates to a vaccine which comprises a compound of formula I.

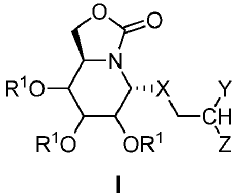

## Description

The invention relates to a multiantennary glycolipid mimetic of formula I or a pharmaceutical composition thereof and their use as a medicament, particularly, for the treatment and/or prevention of an immune disease cursed by Th1/Th2 imbalance. Further the invention relates to a vaccine which comprises a compound of formula I.

### BACKGROUND ART

Carbohydrates play crucial roles in the immune system function and the regulation of the immune response. Not surprisingly, a variety of natural carbohydrate-based immunomodulators have been clinically evaluated for applications ranging from cancer immunotherapy or vaccine adjuvants to the treatment of autoimmune diseases or infections. However, immunoactive carbohydrates obtained from natural sources are usually difficult to obtain in sufficient quantity, purity and homogeneity. Synthetic organic chemistry, including total synthesis and semi-synthetic strategies, offers a more attractive approach to molecularly defined, improved versions of carbohydratecontaining immunostimulants or immunosuppressors, enabling structural modification of the corresponding natural products with a high level of chemical control. The following selected review articles illustrate the importance of carbohydrate derivatives, especially glycolipids, as immunomodulators: Molecules 2013, 18, 15662-15688; Clinical & Translational Immunology 2016, 5, e69; Chemistry A European Journal 2017, 23, 1728-1742; Nature Reviews Chemistry 2021, doi: doi.org/10.1038/s41570-020-00244-3

Among synthetic carbohydrate immunomodulators, glycolipids with agonist or antagonist activity for human invariant natural killer T cells (iNKT cells) define a family of candidates with high promise for clinical development. Natural killer T cells (NKT cells) were originally defined as T cells that constitutively expressed NK-associated receptors in naïve mice. Subsequent classification of subsets with these general properties has defined a major population known as type 1 or invariant NKT cells (iNKT cells), that express an invariant TCRα chain (Va14Ja18 in mouse, Va24Ja18 in human) which is paired with TCRβ chains of limited diversity. iNKT cells recognize lipids and glycolipids presented by the conserved non-polymorphic MHC class I-like molecule, CD1d, and recognize natural self or microbial glycolipids as well as a range of synthetic glycosylceramides.

The prototypic synthetic iNKT cell antigen is a synthetic α-galactosylceramide (αGalCer) known as KRN7000, which contains a C₁₈ phytosphingosine linked with a saturated C₂₆ N-acyl chain. αGalCer has been extensively studied as a model antigen for iNKT cells in humans, as well as mice and other animal models, including rats and nonhuman primates. Upon stimulation with KRN7000, iNKT cells exert multiple immuno-regulatory functions due in part to their rapid secretion of a wide range of cytokines. iNKT cells have a striking capacity to concurrently produce cytokines that are classically associated with both T helper 1 (Th1) responses (e.g., IFNγ, TNFα) and T helper 2 (Th2) responses (IL-4, IL-5, IL-13). Furthermore, their activation leads to induction of dendritic cell (DC) maturation, transactivation of NK cells and help to B cells. Depending on the disease model considered, KRN7000-stimulated iNKT cells have shown an ability to modulate or improve immune responses in the context of tumors, microbial, allergic and autoimmune diseases.

Although αGalCer is the standard model iNKT cell antigen, it bears limitations that hinder its therapeutic effectiveness. A first major issue is the tendency for KRN7000 to elicit high levels of both Th1 and Th2 associated cytokines, which may have directly conflicting activities leading to ineffective and unpredictable immune responses. Moreover, αGalCer can stimulate iNKT cells too potently causing a cytokine storm; that is, iNKT cells release a massive amount of cytokines leading to iNKT cell anergy or inactivation of iNKT cells. Because of the therapeutic potential of iNKT cells and the limitations of αGalCer, there have been intensive efforts to identify structural analogs that have the ability to selectively stimulate a limited range of iNKT cell functions. The following selected review articles provide a general view of this topic: Cell Chemical Biology 2018, 25, 571-584; Organic & Biomolecular Chemistry 2011, 9, 3080-3104. The following selected patent documents collect specific examples on the synthesis and applications of αGalCer analogs as immunomodulators: US 5,936,076; US 7928,077 B2; WO 2007/050668 A1; US 8,580,751 B2; US 2011/0028411 A1, US 8,883,746 B2, WO 2019/102054 A1, US 2008/0260774 A1, US 2015/0071960 A1, US 2010/0035843 A1.

From the ensemble of work reported in the above cited art, it can be inferred that the structures of designed αGalCer-inspired glycolipid antigens for iNKT cells can be conceptually divided into four portions: lipid chains, sphingosine/ceramide head group, the glycosidic bond, and the nature of the sugar. All the four have been chemically modified in search for improve immunomodulatory glycolipid candidates. A particular emphasis has been on obtaining glycolipids that stimulate iNKT cell responses that are more biased toward purely Th1 or Th2 cytokine predominance.

Multiple classes of αGalCer variants effectively trigger Th1 responses from iNKT cells, and these responses have proven effective in adjuvating vaccines and treating some forms of cancer. Early modifications at the sugar moiety showed that the pyranose form and especially the α-anomeric configuration are essential for adjuvant activity. Substitutions or additions at the 6"-OH group (the primary OH group in the galactopyranosyl moiety), for instance by substituted amides, carbamates, and ureas, promote Th1-biased immune responses, which is attributed to their enhanced *in vivo* stability that favors IFNγ over IL4 expression in the long term. The more stable α-carba-GalCer variant, in which the endocyclic oxygen is replaced by a carbon, also is a strongly Th1-inducing immunostimulant glycolipid. Similarly, substitution of the exocyclic glycosidic oxygen by a methylene unit provided the corresponding C-glycoside analogue (α-C-GalCer), which exhibited a marked Th1 response in mice. However, in studies using cell culture models of human iNKT cell responses, α-C-GalCer has been found to be only weakly stimulatory, suggesting that it may not be suitable for development as a human therapeutic. The thioglycoside analogue α-S-GalCer was also synthesized, showing no bioactivity in mice, while inducing a Th1 profile in humans. By contrast, KRN7000 analogues in which the ring oxygen of the galactopyranose residue is replaced by a nitrogen atom along with variation on the ceramide moiety displayed no activity on inducing the cytokine release, as reported in the document Chinese Journal of Chemistry 2017, 35, 1001-1008.

Another strongly Th1-biasing iNKT cell agonist that was previously identified is the aminodiol (sphinganine) analog of KRN7000, denoted AH03-1. In AH03-1, the OH group at position 4' in the sphingosine lipid chain is missing. AH03-1 and other related sphinganine analogs have generally been found to be highly active in vivo in mice, but they were not efficient activators in cell culture models of human iNKT cell responses. A variety of other αGalCer analogues with diverse structural modifications in the lipid moiety have also been synthesized, enabling further structure-activity relationship (SAR) studies. The following article further illustrates the state of the art: PLoS ONE 2010, 5, e14374.

Fewer α-GalCer-related antigens have been discovered that trigger Th2 immunomodulatory responses. Activation of iNKT cells by this type of antigen may be generally useful in decreasing inflammation and ameliorating autoimmune diseases. Two such compounds are C20:2 αGalCer (which has an unsaturated acyl chain) and OCH (which has a truncated sphingosine chain). Some αGalCer analogs with modifications at position 6" in the sugar moiety, for instance 6"-triazole-substituted derivatives and 6-O-alkylated derivatives bearing long ether-linked aliphatic chains, also elicited modest Th2 profiles.

iNKT cells are also associated with the disruption of mucosal homeostasis in the intestines and airways and contribute to allergic airway inflammation in various allergen models, including ovalbumin (OVA) and house dust mite (HDM) extract, through Th2-biased cytokine responses. Indeed, it has been found that iNKT cells are required for the development of airway hyperreactivity (AHR). Compositions that attenuate CD1d-restricted iNKT cell responses can then be used to treat allergeninduced airway hyperactivity associated with activation of CD1d-restricted iNKT cells, such as asthma and allergic rhinitis. Additionally, iNKT cells also contribute to acute immune-mediated hepatitis by inducing liver injury. Prior art has shown that the inhibition of iNKT cell activation using glycolipid antagonists can attenuate iNKT cellinduced liver disease pathogenesis. Examples of α-GalCer analogs with iNKT antagonist behavior with potential for treating iNKT cell-mediated diseases include α-mannoppranosylceramide (a-ManCer) and α-lactosylceramide (a-LacCer), as reported in the non-patent document Frontiers in Chemistry 2019, 7, 811.

Among the reported α-GalCer analogs with immunomodulatory activity, the glycosylserine-type multiantennary glycolipids, of which the compound termed CCL-34 is an iconic example, represent a singular class. CCL-34 comprises a galactopyranosyl moiety α-linked to a serine-based hydrophobic lipid moiety that contains a linear N-linked C₁₁ lipid chain attached to the carbonyl group of the amino acid, thus forming an amide link, and a linear C₁₂ acyl substituent at the amine group. It was found that CCL-34, but not α-GalCer itself, is a Toll-like receptor 4 (TLR4) agonist. The following selected report illustrates the potential of CCL-34 glycolipids as immunomodulators: Scientific Reports, 2020, 10, 8422;

The Toll-like receptor 4 (TLR4) is the mammalian receptor responsible for the Gram-negative bacterial endotoxin recognition (lipopolysaccharide, LPS, and lipooligosaccharide, LOS). TLR4 is mainly expressed on the cell surface of innate immune and epithelial cells, allowing them to sense minute amounts of LPS released by the Gram-negative bacteria. An ordered series of interactions among the lipophilic portion of LPS and LBP3, CD14 and MD-2 co-receptors allows the formation of the activated membrane heterodimeric complex (LPS/MD-2/TLR4)₂ that triggers an intracellular signal inducing the production of pro-inflammatory cytokines and chemokines. TLR4-mediated cytokine production is an essential mechanism by which the host organism responds to infections, however, excessive stimulation of TLR4 by pathogen-associated molecular patterns (PAMPs) can cause uncontrolled cytokine production leading to serious life-threatening syndromes such as acute sepsis and septic shock. TLR4 activation by endogenous factors (DAMPs) has been associated to several inflammatory disorders and auto-immune diseases affecting a variety of organs and body functions. In this context, the development of hit or lead compounds that are able to modulate TLR4 signaling is attracting increasing interest for a wide range of possible therapeutic settings as can be inferred from the following selected reports: Scientific Reports, 2019, 9, 919; Organic & Biomolecular Chemistry 2011, 9, 2492-2504; Biochemical Pharmacology 2007, 73, 1957-1970.

As it can be deducted from the preceding paragraphs and the selection of pertaining patent and non-patent documents cited, there are several examples on immunoregulatory glycolipids capable of modulating the Th1/Th2 cell balance. Virtually all of them contain a mono, di or oligosaccharide moiety as the glycone moiety (the sugar part of the glycolipid) and their synthesis involves the formation of a glycosidic linkage between a glycosyl donor and a precursor-of-the-lipid acceptor as a critical step. This generally implies the formation of both possible anomers, α and β, and the need of cumbersome separations. Moreover, the sugar glycone and the glycosidic linkage might be chemically and metabolically unstable. Lastly, the Th1/Th2 modulating behavior is often under optimal.

In view of the above, it would be useful to identify additional glycolipid-like agents that directly and specifically can induce or modulate the immune response by agonizing or antagonizing either iNKT cells or the TLR4 receptor. Of particular interest is developing new strategies allowing accessing such compounds with high efficiency while warranting: (a) total α-stereoselectivity during the generation of the glycosidic linkage, (b) metabolic stability and (c) compatibility with molecular diversity-oriented strategies, thus permitting systematic modifications of the structure to optimize the immunomodulatory profile.

### SUMMARY OF THE INVENTION

A first aspect of the present invention relates to a compound of formula I: 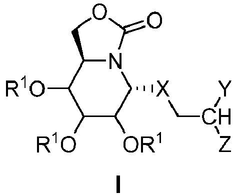 wherein:
each R¹ is independently -H, -CH₂Ph (Bn), -COPh, -CO-C₁-C₁₂ alkyl, C₁-C₁₂ alkyl,
wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is O, S, NH or CH₂;
Y is a group NH-C(=O)-Y', NH-C(=O)-NHY', NH-C(=S)-NHY', NH-SO₂-Y' or a group of formula **la**: 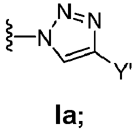
Y' is C₁-C₂₅ alkyl optionally substituted by one or more groups R⁴, preferably by one or two groups R⁴, and more preferably by one group R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁;
C_{y1} is a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄ alkyl, -O-C₁-C₄ alkyl, - OCOO-C₁-C₄ alkyl, -CO-C₁-C₄ alkyl, S- C₁-C₄ alky or N₃-C₁-C₄ alkyl;
Z is -CO-O-C₄-C₂₅ alkyl, -CO-NH-C₄-C₂₅ alkyl or C₄-C₂₅ alkyl, wherein Z is optionally substituted by one or more groups R⁵; preferably Z is -CO-O-C₄-C₂₅ lineal alkyl, -CO-NH-C₄-C₂₅ lineal alkyl or C₄-C₂₅ lineal alkyl, wherein Z is optionally substituted by one or more groups R⁵; and
each R⁵ independently is -OH, -OCH₂Ph (-OBn), -OC(=O)Ph (-OBz), C₁-C₁₂ alkyl, -O-C₁-C₁₂ alkyl, -S-C₁-C₁₂ alkyl, -N(R³)₂, -N₃, halogen or Cy₁.

In another embodiment the invention relates to a compound of formula **I** as defined above wherein each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from - H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl.

In another embodiment the invention relates to a compound of formula **I** as defined above wherein each R¹ is independently selected from -H, -CH₂Ph (Bn), -CO-C₁-C₁₂ alkyl, wherein each R¹ is independently optionally substituted by one or more groups R².

In another embodiment the invention relates to a compound of formula **I** as defined above, wherein each R¹ is independently selected from -H, -CH₂Ph (Bn), -CO-C₁-C₁₂ alkyl.

In another embodiment the invention relates to a compound of formula I as defined above, wherein X is O or S.

In another embodiment the invention relates to a compound of formula **I** as defined above, wherein Y is a group NH-C(=O)-Y', NH-C(=S)-NHY', NH-SO₂-Y' or a group of formula **Ia**.

In another embodiment the invention relates to a compound of formula **I** as defined above, wherein:
Y is a group NH-C(=O)-Y' or NH-C(=S)-NHY', NH-SO₂-Y';
Y' is C₁-C₂₅ alkyl optionally substituted by one or more groups R⁴, preferably by one or two groups R⁴, and more preferably by one group R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁, preferably R⁴ is -OH, -OCH₂Ph (-OBn), C₁-C₁₂ alkyl, -O-C₁-C₁₂ alkyl, -S-C₁-C₁₂ alkyl, -N(R³)₂, -N₃, halogen or Cy₁, more preferably R⁴ is Cy₁; and even more preferably R⁴ is Ph.

In another embodiment the invention relates to a compound of formula **I** as defined above, wherein:
Y is a group of formula **la**; and
Y' is C₁-C₂₅ alkyl optionally substituted by one or more groups R⁴, and preferably Y' is C₁-C₁₂ alkyl optionally substituted by one or more groups R⁴.

In another embodiment the invention relates to a compound of formula **I** as defined above, wherein:
Y is a group of formula **Ia;**
Y' is C₁-C₂₅ alkyl, and preferably Y' is C₁-C₁₂ alkyl.

In another embodiment the invention relates to a compound of formula **I** as defined above, wherein:
Y is a group of formula **Ia**; and
Y' is a C₁-C₂₅ lineal alkyl optionally substituted by one or more groups R⁴, preferably Y' is a C₁-C₂₅ lineal alkyl, and more preferably Y' is a C₁-C₁₂ lineal alkyl.

In another embodiment the invention relates to a compound of formula **I** as defined above, wherein Z is -CO-NH-C₄-C₂₅ alkyl optionally substituted by one or more groups R⁵; preferably Z is -CO-NH-C₈-C₂₀ alkyl optionally substituted by one or more groups R⁵, more preferably Z is -CO-NH-C₁₄-C₁₆ alkyl optionally substituted by one or more groups R⁵, still more preferably Z is -CO-NH-C₄-C₂₅ lineal alkyl; and even more preferably Z is -CO-NH-C₈-C₂₀ lineal alkyl.

In another embodiment the invention relates to a compound of formula **I** as defined above, wherein:
Z is C₄-C₂₅ alkyl, wherein Z is substituted by two groups R⁵; and
each R⁵ independently is -OH or -OC(=O)Ph (-OBz).

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group NH-C(=O)-Y', NH-C(=S)-NHY', NH-SO₂-Y'or a group of formula la;
Z is -CO-NH-C₄-C₂₅ lineal alkyl; and preferably Z is -CO-NH-C₈-C₂₀ lineal alkyl.

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group NH-C(=O)-Y', NH-C(=S)-NHY', NH-SO₂-Y'or a group of formula la;
Z is C₄-C₂₅ alkyl, wherein Z substituted by two groups R⁵; and
each R⁵ independently is -OH or -OC(=O)Ph (-OBz).

In another embodiment the invention relates to a compound of formula **I** as defined above, wherein:
each R¹ is independently selected from -H, -CH₂Ph (Bn), -CO-C₁-C₁₂ alkyl, wherein
each R¹ is independently optionally substituted by one or more groups R², preferably wherein each R¹ is independently selected from -H, -CH₂Ph (Bn), -CO-C₁-C₁₂ alkyl;
X is O or S;
Y is a group NH-C(=O)-Y', NH-C(=S)-NHY', NH-SO₂-Y'or a group of formula **la**;
Z is -CO-NH-C₄-C₂₅ alkyl optionally substituted by one or more groups R⁵; and preferably Z is -CO-NH-C₄-C₂₀ alkyl optionally substituted by one or more groups R⁵.

In another embodiment the invention relates to a compound of formula **I** as defined above, wherein:
each R¹ is independently selected from -H, -CH₂Ph (Bn), -CO-C₁-C₁₂ alkyl, wherein
each R¹ is independently optionally substituted by one or more group R², preferably wherein each R¹ is independently selected from -H, -CH₂Ph (Bn), -CO-C₁-C₁₂ alkyl;
X is O or S;
Y is a group NH-C(=O)-Y', NH-C(=S)-NHY', NH-SO₂-Y'or a group of formula la;
Z is C₄-C₂₅ alkyl, wherein Z is substituted by two groups R⁵; and
each R⁵ independently is -OH or -OC(=O)Ph (-OBz).

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group NH-C(=O)-Y' or NH-C(=S)-NHY', NH-SO₂-Y';
Y' is C₁-C₂₅ alkyl optionally substituted by one or more groups R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁, preferably R⁴ is -OH, -OCH₂Ph (-OBn), C₁-C₁₂ alkyl, -O-C₁-C₁₂ alkyl, -S-C₁-C₁₂ alkyl, -N(R³)₂, -N₃, halogen or Cy₁, more preferably R⁴ is Cy₁; and even more preferably R⁴ is Ph;
Z is -CO-NH-C₄-C₂₅ alkyl optionally substituted by one or more groups R⁵; and preferably Z is -CO-NH-C₄-C₂₀ alkyl optionally substituted by one or more groups R⁵.

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group NH-C(=O)-Y', NH-C(=O)-NHY', NH-C(=S)-NHY' or NH-SO₂-Y';
Y' is C₁-C₂₅ alkyl optionally substituted by one or more groups R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁, preferably R⁴ is -OH, -OCH₂Ph (-OBn), C₁-C₁₂ alkyl, -O-C₁-C₁₂ alkyl, -S-C₁-C₁₂ alkyl, -N(R³)₂, -N₃, halogen or Cy₁, more preferably R⁴ is Cy₁; and even more preferably R⁴ is Ph;
Z is C₄-C₂₅ alkyl, wherein Z substituted by two groups R⁵; and
each R⁵ independently is -OH or -OC(=O)Ph (-OBz).

In another embodiment the invention relates to a compound of formula **I** as defined above, wherein:
each R¹ is independently selected from -H, -CH₂Ph (Bn), -CO-C₁-C₁₂ alkyl, wherein
each R¹ is independently optionally substituted by one or more groups R², X is O or
S, preferably wherein each R¹ is independently selected from -H, -CH₂Ph (Bn), -CO-C₁-C₁₂ alkyl;
X is O or S;
Y is a group NH-C(=O)-Y' or NH-C(=S)-NHY', NH-SO₂-Y';
Y' is C₁-C₂₅ alkyl optionally substituted by one or more groups R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁, preferably R⁴ is -OH, -OCH₂Ph (-OBn), C₁-C₁₂ alkyl, -O-C₁-C₁₂ alkyl, -S-C₁-C₁₂ alkyl, -N(R³)₂, -N₃, halogen or Cy₁, more preferably R⁴ is Cy₁; and even more preferably R⁴ is Ph;
Z is -CO-NH-C₄-C₂₅ lineal alkyl; and preferably Z is -CO-NH-C₄-C₂₀ lineal alkyl.

In another embodiment the invention relates to a compound of formula **I** as defined above, wherein:
each R¹ is independently selected from -H, -CH₂Ph (Bn), -CO-C₁-C₁₂ alkyl, wherein
each R¹ is independently optionally substituted by one or more groups R², X is O or
S, preferably wherein each R¹ is independently selected from -H, -CH₂Ph (Bn), -CO-C₁-C₁₂ alkyl;
Y is a group NH-C(=O)-Y' or a group of Formula **la**;
Y' is C₁-C₂₅ alkyl optionally substituted by one or more groups R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂, - N₃, halogen or Cy₁, preferably R⁴ is -OH, -OCH₂Ph (-OBn), C₁-C₁₂ alkyl, -O-C₁-C₁₂ alkyl, -S-C₁-C₁₂ alkyl, -N(R³)₂, -N₃, halogen or Cy₁, more preferably R⁴ is Cy₁; and even more preferably R⁴ is Ph;
Z is C₄-C₂₅ alkyl, wherein Z is substituted by two groups R⁵; and
each R⁵ independently is -OH or -OC(=O)Ph (-OBz).

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group of formula **la**;
Y' is C₁-C₂₅ alkyl optionally substituted by one or more groups R⁴, preferably Y' is a C₁-C₂₅ lineal alkyl, and more preferably Y' is a C₁-C₁₂ lineal alkyl;
Z is -CO-NH-C₄-C₂₅ alkyl optionally substituted by one or more groups R⁵; and preferably Z is -CO-NH-C₈-C₂₀ alkyl optionally substituted by one or more groups R⁵.

In another embodiment the invention relates to a compound of formula **I** as defined above, wherein:
each R¹ is independently selected from -H, -CH₂Ph (Bn), -COPh, -CO-C₁-C₄ alkyl, C₁-C₄ alkyl, wherein each R¹ is independently optionally substituted by one or more groups R², preferably wherein each R¹ is independently selected from -H, -CH₂Ph (Bn), -COPh, -CO-CH₃, C₁-C₄ alkyl, wherein each R¹ is independently optionally substituted by one or more groups R²; and more preferably wherein each R¹ is independently selected from -H, -CH₂Ph (Bn), -COPh, -CO-CH₃, C₁-C₄ alkyl;
X is O or S;
Y is a group of formula **la**;
Y' is C₁-C₂₅ alkyl optionally substituted by one or more groups R⁴, and preferably Y' is C₁-C₂₅ alkyl optionally substituted by one or more groups R⁴, and more preferably Y' is C₁-C₁₂ lineal alkyl;
Z is C₄-C₂₅ alkyl, wherein Z is substituted by two groups R⁵; and
each R⁵ independently is -OH or -OCH₂Ph (-OBn).

In another embodiment the invention relates to a compound of formula **I** as defined above, wherein:
each R¹ is independently selected from -H, -CH₂Ph (Bn), -CO-C₁-C₁₂ alkyl, wherein
each R¹ is independently optionally substituted by one or more groups R², X is O or
S, preferably wherein each R¹ is independently selected from -H, -CH₂Ph (Bn), -CO-C₁-C₁₂ alkyl;
Y is a group of formula la;
Y' is C₁-C₂₅ alkyl optionally substituted by one or more groups R⁴, and preferably Y' is C₁-C₂₅ alkyl optionally substituted by one or more groups R⁴, and more preferably Y' is C₁-C₁₂ lineal alkyl;
Z is C₄-C₂₅ alkyl, wherein Z substituted by two groups R⁵; and
each R⁵ independently is -OH or -OCH₂Ph (-OBz).

In another embodiment, the compound of formula **I** as defined above is selected from:
1 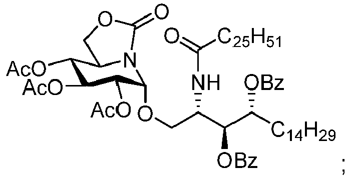
2 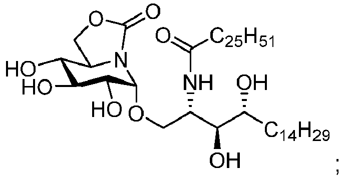
3 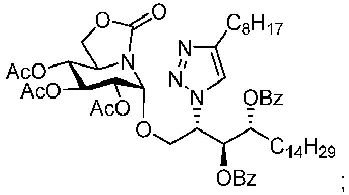
4 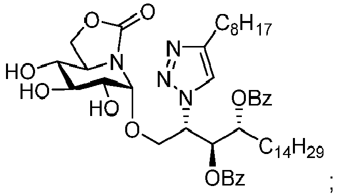
5 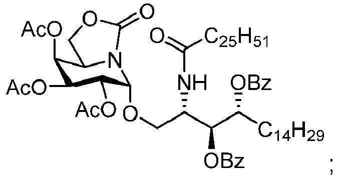
6 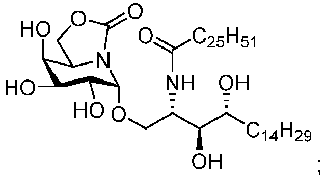
7 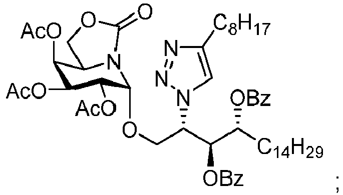
8 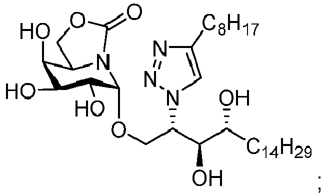
9 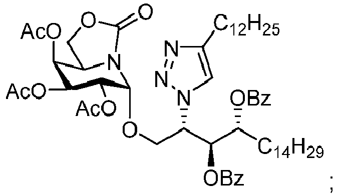
10 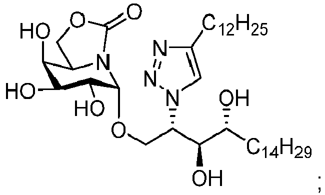
11 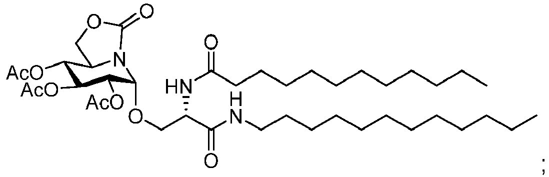
12 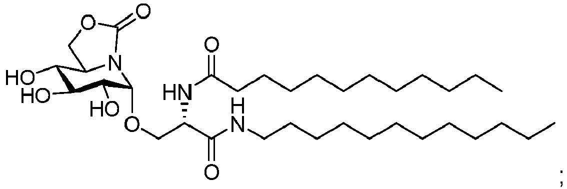
13 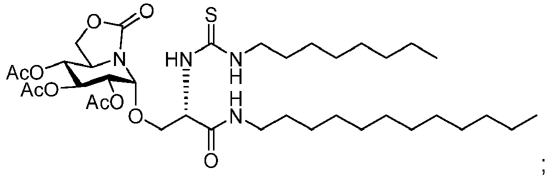
14 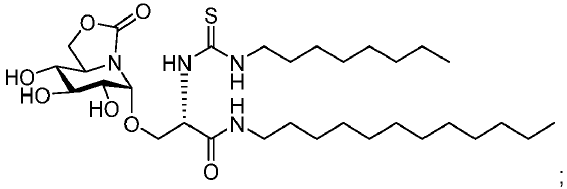
15 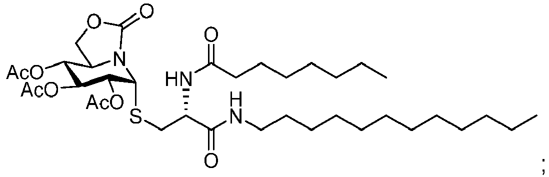
16 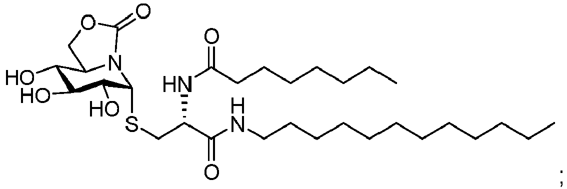
17 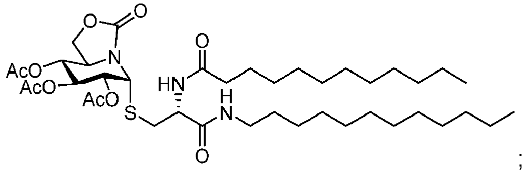
18 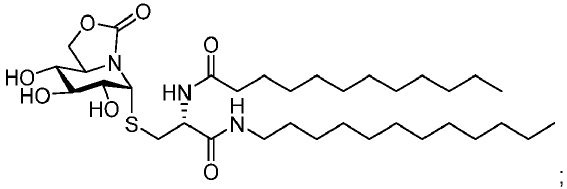
19 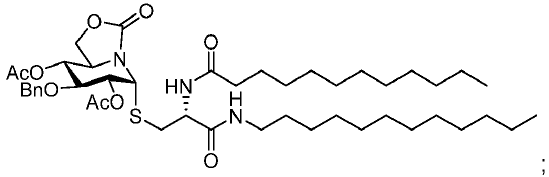
20 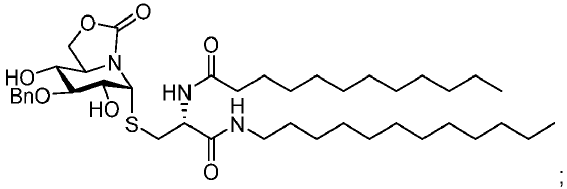
21 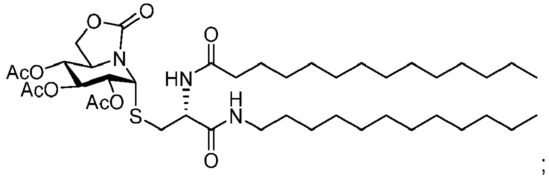
22 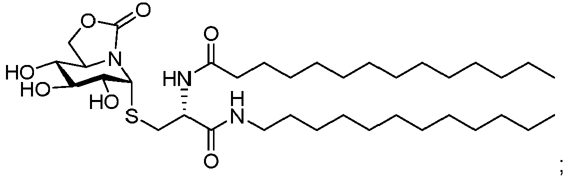
23 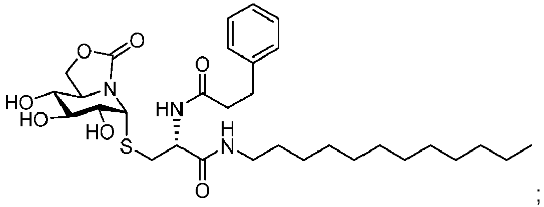
24 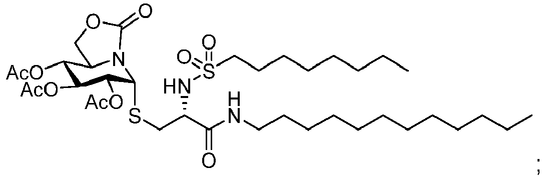
25 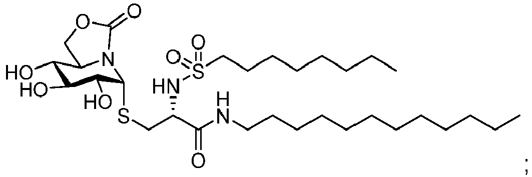
26 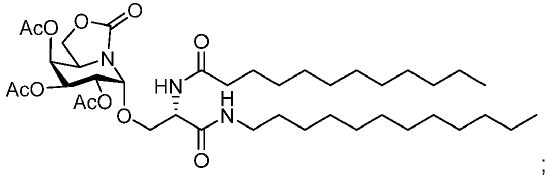
27 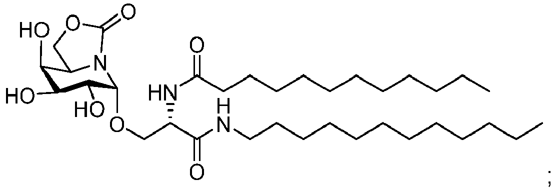
28 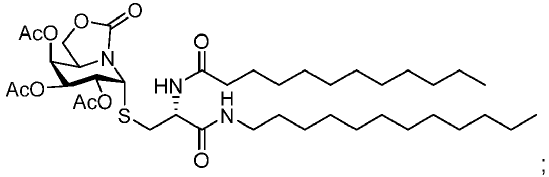
29 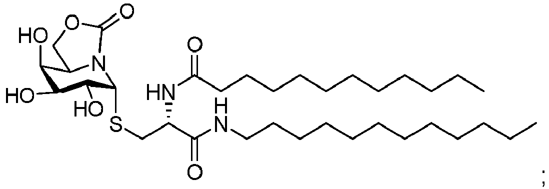
30 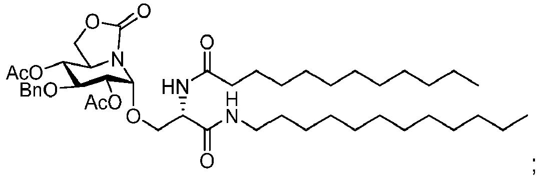 and
31

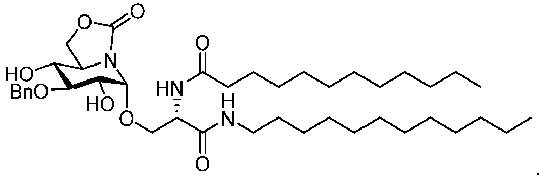

In some embodiments, any configurational pattern of the polysubstituted 2-oxa-3-oxoindolizidin-5-yl, any configuration of the CH group bearing the Y and Z substituents and anyone of the substituents R¹, X, Y and/or Z in compounds 1-31 may independently be combined, *i.e.* individual substituents may be selected from the multiple compounds as if each specific combination was explicitly listed.

As used herein the singular forms "a", "and", and "the" include plural referents unless the context clearly dictates otherwise. For example, "a compound" refers to one or more of such compounds as known to those skilled in the art.

Throughout this application, it is contemplated that the term "compound" or "compounds" refers to the compounds discussed herein and includes precursors and derivatives of the compounds, including acyl-protected derivatives, and pharmaceutically acceptable salts of the compounds, precursors, and derivatives.

In another aspect, the compounds of the present disclosure are capable of acting as agonist or antagonists of iNKT cells or as agonists or antagonists of the TLR-4 receptor in immune system cells. In yet another aspect, the compounds of the present disclosure are capable of eliciting a Th1-type, a Th2-type or a Th1-type and a Th2-type response. In another implementation, the compounds of the invention are capable of counterbalance a Th1-type, a Th2-type or a Th1-type and a Th2-type response elicited by a third agent. In an exemplary implementation, the compounds of the invention are capable of acting as agonist or antagonists of iNKT cells or as agonists or antagonists of the TLR-4 receptor in immune system cells in vitro. In another implementation, the compounds of the invention are capable of acting as agonist or antagonists of iNKT cells or as agonists or antagonists of the TLR-4 receptor in immune system cells in vitro.

The invention also includes prodrugs of the compounds, pharmaceutical compositions including the compounds and a pharmaceutically acceptable carrier, and pharmaceutical compositions including prodrugs of the compounds and a pharmaceutically acceptable carrier.

The compounds of formula **I** of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. Any formulas, structures or names of compounds described in this specification that do not specify a particular stereochemistry are meant to encompass any and all existing isomers as described above and mixtures thereof in any proportion. When stereochemistry is specified, the invention is meant to encompass that particular isomer in pure form or as part of a mixture with other isomers in any proportion.

Accordingly, the compounds of formula **I** of the invention may contain chiral centers or double bonds, In the case that contains chiral centers, those may indistinctly have the (*R*) or (S) configuration. In the case that contains double bonds, those may indistinctly have the (Z) or (E) configuration.

Along the present description the term "multiantennary lipid" refers to a lipophilic moiety that contains two or more hydrocarbon or substituted hydrocarbon tails.

The terms "C₁-C₄ alkyl", "C₁-C₁₂ alkyl", "C₁-C₂₅ alkyl", C₈-C₂₀ alkyl, "C₄-C₂₅ alkyl" and the like, as a group or part of a group, means a straight or branched alkyl chain which contains from 1 to 4, from 1 to 12, from 1 to 25, from 8 to 20 or from 4 to 25 carbon atoms and the like, respectively.

The term "lineal alkyl" as part of a group such as "C₁-C₂₅ lineal alkyl" or "-CO-NH-C₄-C₂₅ lineal alkyl" means a straight alkyl chain as defined above.

"Halogen" means fluoro, chloro, bromo or iodo.

The term Cy₁ refers to a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄ alkyl, - O-C₁-C₄ alkyl, -OCOO-C₁-C₄ alkyl, -CO-C₁-C₄ alkyl, S- C₁-C₄ alky or N₃-C₁-C₄ alkyl. Examples include among others aziridine, oxirane, thiirane, azetidine,oxetane, thietane, azolidine, oxolane, thiolane, phopholane, piperidine, piperazine tetrahydropyrane, oxane, 1,4-dioxane, morpholine, phosphinane, phenyl, biphenyl, naphthyl, indanyl, indenyl, tetrahydronaphthyl, 2,3-dihydrobenzofuranyl, dihydrobenzopyranyl, 1,4-benzodioxanyl, furan, thiophene, pyrrole, oxazole, thiazole, imidazole, pyrazole, isoxazole, isothiazole, 1,2,3-oxadiazole, 1,2,3-triazole, 1,2,4-triazole, 1,3,4-thiadiazole, tetrazole, pyridine, pyridazine, pyrimidine, pyrazine, 1,3,5-triazine, imidazole, benzimidazole, benzoxazole, benzothiazole, indolizine, indole, isoindole, benzofuran, benzothiophene, 1H-indazole, purine, 4Hquinolizine, quinoline, isoquinoline, cinnoline, phthalazine, quinazoline, quinoxaline, 1,8-naphthyridine and pteridine.

When in the definitions used throughout the present specification for cyclic groups the examples given refer to a radical of a ring in general terms, for example pyridyl, thienyl or indolyl, all the available bonding positions are included, unless a limitation is indicated in the corresponding definition for said cyclic group, for example that the ring is bonded through a C atom in Cy₁, in which case such limitation applies.

For example, "optionally substituted" means that the referred group may or may not be substituted and that the description includes both substituted groups and groups having no substitution, and the substitution may occur one or more times.

The expression "optionally substituted with one or more" means that a group can be substituted with one or more, preferably with 1, 2, 3 or 4 substituents, more preferably with 1, 2 or 3 substituents, and still more preferably with 1 or 2 substituents, provided that said group has enough positions susceptible of being substituted. The substituents can be the same or different and can be placed on any available position.

The compound of the present invention can be produced by various methods known per se that involve the reaction of a suitable polysubstituted 2-oxa-3-oxoindolizidine derivative bearing a reactive group at position C-5 with an appropriate precursor of the multiantennary lipid portion to generate the glycosidic-like linkage. This reaction is termed the conjugation step. For example, a compound of formula **I** can be obtained by conjugation of polysubstituted 2-oxa-3-oxoindolizidine and lipid precursors according to the following Scheme 1 or a method analogous thereto.

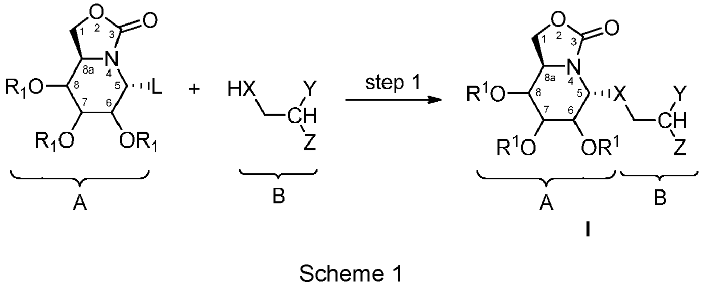

Wherein in scheme 1, L is a leaving group, the configurational patterns of the glycone and lipid moieties A and B are defined as above described for **I** and R¹, Y and Z are defined as above described for **I**, except that any functional group that can interfere in step 1 is adequately protected to avoid side reactions. For instance, without limitation, hydroxyl groups can be protected as ester or ether derivatives, amino groups can be protected as amide or carbamate derivatives and thiol groups can be protected as thioester or thioether derivatives. Examples of esters are acetyl and benzoyl derivatives. Examples of ethers are benzyl or p-methoxybenzyl derivatives. Examples of amides are trifluoroacetamide derivatives. Examples of carbamates are *tert*-butyloxycarbonyl (Boc), benzyloxycarbonyl (Cbz) or fluorenyloxycarbonyl (Fmoc) derivatives. Examples of thioesters are S-acetyl derivatives. Examples of thioether derivatives are S-p-methoxybenzyl or S-2,4,6-trimethoxybenzyl thioether derivatives. Optionally, the protecting groups can be totally or partially removed in a later step. Examples of the leaving group L include hydroxyl, acyloxy (acetoxy and the like) trichloroacetamidoyloxy, phosphoric acid ester [-OP(=O)(OPh)₂ and the like], halogen (Br, F and the like) and the like.

In some embodiments, the glycolipid mimetic obtained after the conjugation step as above mentioned is next subjected to further transformations to obtain the final multiantennary glycolipid mimetic. For example, a compound of formula **I** with a configurational profile analogous to that of an α-D-glucopyranoside, wherein X is O and Y is NHC(=O)Y', represented by the general formula (II), such as, for instance, compounds **11, 12, 30** or **31,** and a compound of formula **I** with a configurational profile analogous to that of an α-D-glucopyranoside, wherein X is O and Y is NHC(=S)NHY', represented by the general formula (**III**), such as, for instance, compounds **13** or **14,** can be obtained according to the following Scheme 2 or a method analogous thereto; a compound of formula **I** with a configurational profile analogous to that of an α-D-glucopyranoside, wherein X is S and Y is NHC(=O)Y', represented by the general formula (IV), such as, for instance, compounds **15-24,** and a compound of formula **I** with a configurational profile analogous to that of an α-D-glucopyranoside, wherein X is S and Y is NHSO₂Y', represented by the general formula (V), such as, for instance, compounds **24** or **25,** can be obtained according to the following Scheme 3 or a method analogous thereto; a compound of formula **I** with a configurational profile analogous to that of an α-D-galactopyranoside, wherein X is O and Y is NHC(=O)Y', represented by the general formula (**VI**), such as, for instance, compounds **26** or **27,** can be obtained according to the following Scheme 4 or a method analogous thereto; and a compound of formula **I** with a configurational profile analogous to that of an α-D-galactopyranoside, wherein X is S and Y is NHC(=O)Y', represented by the general formula general formula (VII), such as, for instance, compounds 28 or 29, can be obtained according to the following Scheme 5 or a method analogous thereto.

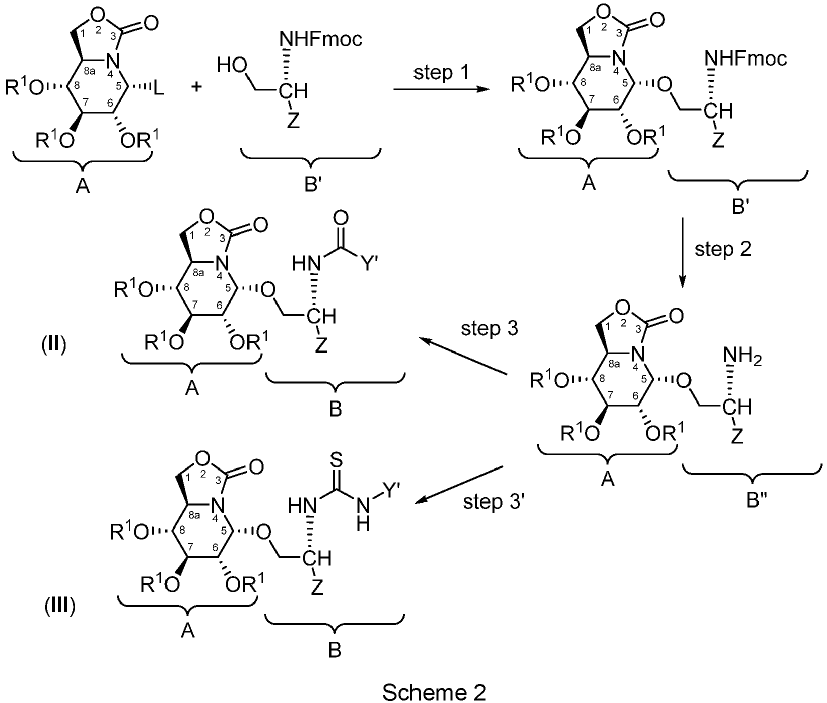

Wherein L is a leaving group, the configurational patterns of the B' and B" lipid moieties in the lipid precursor and the glycolipid mimetic intermediates are identical to the configurational patterns of B in compound (II) and (III), R¹ and Y' are defined as above described for I and Z is selected from -CO-NH-C₄-C₂₅ alkyl optionally substituted by one or more groups R⁵, except that any functional group that can interfere in steps 1, 2 or 3 is adequately protected to avoid side reactions. For instance, without limitation, hydroxyl groups can be protected as ester or ether derivatives, amino groups can be protected as amide or carbamate derivatives and thiol groups can be protected as thioester or thioether derivatives. Examples of esters are acetyl and benzoyl derivatives. Examples of ethers are benzyl or p-methoxybenzyl derivatives. Examples of amides are trifluoroacetamide derivatives. Examples of carbamates are *tert*-butyloxycarbonyl (Boc), benzyloxycarbonyl (Cbz) or fluorenyloxycarbonyl (Fmoc) derivatives. Examples of thioesters are S-acetyl derivatives. Examples of thioether derivatives are S-p-methoxybenzyl or S-2,4,6-trimethoxybenzyl thioether derivatives. Optionally, the protecting groups can be totally or partially removed in a later step.

Examples of the leaving group L include hydroxyl, acyloxy (acetoxy and the like) trichloroacetamidoyloxy, phosphoric acid ester [-OP(=O)(OPh)₂ and the like], halogen (Br, F and the like) and the like.

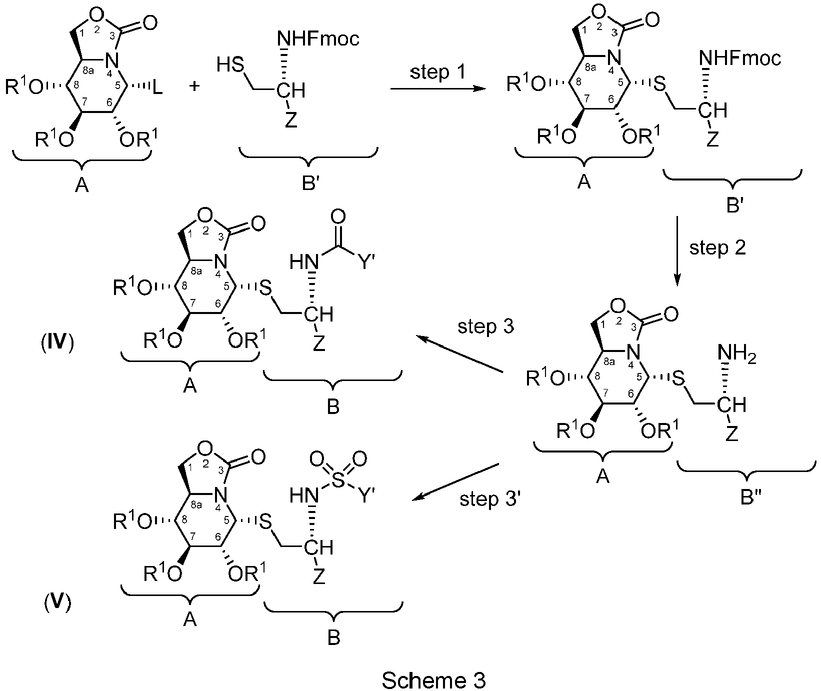

Wherein L is a leaving group, the configurational patterns of the B' and B" lipid moieties in the lipid precursor and the glycolipid mimetic intermediates are identical to the configurational patterns of B in compound (**IV**) and (**V**), R¹ and Y' are defined as above described for **I** and Z is selected from -CO-NH-C₄-C₂₅ alkyl optionally substituted by one or more groups R⁵, except that any functional group that can interfere in in steps 1, 2 or 3 is adequately protected to avoid side reactions. For instance, without limitation, hydroxyl groups can be protected as ester or ether derivatives, amino groups can be protected as amide or carbamate derivatives and thiol groups can be protected as thioester or thioether derivatives. Examples of esters are acetyl and benzoyl derivatives. Examples of ethers are benzyl or p-methoxybenzyl derivatives. Examples of amides are trifluoroacetamide derivatives. Examples of carbamates are *tert*-butyloxycarbonyl (Boc), benzyloxycarbonyl (Cbz) or fluorenyloxycarbonyl (Fmoc) derivatives. Examples of thioesters are S-acetyl derivatives. Examples of thioether derivatives are S-p-methoxybenzyl or S-2,4,6-trimethoxybenzyl thioether derivatives. Optionally, the protecting groups can be totally or partially removed in a later step.

Examples of the leaving group L include hydroxyl, acyloxy (acetoxy and the like) trichloroacetamidoyloxy, phosphoric acid ester [-OP(=O)(OPh)₂ and the like], halogen (Br, F and the like) and the like.

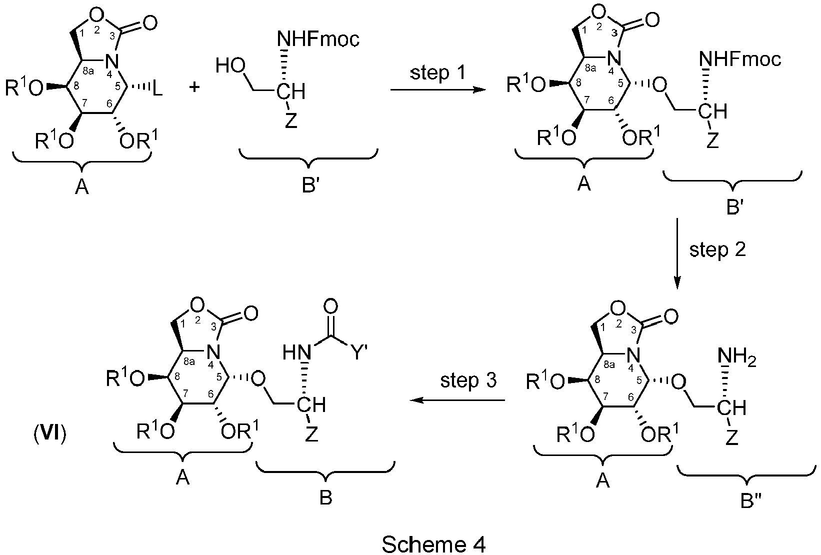

Wherein L is a leaving group, the configurational patterns of the B' and B" lipid moieties in the lipid precursor and the glycolipid mimetic intermediates are identical to the configurational patterns of B in compound (**VI**), R¹ and Y' are defined as above described for **I** and Z is selected from -CO-NH-C₄-C₂₅ alkyl optionally substituted by one or more groups R⁵,, except that any functional group that can interfere in in steps 1, 2 or 3 is adequately protected to avoid side reactions. For instance, without limitation, hydroxyl groups can be protected as ester or ether derivatives, amino groups can be protected as amide or carbamate derivatives and thiol groups can be protected as thioester or thioether derivatives. Examples of esters are acetyl and benzoyl derivatives. Examples of ethers are benzyl or p-methoxybenzyl derivatives. Examples of amides are trifluoroacetamide derivatives. Examples of carbamates are *tert*-butyloxycarbonyl (Boc), benzyloxycarbonyl (Cbz) or fluorenyloxycarbonyl (Fmoc) derivatives. Examples of thioesters are S-acetyl derivatives. Examples of thioether derivatives are S-p-methoxybenzyl or S-2,4,6-trimethoxybenzyl thioether derivatives. Optionally, the protecting groups can be totally or partially removed in a later step. Examples of the leaving group L include hydroxyl, acyloxy (acetoxy and the like) trichloroacetamidoyloxy, phosphoric acid ester [-OP(=O)(OPh)₂ and the like], halogen (Br, F and the like) and the like.

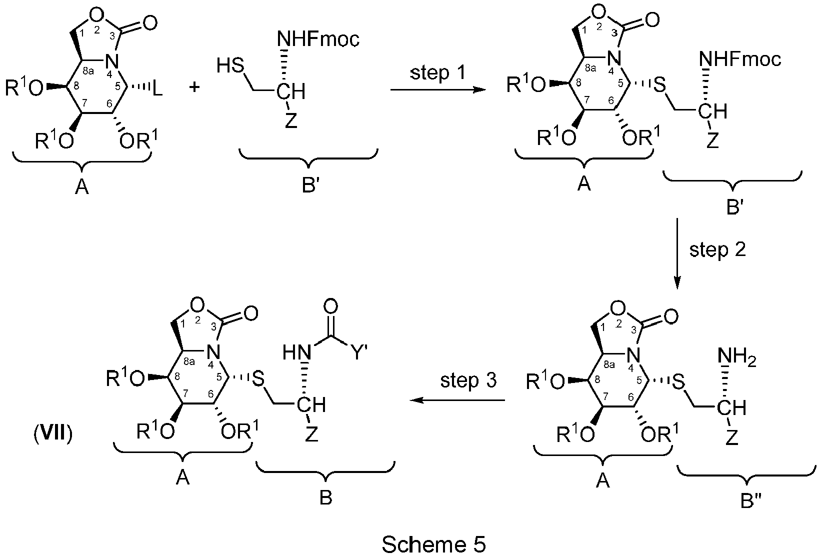

Wherein L is a leaving group, the configurational patterns of the B' and B" lipid moieties in the lipid precursor and the glycolipid mimetic intermediates are identical to the configurational patterns of B in compound (**VII**), R¹ and Y' are defined as above described for **I** and Z is selected from -CO-NH-C₄-C₂₅ alkyl optionally substituted by one or more groups R⁵, except that any functional group that can interfere in in steps 1, 2 or 3 is adequately protected to avoid side reactions. For instance, without limitation, hydroxyl groups can be protected as ester or ether derivatives, amino groups can be protected as amide or carbamate derivatives and thiol groups can be protected as thioester or thioether derivatives. Examples of esters are acetyl and benzoyl derivatives. Examples of ethers are benzyl or p-methoxybenzyl derivatives. Examples of amides are trifluoroacetamide derivatives. Examples of carbamates are *tert*-butyloxycarbonyl (Boc), benzyloxycarbonyl (Cbz) or fluorenyloxycarbonyl (Fmoc) derivatives. Examples of thioesters are S-acetyl derivatives. Examples of thioether derivatives are S-p-methoxybenzyl or S-2,4,6-trimethoxybenzyl thioether derivatives. Optionally, the protecting groups can be totally or partially removed in a later step.

Examples of the leaving group L include hydroxyl, acyloxy (acetoxy and the like) trichloroacetamidoyloxy, phosphoric acid ester [-OP(=O)(OPh)₂ and the like], halogen (F, Cl, Br or I) and the like.

Another aspect of the invention relates to a pharmaceutical composition which comprises at least one compound of formula **I** or any mixtures or combinations thereof. The composition of the invention my optionally comprise at least one of a pharmaceutically acceptable carrier, diluent, excipient and/or additive.

Another aspect of the invention relates to a compound of formula **I** or a pharmaceutical composition thereof as defined above for use as a medicament.

Another aspect of the invention relates to the use of a compound of formula **I** as defined above or a pharmaceutical composition thereof for the manufacture of a medicament.

Another aspect of the invention relates to a method of treatment or prevention of a disease in a subject in need, especially a human being, which comprises administering to the subject in need an effective amount of a compound of formula **I** as defined above or a pharmaceutical composition thereof.

Another aspect of the invention relates to a compound of formula **I** as defined above for use in the treatment and/or prevention of an immune disease cursed by Th1/Th2 imbalance.

In another embodiment the invention relates to a compound of formula **I** as defined above for use in the treatment and/or prevention of a disease selected from metabolic syndrome, non-alcoholic fatty liver disease (NAFLD), non-alcoholic steatohepatitis (NASH), immune mediated hepatitis, an autoimmune disease, graft rejection pathology, atherosclerosis, airway hyperactivity, asthma, allergic rhinitis and a neurodegenerative disorder.

In another embodiment the invention relates to a compound of formula **I** for the use defined above wherein the autoimmune disease is selected from rheumatoid arthritis, inflammatory bowel disease and gout.

In another embodiment the invention relates to a compound of formula **I** for the use defined above wherein the neurodegenerative disorder is selected from Parkinson's disease or Alzheimer's disease.

Throughout the present specification, by the term "treatment" is meant eliminating, reducing or ameliorating the cause or the effects of a disease. For purposes of this invention treatment includes, but is not limited to, alleviation, amelioration or elimination of one or more symptoms of the disease; diminishment of the extent of the disease; stabilized (i.e. not worsening) state of disease; delay or slowing of disease progression; amelioration or palliation of the disease state; and remission of the disease (whether partial or total).

As used herein, "prevention" refers to preventing the occurrence of a disease in a subject that is predisposed to or has risk factors but does not yet display symptoms of the disease. Prevention includes also preventing the recurrence of a disease in a subject that has previously suffered said disease.

Another aspect of the invention relates to a vaccine which comprises an effective amount of a compound of formula **I** or a pharmaceutical salt thereof and a vaccine agent.

In another embodiment the invention relates to the vaccine defined above, wherein the vaccine agent is selected from a killed microorganism, a live attenuated microorganism, a toxoid and a fragment of an inactivated or attenuated microorganism, a synthetic peptide or glycopeptide construct, a ribonucleic acid (RNA)-based vaccine or a deoxyribonucleic acid (DNA)-based vaccine.

In another embodiment the invention relates to the vaccine defined above, wherein the microorganism of the vaccine agent is a virus, a bacterium or a fungus.

In another embodiment the invention relates to the vaccine defined above, wherein the toxoid is a tetanus or a diphtheria toxoid.

In another embodiment the invention relates to the vaccine defined above, wherein the RNA is a messenger RNA (mRNA).

In another embodiment the invention relates to the vaccine defined above, wherein the DNA is a plasmid DNA (pDNA).

In another embodiment the RNA-based vaccine or the DNA-based vaccine comprises a pharmaceutically acceptable delivery system.

In another embodiment the delivery system is non-viral based or viral-based.

In another embodiment the non-viral based delivery system is selected from cationic lipids, cationic polymers, molecule-based systems and nanoparticle-based system.

In another embodiment the viral based delivery system is selected from engineered adenovirus and engineered lentivirus systems.

The therapeutic composition of the invention acts as an immunologic adjuvant and is capable of augmenting the immune response elicited by the vaccine agent by stimulating the immune systems, which results in the subject responding to the vaccine more vigorously than without the compound. In other instances, the therapeutic composition acts as an immunodepleting agent, allowing preventing or decreasing unwanted immunological reactions to the vaccine in the treated subject.

Considering the above, this invention provides a novel multiantennary glycolipid that is metabolically stable and can be selectively modified to optimize the immunomodulatory profile. The present invention also aims to provide a method to prepare the said glycolipid mimetic with total α-anomeric stereoselectivity. Further on, the present invention provides a medicament such as a vaccine formulation or an anti-inflammatory agent containing the novel glycolipid mimetic.

Accordingly, a compound wherein the sugar portion which is part of the multiantennary immunomodulatory glycolipids is converted to a polysubstituted 2-oxa-3-oxoindolizidin-5-yl radical selectively induces production of cytokines associated to Th1 or Th2 responses.

The compounds of the invention can be prepared with total α-anomeric stereoselectivity and is not degraded by the α-glycosidases that hydrolyze the glycosidic linkage in the natural or synthetic glycolipids, which warrants a higher metabolic stability.

Further, the compounds of the invention are useful for the modulation of the Th1/Th2 cell balance toward either pro-inflammatory or anti-inflammatory cytokine producing cells. And, accordingly, they can be used, for instance, as adjuvant in vaccine formulations or in the treatment and/or prevention of inflammation-associated conditions, depending on the type of immune response (Th1 or Th2) elicited.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

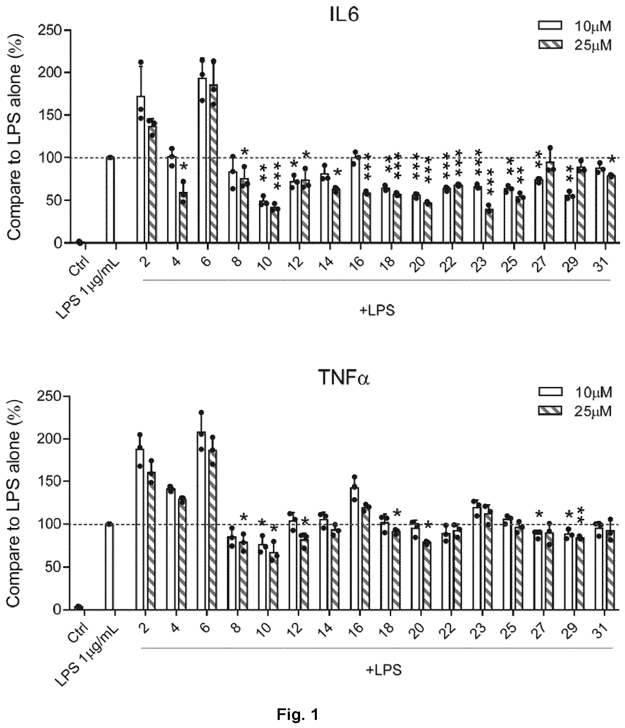

**Fig. 1****.** Shows suppression of LPS-induced inflammation (Splenocytes in vitro). Splenocytes were treated concurrently with the compounds of the invention and LPS to assess the suppressive function of compounds on LPS-induced inflammation, with several compounds showing prominent suppressive function.

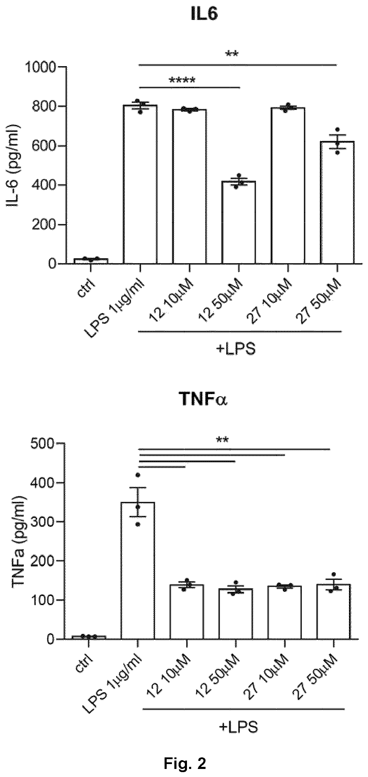

**Fig. 2**. Shows suppression of LPS-induced inflammation in bone marrow-derived macrophages (BMDM *in vitro).* Compounds **12** and **27** were assessed for their capability to suppress LPS-induced inflammation in bone marrow-derived macrophages (BMDM), with both showing significant suppression of IL-6 and TNFα.

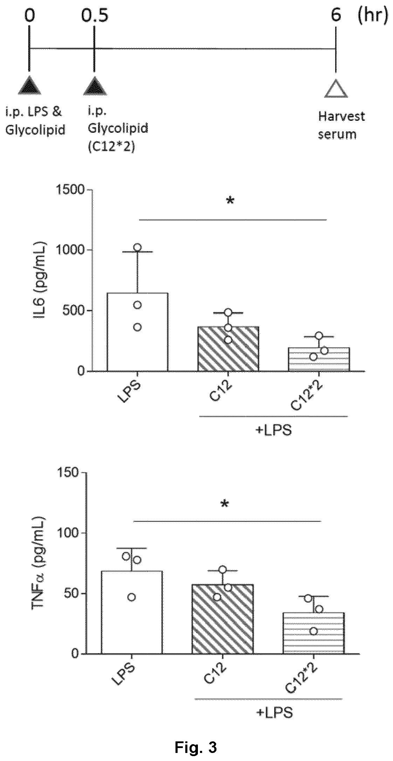

**Fig. 3****.** Shows suppression on LPS-induced inflammation *in vivo.* The *in vivo* capability of compound **12** in suppressing LPS-induced inflammation in B6 mice was assessed as indicated in the scheme. Compound **12** was shown to significantly suppressing LPS-induced serum IL-6 and TNFα when injected twice in a half hour time after LPS-treatment.

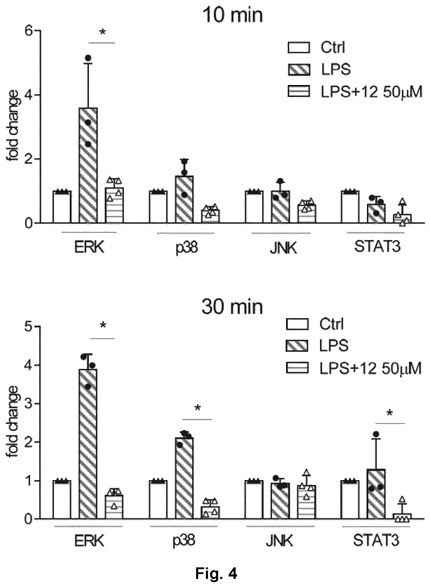

**Fig. 4****.** Shows that ERK and p38 of the MAPK family and STAT3 are suppressed by the new compounds. Compound **12,** a representative compound of the invention, was examined in term of signal transduction to explore how the new compounds exert their suppressive effect, using the multiplex bead array. Phosphorylation of the mitogenactivated protein kinases (MAPKs) ERK and p38 but not JNK, was significantly reduced by compound **12** comparing to LPS treatment alone. A significant reduction of phosphorylation in STAT3 was also observed.

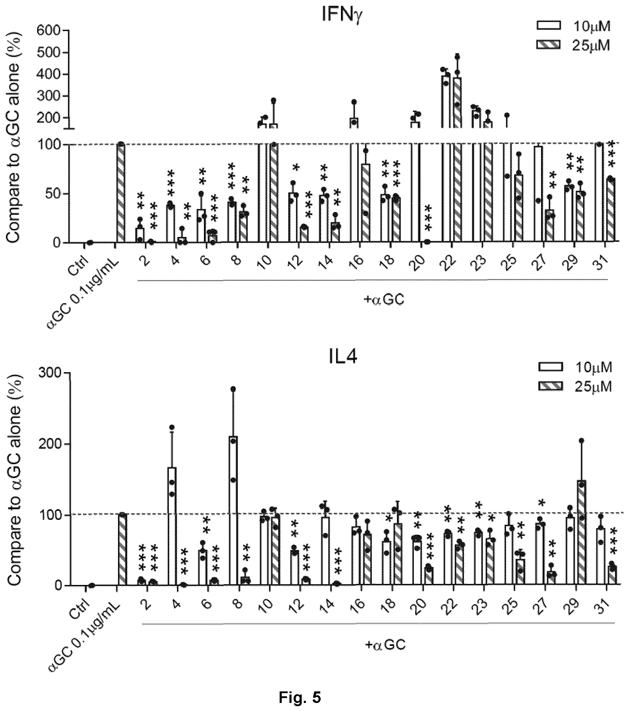

**Fig. 5****.** Shows suppression of αGalCer-induced inflammation (Splenocytes *in vitro).* Splenocytes were treated concurrently with the compounds of the invention and αGalCer to assess the suppressive function of compounds on αGalCer-induced inflammation, with several compounds showing prominent suppressive function.

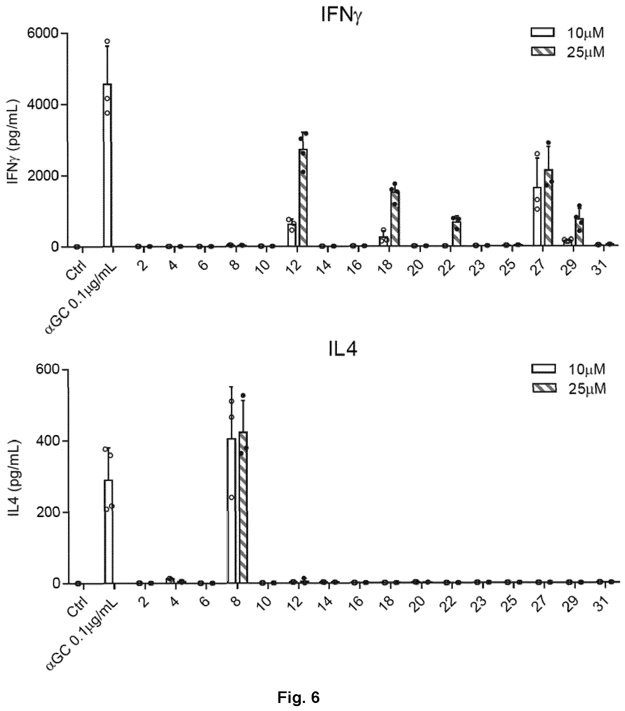

**Fig. 6****.** Shows immune-stimulating function of compounds (Splenocytes *in vitro).* Splenocytes were treated with the compounds of the invention alone to assess the immune-stimulating function of the compounds. Compounds **12, 18, 22, 27,** and **29** induced significant IFNγ secretion, and compound 8 induced significant IL-4 secretion.

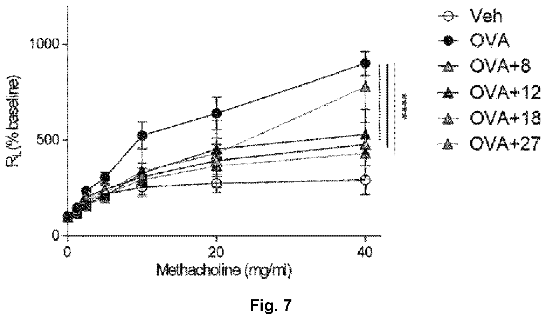

**Fig. 7****.** Shows suppression on ovalbumin (OVA)-induced airway hyperreactivity (AHR) *in vivo.* The capability of selected compounds **8, 12, 18,** and **27** in suppressing Th2 inflammation *in vivo* was assessed using the OAV-induced AHR mouse model. Compounds **8, 12**, and **18** were observed to suppress AHR.

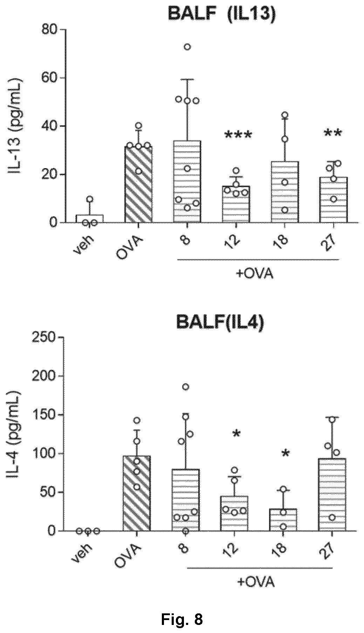

**Fig. 8****.** Shows suppression on OVA-induced airway inflammation *in vivo* (BALF). The bronchoalveolar lavage fluid (BALF) from mice in the OAV-induced AHR experiment was examined for th IL-13 and IL-4 levels. Compound **12** significantly suppressed both IL-13 and IL-4 levels. Compound **27** only showed significant suppression on IL-13, and compound **18** only showed significant suppression on IL-4.

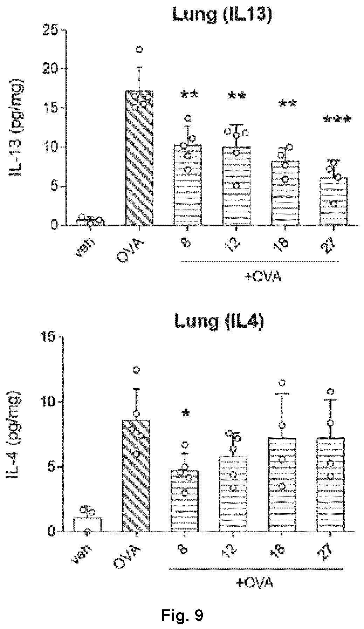

**Fig. 9****.** Shows suppression on OVA-induced airway inflammation *in vivo* (Lung protein). The lungs from mice in the OAV-induced AHR experiment was examined for their IL-13 and IL-4 protein level. Compound 12 significantly suppressed both IL-13 and IL-4 level. Compounds **8, 18,** and **27** only showed significant suppression on IL-13.

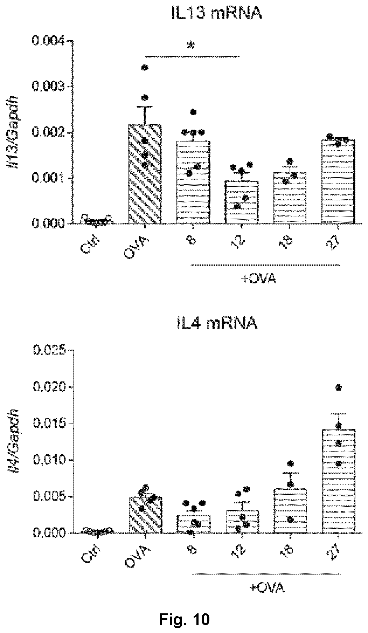

**Fig. 10****.** Shows suppression on OVA-induced airway inflammation *in vivo* (Lung mRNA). The lungs from mice in the OAV-induced AHR experiment was examined for their IL-13 and IL-4 mRNA levels. Compound **12** significantly suppressed IL-13 mRNA level.

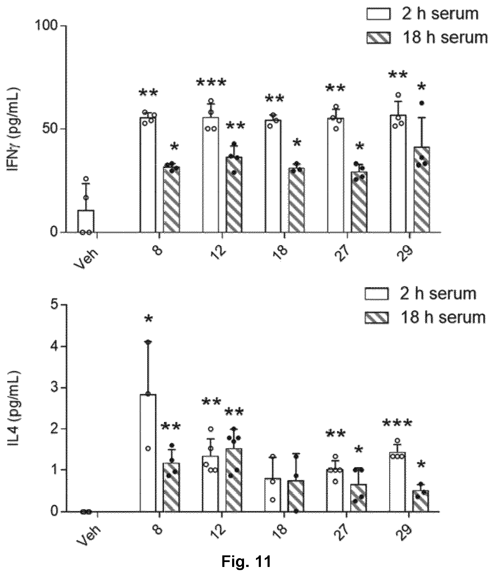

**Fig. 11****.** Shows the adjuvancy function of compounds *in vivo.* The adjuvancy capability of the selected compounds **8, 12, 18, 27,** and **29** *in vivo was* assessed by measuring the serum level of IFNγ and IL-4 induced after injecting them into B6 mice. Compounds **8, 12, 27,** and **29** showed significant capability in inducing both IFNγ and IL-4 2 hours after injection and still with significant detectable level of both cytokines at 18 hours after injection. Compound 18 only showed significant serum IFNγ level at both time points.

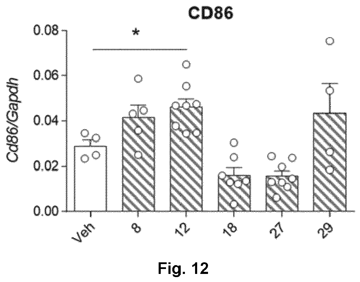

**Fig. 12****.** Shows that compound **12** promotes dendritic cell (DC) maturation *in vivo.* The capability of selected compounds **8, 12, 18, 27,** and **29** in promoting DC maturation *in vivo was* assessed by the mRNA level of DC-maturation marker CD86 in the spleen of mice injected with the compounds of the invention. Compound **12** was observed to induce significant CD86 mRNA increase.

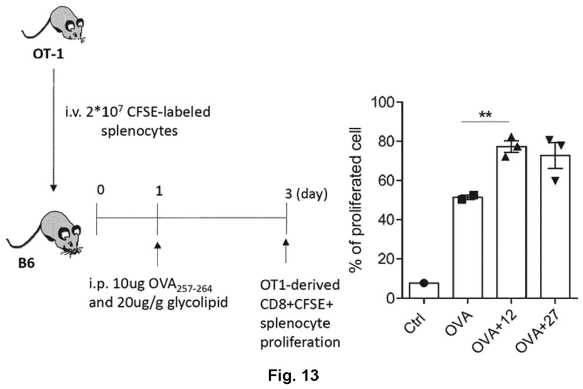

**Fig. 13****.** Shows that compound **12** promotes Th1 immune response *in vivo.* The adjuvancy capability of selected compounds **12** and **27** *in vivo* was assessed by their capability in promoting the proliferation of CD8⁺ T cells in the spleen in the OT-1 adoptive transfer mouse model as described in the scheme. Compound **12** was observed to induce significant CD8⁺ T cell proliferation, indicating compound **12** can promote Th1 immune response.

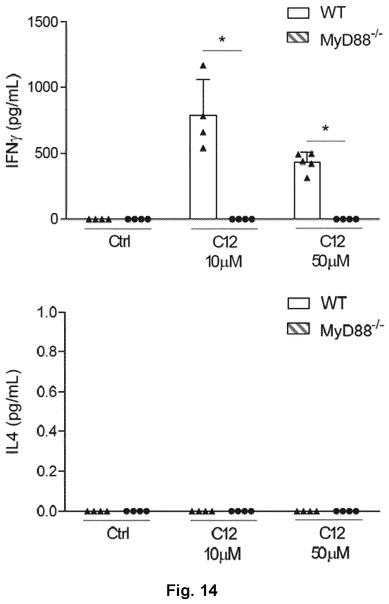

**Fig. 14****.** Shows that compound **12** adjuvancy is myeloid differentiation primary response 88 (MyD88)-dependent and Th1-biased (Splenocytes *in vitro).* The dependency of the compounds of the invention on functional MyD88 for biological function was examined using the representative compound **12,** with immune cells isolated from MyD88 knocked-out mice. We found that the ability of compound **12** to induce IFNγ in splenocytes isolated from MyD88 KO mice was completely abrogated. IL-4 was not detectable with compound **12** treatment, indicating a Th1-biased property.

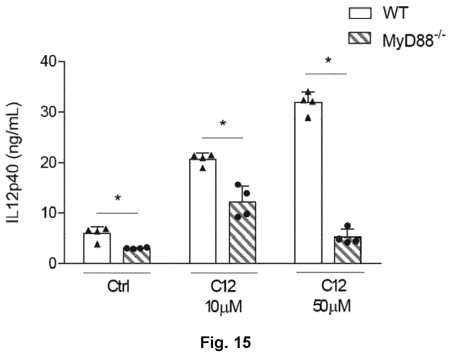

**Fig. 15****.** Shows compound **12** adjuvancy is MyD88-dependent (bone marrow-derived dendritic cells - BMDCs - *in vitro).* Continuing the examination on the dependency of the compounds of the invention on functional MyD88 for biological function, we found that the ability of bone marrow-derived dendritic cells (BMDCs) from MyD88 KO mice to produce IL-12p40 in response to compound **12** was severely curtailed as compared to BMDC from wild-type mice. The effect was dose-dependent, and the difference became more pronounced at a higher concentration.

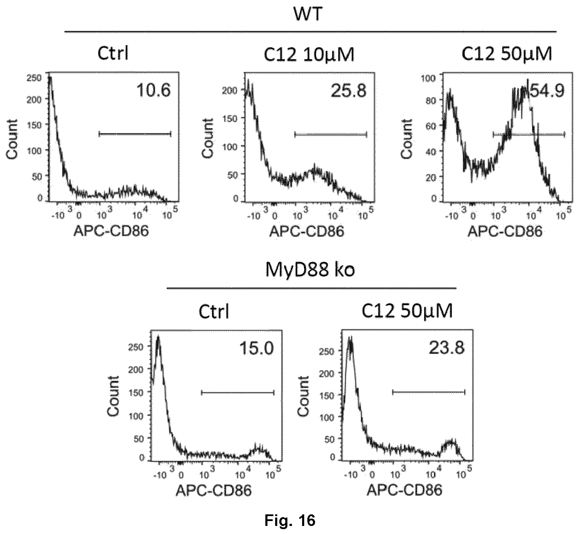

**Fig. 16****.** Shows that the promotion of BMDC maturation by compound **12** is MyD88-dependent. Continuing the examination on the dependency of the compounds of the invention on functional MyD88 for biological function, we found that the maturation of BMDCs induced by compound **12** treatment was impeded in BMDCs from MyD88 deficient mice.

### Examples

### Methods

All reagents and solvents used in the preparations disclosed in the following examples were purchased from commercial sources and used without further purification, unless otherwise specified. Thin-layer chromatography (TLC) was carried out on aluminum sheets coated with Silica gel 60 F₂₅₄ Merck with visualization by UV light (λ 254 nm) and by charring with 10% ethanolic H₂SO₄, 0.1% ethanolic ninhydrin and heating at 100 °C. With preparative purposes, column chromatography was carried out on Silice 60 A.C.C. Chromagel (SDS 70-200 and 35-70 µm). Optical rotations were measured at 20 ± 2 °C in 1 cm tubes on a Jasco P-2000 polarimeter using a sodium lamp (λ 589 nm). Elemental analyses were carried out at the Instituto de Investigaciones Químicas (Sevilla, Spain) using an elemental analyser Leco CHNS-932 o Leco TruSpec CHN. NMR experiments were performed at 300 (75.5), and 500 (125.7, 202) MHz with Bruker 300 ADVANCE and 500 DRX. 1D TOCSY, 2D COSY, HMQC and HSQC experiments were used to assist on NMR assignments. The chemical shift values are given in ppm (part per million), using the solvent as internal standard, tetramethylsilane (for CDCl₃). The values of the coupling constant (J) are measured in Hz. Abbreviations to indicate the multiplicities of the signals are: s (singlet), bs (broad singlet), d (doublet), t (triplet), q (quartet) and m (multiplet). Mass spectra (High-resolution mass spectra) were carried out on a Bruker Daltonics Esquire6000^{™} (LTQ-Orbitrap XL ETD). The samples were introduced via solid probe heated from 30 to 280 °C. ESI as ionization source (Electrospray Ionization) was used to which methanol was used as solvent. The samples were introduced via direct injection using a Cole-Parmer syringe at a flow rate of 2 µl/min. Ions were scanned between 300 and 3000 Da with a scan speed of 13000 Da/s at unit resolution using resonance ejection at the multipole resonance of one-third of the radio frequency (Ω = 781.25 kHz).

### Materials

In exemplary disclosures, the following precursors used in the synthesis of specific compounds of the invention, were accessed through known methods:
(1*R*)-1,2,3,4-Tetra-*O*-acetyl-5*N*,6*O*-oxomethylidenenojirimycin **(32)** was prepared as described in V. M. Diaz Perez, M. I. Garcia Moreno, C. Ortiz Mellet, J. Fuentes, J. C. Diaz Arribas, F. J. Cañada and J. M. Garcia Fernandez, J. Org. Chem., 2000, 65, 136-143. 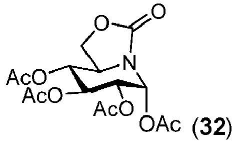
(1*R*)-1,2,3,4-Tetra-*O*-acetyl-5*N*,6*O*-oxomethylidenegalactonojirimycin (33) was prepared as described in P. Diaz Perez, M. I. García-Moreno, C. Ortiz Mellet and J. M. Garcia Fernandez, Eur. J. Org. Chem., 2005, 14, 2903-2913).

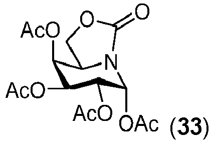

The biantennary lipid acceptor **34** was prepared as described in J. Bi, J. Wang, K. Zhou, Y. Wang, M. Fang and Y. Du, ACS Med. Chem. Lett., 2015, 6, 476-480.

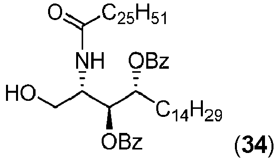

The sphyngosine precursor **35** was prepared as described in N. Veerapen, M. Brigl, S. Garg, V. Cerundolo, L. R. Cox, M. B. Brenner, G. S. Besra, Bioorg. Med. Chem. Lett., 2009, 19 4288-4291.

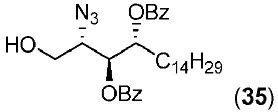

The L-serine acceptor **36** was synthetized as described in L. De Huang, H. J. Lin, P. H. Huang, W. C. Hsiao, L. V. Raghava Reddy, S. L. Fu and C. C. Lin, Org. Biomol. Chem., 2011, 9, 2492-2504.

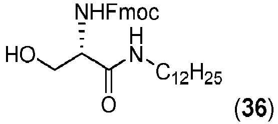

5-Azido-3-*O*-benzyl-5-deoxy-1,2-*O*-isopropylidene-α-D-glucofuranose **(42)** was preparerd as described in M. Aguilar-Moncayo, C. Ortiz Mellet, J. M. Garcia Fernandez, and M. I. García-Moreno. J. Org. Chem. 2009, 74, 3595-3598.

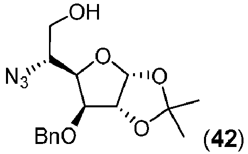

α-GalCer (aGC, KRN7000) was purchased from Funakoshi (Tokyo, Japan). RPMI 1640, DMEM, newborn calf serum (NBCS), and red blood cell (RBC) lysis buffer were purchased from Gibco (Waltham, MA, USA). Fetal bovine serum (FBS) was purchased from HyClone (Logan, UT, USA). Recombinant mouse IL-2 and GM-CSF were purchased from BioLegend (San Diego, CA, USA) and PeproTech (Rocky Hill, NJ, USA). Recombinant human IL-2 was purchased from eBioscience (San Diego, CA, USA). Ovalbumin (OVA) was purchased from Worthington Biochemicals (Freehold, NJ, USA).

### Mice

Eight- to ten-week-old C57BL/6 and BALB/c female mice were purchased from National Laboratory Animal Center (Taipei, Taiwan). All animals were housed under specific pathogen-free conditions.

### In vitro culture of mouse splenocytes and human peripheral blood mononuclear cells (PBMCs)

The whole spleen was surgically removed from a mouse and mechanically dissociated into a single cell suspension of splenocytes, which were then cultured in the complete RPMI 1640 medium (cRPMI). Human peripheral blood mononuclear cells (PBMCs) from healthy volunteers were collected from whole blood through gradient-centrifugation with Histopaque-1077 (Sigma-Aldrich).

### In vivo treatment

For ovalbumin (OVA) challenge, mice were sensitized intraperitoneally (i.p.) with 50 µg of OVA with 2% alhydrogel adjuvant (InvivoGen). Mice were challenged three times with 50 µg of OVA on day 7 post-sensitization, either daily or every other day. Mice were sacrificed the day after the last OVA challenge. The scheme of the OT-1 adoptive transfer mouse model for examining the proliferation of CD8⁺ T cells in adjuvanting OVA challenge is described in **Figure 13****.**

### Measurement of airway hyperreactivity (AHR)

Mice were anesthetized with 100 mg per kg body weight pentobarbital (Sigma-Aldrich), tracheotomised and mechanically ventilated via the FinePointe RC system (Buxco Research Systems, Wilmington, NC). Lung function was assessed by measuring lung resistance and dynamic compliance in response to increasing doses of aerosolized methacholine (1.25-40 mg/mL, Sigma-Aldrich).

### Collection and analysis of bronchoalveolar lavage fluid (BALF)

Upon exposure of the trachea, the lungs were lavaged twice with 1 mL of PBS supplemented with 2% fetal calf serum (FCS). BALF was pooled and bronchoalveolar (BAL) cells were pelleted by centrifugation and fixed onto cytospin slides. The slides were stained with Diff-Quik solution (Polysciences Inc) and BAL differential cell count was performed.

### Flow cytometry

Single cell suspensions were stained with fixable viability dye eFluor^{®} 780 (eBioscience) for 30 mins at 4°C and Fc receptors were blocked with anti-CD16/32 (BioLegend) blocking antibody prior to surface staining with monoclonal antibodies.

### Statistics

Data were analyzed using the GraphPad Prism (GraphPad Software, San Diego, CA, USA). Statistical analysis was determined using the Student's t-test and the two-way analysis of variance (ANOVA).

### EXAMPLE 1. Synthesis of compound 1.

To a solution of **32** (20 mg, 0.05 mmol) and **34** (0.10 mmol), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:4→1:3→1:2 EtOAc-cyclohexane). Yield: 48 mg (66%). R*_{f}* = 0.44 (1:2 EtOAc-cyclohexane). [α]_{D} +42.2 (c 1.0 in DCM). ¹H NMR (500 MHz, CDCl₃): δ 8.01-7.34 (m, 10 H, CH-arom), 6.84 (d, 1 H, *J*_{NH,2'} = 9.3 Hz, NH), 5.70 (dd, 1 H, *J*_{3',4'} = 9.5 Hz, *J*_{2',3'} = 3.0 Hz, H-3'), 5.46 (t, 1 H, *J*_{2,3} = *J*_{3,4} = 10.0 Hz, H-3), 5.31 (dt, 1 H, *J*_{4',5'} = 9.3 Hz, H-4'), 5.19 (d, 1 H, *J*_{1,2} = 3.8 Hz, H-1), 4.94-4.87 (m, 2 H, H-2, H-4), 4.61 (m, 1 H, H-2'), 4.40 (t, 1 H, *J*_{5,6a} = *J*_{6a,6b} = 8.9 Hz, H-6a), 4.20 (dd, 1 H, *J*_{5,6b} = 7.4 Hz, H-6b), 3.90 (m, 1 H, H-5), 3.71 (dd, 1 H, *J*_{1a',1b'} = 11.0 Hz, *J*_{1a',2'} = 2.8 Hz, H-1a'), 3.60 (dd, 1 H, *J*_{1a',2'} = 3.81 Hz, H-1b'), 2.36 (t, 2 H, ³*J*_{H,H} = 7.4 Hz, COCH₂), 2.09, 2.07, 1.97 (3 s, 9 H, CH₃CO), 1.90-1.23 (m, 75 H, CH₂, CH₂C*H₃*). ¹³C NMR (125.7 MHz, CDCl₃): δ 173.2, 169.9, 169.8 (CO ester), 155.0 (CO carbamate), 133.4-128.4 (C-arom), 79.9 (C-1), 74.1 (C-4'), 72.5 (C-4), 71.8 (C-3'), 70.0 (C-4), 69.3 (C-3), 68.1 (C-1'), 66.8 (C-6), 51.8 (C-5), 48.6 (C-2'), 36.7 (COCH₂), 31.9-14.1 (CH₃CO, CH₂, CH₂CH₃). ESIMS: m/z 1239.80 [M + Na]⁺. HRMS (ESI) calcd for C₇₁H₁₁₂N₂O₁₄Na 1239.8006. Found 1239.8006.

### EXAMPLE 2. Synthesis of compound 2.

Compound **2** was obtained by treatment of a solution of **1** in MeOH (2.6 mL) with 1 M NaOMe in MeOH (42 µL), followed by neutralization with Amberlite^{®} IR120 hydrogen form, filtration, evaporation and freeze drying. Column chromatography 100:10:1 DCM-MeOH-H₂O. Yield: 26 mg (quant.). R*_{f}* = 0.64 (70:10:1 DCM-MeOH-H₂O). [α]_{D} +16.76 (c 0.81 10:1 DCM-MeOH). ¹H NMR (700 MHz, 2:1 CDCl₃-CD₃OD): δ 5.08 (d, 1 H, *J*_{1,2} = 4.2 Hz, H-1), 4.50 (t, 1 H, *J*_{5,6a} = *J*_{6a,6b} = 8.6 Hz, H-6a), 4.23 (dd, 1 H, *J*_{5,6b} = 6.1 Hz, H-6b), 4.20 (m, 1 H, H-2'), 3.80 (dd, 1 H, *J*_{1a',1b'} = 10.3 Hz, *J*_{1a',2'} = 4.3 Hz, H-1a'), 3.72 (m, 1 H, H-5), 3.66 (dd, 1 H, *J*_{1b',2'} = 4.5 Hz, H-1b'), 3.60 (t, 1 H, *J*_{2,3} = *J*_{3,4} = 9.3 Hz, H-3), 3.55 (t, 1 H, *J*_{2',3'} = *J*_{3',4'} = 6.0 Hz. H-4'), 3.50 (m, 1 H, H-3'), 3.42 (dd, 1 H, *J*_{2,3} = 9.7 Hz, H-2), 3.29 (t, 1 H, *J*_{4,5} = 9.3 Hz, H-4), 2.19 (t, 2 H, ³*J*_{H,H} = 7.4 Hz, COCH₂), 1.24 (m, 72 H, CH₂), 0.85 (t, 6 H, ³*J*_{H,H} = 6.9 Hz, CH₃). ¹³C NMR (125.7 MHz, 2:1 CDCl₃-CD₃OD): δ 173.4 (CO ester), 156.1 (CO carbamate), 81.7 (C-1), 73.9 (C-3'), 73.2 (C-4), 72.4 (C-3), 71.3 (C-4'), 70.6 (C-2), 67.7 (C-1'), 66.3 (C-6), 52.4 (C-5), 49.2 (C-2'), 35.6-13.0 (CH₂, CH₂*C*H₃). HRMS (ESI): *m*/*z* 905.71 [M + Na]⁺. HRMS (ESI) calcd for C₅₁H₉₈N₂O₉Na 905.7165. Found 905.7147.

### EXAMPLE 3. Synthesis of compound 3.

Compound 3 was obtained by conjugation of glycosyl donor 32 and lipid acceptor 37. Acceptor 37 was prepared from 35 according to the following Scheme 6, through the procedure described hereinafter.

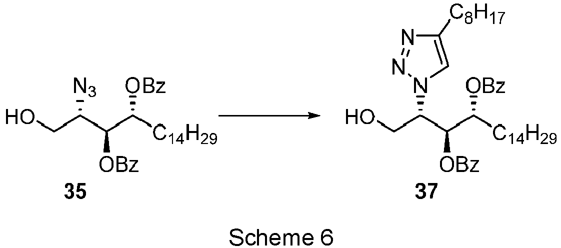

To a solution of the azido derivative 35 (200 mg, 0.583 mmol) in DMF-H₂O 4:1 (5.8 mL), 1-decyne (157 µL, 0.874 mmol), CuSO₄ (13 mg, 0.087 mmol) and ascorbic acid (30 mg, 0.174 mmol) were added and the solution was stirred overnight. The solvent was evaporated and the resulting residue was purified by column chromatography using 1:4 EtOAc-cyclohexane as eluent to afford **37**. Yield: 361 mg (90%). R*_{f}* = 0.4 (1:4 EtOAc-cyclohexane). ¹H NMR (500 MHz, CDCl₃): δ = 8.05-7.30 (m, 11 H, CH-arom, CH-triazole), 5.86 (dd, 1 H, *J*_{3,4} = 8.7 Hz, *J*_{2,3} = 3.1 Hz, H-3), 5.22 (s, 1 H, OH), 4.96 (m, 2 H, H-2, H-4), 4.07 (dd, 1 H, *J*_{1a,1b} = 12.5 Hz, *J*_{1a,2} = 4.6 Hz, H-1a), 3.96 (dd, 1 H, *J*_{1b,2} = 2.7 Hz, H-1b), 2.66 (t, 2 H, ³*J*_{H,H} = 7.4 Hz, CH₂), 1.68-0.80 (m, 49 H, CH₂, CH₂CH₃). ¹³C NMR (125.7 MHz, CDCl₃): δ 171.2, 166.1 (CO), 149.0 (C-4 triazole), 136.5-128.4 (C-arom), 121.1 (C-5 triazole), 73.3 (C-3), 72.1 (C-4), 61.6 (C-1), 61.3 (C-2), 31.9-14.0 (CH₂, CH₂*C*H₃). HRMS (ESI) calcd for C₄₂H₆₄N₃O₅ 690.4840. Found 690.4838.

In a subsequent step, to a solution of **32** (20 mg, 0.05 mmol) and **37** (0.10 mmol) in DCM (1.2 mL), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford the target compound 3. Yield: 33 mg (65%). R*_{f}* = 0.5 (1:2 EtOAc-cyclohexane). [α]_{D} +13.04 (c 1.0 in DCM). ¹H NMR (500 MHz, CDCl₃): δ 7.94-7.33 (m, 10 H, CH-arom), 7.60 (s, 1 H, CH-triazole), 5.94 (dd, 1 H, *J*_{3',4'} = 8.5 Hz, *J*_{2',3'} = 3.5 Hz, H-3'), 5.31 (t, 1 H, *J*_{2,3} = *J*_{3,4} = 10.0 Hz, H-3), 5.20 (m, 1 H, H-2'), 5.17 (d, 1 H, *J*_{1,2} = 3.8 Hz, H-1), 4.95 (dt, 1 H, *J*_{4',5'} = 9.3 Hz, H-4'), 4.75 (m, 2 H, H-2, H-4), 4.31 (t, 1 H, *J*_{5,6a} = *J*_{6a,6b} = 8.9 Hz, H-6a), 4.11 (dd, 1 H, *J*_{5,6b} = 7.5 Hz, H-6b), 3.93 (m, 2 H, H-1a', H-1b'), 3.49 (m, 1 H, H-5), 2.69 (t, 2 H, ³*J*_{H,H} = 7.4 Hz, COCH₂), 1.95, 1.94, 1.89 (3 s, 9 H, CH₃CO), 1.62-0.80 (m, 46 H, CH₂, CH₂CH₃). ¹³C NMR (125.7 MHz, CDCl₃): δ 169.9, 169.8, 169.4, 165.9, 164.9 (CO ester), 155.4 (CO carbamate), 149.2 (C-4 triazole), 133.7-128.2 (C-arom), 120.8 (C-5 triazole), 79.5 (C-1), 72.9 (C-4'), 72.3 (C-4), 71.2 (C-3'), 69.8 (C-2), 68.8 (C-3), 66.9 (C-1'), 66.8 (C-6), 59.7 (C-2'), 51.5 (C-2'), 31.9 (COCH₂), 30.1-22.6 (CH₃CO, CH₂, CH₂*C*H₃). HRMS (ESI) calcd for C₅₅H₇₈N₄O₁₃Na 1025.5458. Found 1025.5450.

### EXAMPLE 4. Synthesis of compound 4.

Compound **4** was obtained by treatment of a solution of 3 in MeOH (2.6 mL) with 1 M NaOMe in MeOH (42 µL), followed by neutralization with Amberlite^{®} IR120 hydrogen form, filtration, evaporation and freeze drying. Column chromatography 100:10:1 DCM-MeOH-H₂O. Yield: 22 mg (quant.). R*_{f}* = 0.6 (70:10:1 DCM-MeOH-H₂O). [α]_{D} +15.8 (c 1.0 in MeOH). ¹H NMR (500 CD₃OD): δ 7.88 (s, 1 H, CH-triazole), 5.12 (q, 1 H, *J*_{1',2'} = *J*_{2',3'} = 5.7 Hz, H-2'), 5.05 (d, 1 H, *J*_{1,2} = 4.2 Hz, H-1), 4.51 (t, 1 H, *J*_{5,6a} = *J*_{6a,6b} = 8.6 Hz, H-6a), 4.22 (dd, 1 H, *J*_{5,6b} = 6.1 Hz, H-6b), 4.18 (d, 1 H, *J*_{3',4'} = 5.8 Hz, H-4'), 3.86 (dd, 1 H, *J*_{2,3} = 8.0 Hz, *J*_{3,4} = 5.1 Hz, H-3), 3.50 (m, 3 H, H-5, H1a', H1b'), 3.40 (dd, 1 H, H-2), 3.37(m, 1 H, H-3'), 3.29 (t, 1 H, *J*_{4,5} = 9.3 Hz, H-4), 2.71 (t, 2 H, ³*J*_{H,H} = 7.4 Hz, COCH₂), 1.69-0.91 (m, 46 H, CH₂, CH₂CH₃). ¹³C NMR (125.7 MHz, CD₃OD): δ 157.1 (CO carbamate), 147.7 (C-4 triazole), 121.7 (C-5 triazole), 82.9 (C-1), 74.5 (C-3), 74.1 (C-4), 73.0 (C-1'), 71.5 (C-3', C-2), 67.4 (C-4'), 67.1 (C-6), 61.7 (C-2'), 53.4 (C-5), 32.4-13.0 (CH₂, CH₂*C*H₃). ESIMS: *m*/*z* 669.37 [M + H]⁺. Anal. calcd. for C₃₅H₆₄N₄O₈S: C 62.85, H 9.64, N 8.38. Found C 63.23, H 9.69, N 8.31.

### EXAMPLE 5. Synthesis of compound 5.

To a solution of **33** (20 mg, 0.05 mmol) and **34** (0.10 mmol) in DCM (1.2 mL), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1 :4→1 :3→1:2 EtOAc-cyclohexane). Yield: 44 mg (77%). R*_{f}* = 0.47 (1:2 EtOAc-cyclohexane). [α]_{D} +41.41 (c 1.0 in DCM). ¹H NMR (500 MHz, CDCl₃): δ 8.01-7.47 (m, 10 H, CH-arom), 6.42 (d, 1 H, *J*_{NH,2'} = 9.6 Hz, NH), 5.63 (dd, 1 H, *J*_{3',4'} = 9.1 Hz, *J*_{2',3'} = 2.9 Hz, H-3'), 5.40 (t, 1 H, *J*_{3,4} = *J*_{4,5} = 2.4 Hz, H-4), 5.24-5.21 (m, 2 H, H-4', H-1), 5.25 (dd, 1 H, *J*_{2,3} = 10.8 Hz, H-3), 5.13 (dd, 1 H, *J*_{1,2} = 4.3 Hz, H-2), 4.64-4.58 (m, 1 H, H-2'), 4.36 (t, 1 H, *J*_{5,6a} = *J*_{6a,6b} = 9.0 Hz, H-6a), 4.19-4.15 (m, 1 H, H-5), 4.00 (dd, 1 H, *J*_{5,6b} = 5.9 Hz, H-6b), 3.71 (dd, 1 H, *J*_{1a',1b'} = 11.0 Hz, *J*_{1a',2'} = 2.9 Hz, H-1a'), 3.62 (dd, 1 H, *J*_{1a',2'} = 3.9 Hz, H-1b'), 2.30 (t, 2 H, ³*J*_{H,H} = 7.7 Hz, COCH₂), 2.14, 1.99, 1.95 (3 s, 9 H, CH₃CO), 1.80-0.80 (m, 75 H, CH₂, CH₂CH₃). ¹³C NMR (125.7 MHz, CDCl₃): δ 173.1 (CO amide), 170.2, 169.9 (CO ester), 155.8 (CO carbamate), 133.5-128.4 (C-arom), 80.6 (C-1), 74.0 (C-4'), 72.3 (C-3'), 68.6 (C-1'), 68.0 (C-4), 67.9 (C-3), 66.9 (C-2), 63.1 (C-6), 50.7 (C-5), 48.7 (C-2'), 36.8-14.1 (CH₃CO, CH₂, CH₂*C*H₃). HESIMS: *m*/*z* 1239.80 [M + Na]⁺. HRMS (ESI) calcd for C₇₁H₁₁₂N₂O₁₄Na 1239.8006. Found 1239.7996.

### EXAMPLE 6. Synthesis of compound 6.

Compound 6 was obtained by treatment of a solution of 5 in MeOH (2.6 mL) with 1 M NaOMe in MeOH (42 µL), followed by neutralization with Amberlite^{®} IR120 hydrogen form, filtration, evaporation and freeze drying. Column chromatography 100:10:1 DCM-MeOH-H₂O. Yield: 18 mg (quant.). R*_{f}* = 0.65 (70:10:1 DCM-MeOH-H₂O). [α]_{D} +7.9 (c 0.6 in DCM-MeOH). ¹H NMR (500 MHz, 6:1 CDCl₃-CD₃OD): δ 5.09 (d, 1 H, *J*_{1,2} = 4.1 Hz, H-1), 4.41 (dd, 1 H, *J*_{5,6a} = *J*_{6a,6b} = 8.9 Hz, H-6a), 4.31 (t, 1 H, H-6b), 4.17 (q, 1 H, *J*_{1',2'} = *J*_{2',3'} = 4.6 Hz, H-2'), 3.94 (m, 1 H, H-5), 3.82 (dd, 1 H, *J*_{1a',1b'} = 10.3 Hz, H-1a'), 3.81 (t, 1 H, *J*_{3,4} = *J*_{4,5} = 2.2 Hz, H-4), 3.75 (dd, 1 H, *J*_{2,3} = 9.7 Hz, *J*_{1,2} = 4.1 Hz, H-2), 3.69 (dd, 1 H, H-3), 3.64 (m, 1 H, H-1b'), 3.54-3.42 (m, 2 H, H-4', H-3'), 2.14 (t, 2 H, ³*J*_{H,H} = 7.4 Hz, COCH₂), 1.63-0.82 (m, 75 H, CH₂, CH₂CH₃). ¹³C NMR (150.9 MHz, 6:1 CDCl₃-CD₃OD): δ 173.4 (CO amide), 156.1 (CO carbamate), 82.7 (C-1), 74.9 (C-3'), 72.2 (C-4'), 69.8 (C-3), 68.4 (C-4), 68.4 (C-1'), 67.9 (C-2), 63.5 (C-6), 52.5 (C-5), 50.2 (C-2'), 36.5 (COCH₂), 34.0-13.8 (CH₂, CH₂*C*H₃). ESIMS: *m*/*z* 905.71 [M + H]⁺. HRMS (ESI) calcd for C₅₁H₉₈N₂O₉Na 905.7165. Found 905.7151.

### EXAMPLE 7. Synthesis of compound 7.

To a solution of **33** (20 mg, 0.05 mmol) and **37** (0.10 mmol) in DCM (1.2 mL), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane). Yield: 35 mg (70%). R*_{f}*= 0.47 (1:2 EtOAc-cyclohexane). [α]_{D} +14.10 (c 1.0 in DCM). ¹H NMR (500 MHz, CDCl₃): δ 7.94-7.36 (m, 10 H, CH-arom), 5.91 (dd, 1 H, *J*_{3',4'} = 7.8 Hz, *J*_{2',3'} = 4.1 Hz, H-3'), 5.28 (bd, 2 H, H-1, H-4), 5.16 (m, 1 H, H-2'), 5.10 (dd, 1 H, , *J*_{2,3} = 10.8 Hz, *J*_{1,2} = 4.3 Hz, H-2), 4.98 (m, 2 H, H-3, H-4'), 4.29 (t, 1 H, *J*_{5,6a} = *J*_{6a,6b} = 9.1 Hz, H-6a), 4.01 (dd, 1 H, *J*_{5,6b} = 3.1 Hz, H-6b), 3.91 (m, 2 H, H-1a', H-1b'), 3.79 (m, 1 H, H-5), 2.66 (m, 2 H, CH₂), 2.07, 1.91, 1.86 (3 s, 9 H, CH₃CO), 1.54-0.80 (m, 46 H, CH₂, CH₂*C*H₃). ¹³C NMR (125.7 MHz, CDCl₃): δ 170.1, 170.0, 169.6, 166.0, 164.9 (CO ester), 155.6 (CO carbamate), 149.2 (C-4 triazole), 139.1-128.4 (C-arom), 120.3 (C-5 triazole), 80.1 (C-1), 72.9 (C-4'), 71.6 (C-4), 67.9 (C-3'), 67.8 (C-2), 66.9 (C-3), 66.7 (C-1'), 63.0 (C-6), 59.9 (C-2'), 50.4 (C-5), 31.9 (COCH₂), 30.0-14.1 (CH₃CO, CH₂, CH₂*C*H₃). HRMS (ESI) calcd for C₅₅H₇₈N₄O₁₃Na 1025.5458. Found 1025.5450.

### EXAMPLE 8. Synthesis of compound 8.

Compound **8** was obtained by treatment of a solution of **7** in MeOH (2.6 mL) with 1 M NaOMe in MeOH (42 µL), followed by neutralization with Amberlite^{®} IR120 hydrogen form, filtration, evaporation and freeze drying. Column chromatography 100:10:1 DCM-MeOH-H₂O. Yield: 23 mg (quant.). R*_{f}* = 0.6 (70:10:1 DCM-MeOH-H₂O). [α]_{D} +16.2 (c 1.0 in MeOH). ¹H NMR (500 CD₃OD): δ 7.89 (s, 1 H, CH-triazole), 5.11 (m, 2 H, H-1, H-2'), 4.38 (d, 2 H, H-4, H-6a), 4.16 (d, 2 H, *J*_{5,6b} = 5.7 Hz, H-3, H-6b), 3.87 (d, 1 H, *J*_{3',4'} = 5.1 Hz, H-4'), 3.79 (m, 3 H, H-1a', H-1b', H-3'), 3.66 (dd, 1 H, H-2), 3.38 (m, 1 H, H-5), 2.71 (t, 2 H, ³*J*_{H,H} = 7.4 Hz, COCH₂), 1.69-0.91 (m, 46 H, CH₂, CH₂CH₃). ¹³C NMR (125.7 MHz, CD₃OD): δ 157.8 (CO carbamate), 147.7 (C-4 triazole), 121.7 (C-5 triazole), 83.1 (C-1), 74.4 (C-3), 71.5 (C-4), 69.9 (C-1'), 69.0 (C-3') 67.6 (C-2), 67.2 (C-4'), 63.7 (C-6), 61.8 (C-2'), 52.9 (C-5), 32.3-13.0 (CH₂, CH₂*C*H₃). ESIMS: *m*/*z* 669.33 [M + H]⁺. Anal. calcd. for C₃₅H₆₄N₄O₈S: C 62.85, H 9.64, N 8.38. Found C 63.30, H 8.71, N 8.33.

### EXAMPLE 9. Synthesis of compound 9.

Compound **9** was obtained by conjugation of glycosyl donor **33** and lipid acceptor **38.** Acceptor **38** was prepared from **35** according to the following Scheme 7, through the procedure described hereinafter.

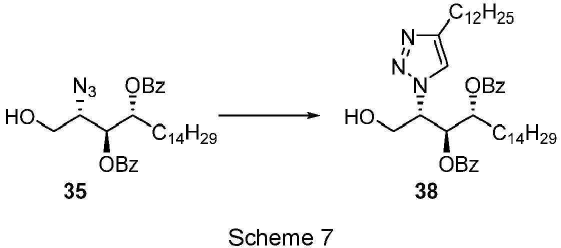

To a solution of the azido derivative **35** (150 mg, 0.272 mmol) in DMF-H₂O 4:1 (3.7 mL), 1-tetradecyne (133 µL, 0.544 mmol), CuSO₄ (10 mg, 0.041 mmol) and ascorbic acid (16 mg, 0.082 mmol) were added and the solution was stirred overnight. The solvent was evaporated and the resulting residue was purified by column chromatography using 1:4 EtOAc-cyclohexane as eluent to afford **38.** Yield: 120 mg (60%). R*_{f}*= 0.4 (1:4 EtOAc-cyclohexane). ¹H NMR (300 MHz, CDCl₃): δ = 7.98-7.18 (m, 11 H, CH-arom, CH-triazole), 5.86 (dd, 1 H, *J*_{3,4} = 8.7 Hz, *J*_{2,3} = 3.1 Hz, H-3), 4.96 (m, 2 H, H-2, H-4), 4.06 (dd, 1 H, *J*_{1a,1b} = 12.5 Hz, *J*_{1a,2} = 4.6 Hz, H-1a), 3.95 (dd, 1 H, *J*_{1b,2} = 2.7 Hz, H-1b), 2.65 (t, 2 H, ³*J*_{H,H} = 7.4 Hz, CH₂), 1.60-0.80 (m, 58 H, CH₂, CH₂CH₃). ¹³C NMR (75.5 MHz, CDCl₃): δ 166.1, 165.9 (CO), 133.9-128.4 (C-arom), 121.1 (C-triazole), 73.3 (C-3), 72.1 (C-4), 61.6 (C-1), 61.3 (C-2), 31.9-14.0 (CH₂, CH₂*C*H₃). HRMS (ESI) calcd for C₄₆H₇₂N₃O₅ 746.5466. Found 746.5461.

In a subsequent step, to a solution of **33** (27 mg, 0.07 mmol) and **38** (0.10 mmol) in DCM (1.2 mL), BF₃·OEt₂ (32 µL, 0.35 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford the target compound **9.** Yield: 54 mg (70%). R*_{f}* = 0.50 (1:2 EtOAc-cyclohexane). [α]_{D} +17.15 (c 1.0 in DCM). ¹H NMR (500 MHz, CDCl₃): δ 7.94-7.36 (m, 11 H, CH-arom, C-triazole), 5.90 (dd, 1 H, *J*_{3',4'} = 7.2 Hz, *J*_{2',3'} = 4.2 Hz, H-3'), 5.28 (bd, 2 H, H-1, H-4), 5.16 (m, 1 H, H-2'), 5.08 (dd, 1 H, *J*_{2,3} = 10.8 Hz, *J*_{1,2} = 4.3 Hz, H-2), 4.98 (m, 2 H, H-3, H-4'), 4.29 (t, 1 H, *J*_{5,6a} = *J*_{6a,6b} = 9.1 Hz, H-6a), 4.01 (dd, 1 H, *J*_{5,6b} = 2.5 Hz, H-6b), 3.91 (m, 2 H, H-1a', H-1b'), 3.79 (m, 1 H, H-5), 2.66 (m, 2 H, CH₂), 2.07, 1.91, 1.86 (3 s, 9 H, CH₃CO), 1.58-0.80 (m, 55 H, CH₂, CH₂CH₃). ¹³C NMR (125.7 MHz, CDCl₃): δ 170.1, 170.0, 169.6, 166.0, 164.9 (CO), 155.6 (CO carbamate), 149.2-128.5 (C-arom), 120.4 (C-triazole), 80.1 (C-1), 72.8 (C-4'), 71.6 (C-4), 67.9 (C-3'), 67.8 (C-2), 66.9 (C-3), 66.7 (C-1'), 63.0 (C-6), 59.9 (C-2'), 50.4 (C-5), 31.9 (COCH₂), 29.7-14.1 (CH₃CO, CH₂, CH₂*C*H₃). HRMS (ESI) calcd for [C₅₉H₈₆N₄NaO_{13]}⁺ 1081.6084; found 1081.6082.

### EXAMPLE 10. Synthesis of compound 10.

Compound **10** was obtained by treatment of a solution of **9** in MeOH (2.6 mL) with 1 M NaOMe in MeOH (42 µL), followed by neutralization with Amberlite^{®} IR120 hydrogen form, filtration, evaporation and freeze drying. Column chromatography using 100:10:1 DCM-MeOH-H₂O. Yield: 65 mg (quant.). R*_{f}* = 0.6 (70:10:1 DCM-MeOH-H₂O). [α]_{D} +10.2 (c 1.0 in MeOH). ¹H NMR (500 CD₃OD): δ 7.87 (s, 1 H, CH-triazole), 5.08 (m, 2 H, H-1, H-2'), 4.36 (d, 2 H, H-4, H-6a), 4.14 (d, 2 H, *J*_{5,6b} = 5.7 Hz, H-3, H-6b), 3.86 (dd, 1 H, *J*_{3',4'} = 5.4 Hz, H-4'), 3.78 (m, 3 H, H-1a', H-1b', H-3'), 3.65 (dd, 1 H, *J*_{2,3} = 9.9 Hz, *J*_{1,2} = 3.1 Hz H-2), 3.36 (m, 1 H, H-5), 2.69 (t, 2 H, ³*J*_{H,H} = 7.4 Hz, COCH₂), 1.67-0.91 (m, 57 H, CH₂, CH₂C*H₃*). ¹³C NMR (125.7 MHz, CD₃OD): δ 161.7 (CO carbamate), 151.6, 125.6 (C-triazole), 87.0 (C-1), 78.3 (C-3), 75.4 (C-4), 73.8 (C-1'), 72.9 (C-3') 71.5 (C-2), 71.5 (C-4'), 67.6 (C-6), 65.7 (C-2'), 56.9 (C-5), 35.6-16.9 (CH₂, CH₂C*H₃*). ESIMS: *m*/*z* 725.65 [M + H]⁺.

### EXAMPLE 11. Synthesis of compound 11.

The synthesis of compound **11** comprised a first conjugation between glycosyl donor 32 and acceptor 36 to give intermediate 39

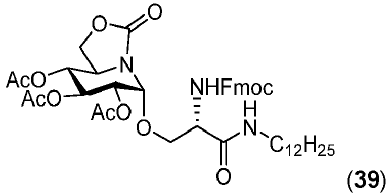

To a solution of **32** (20 mg, 0.05 mmol) in dry DCM (1.2 mL), the *N*-modified-L-serine **36** (0.10 mmol) and BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford 39. Yield: 106 mg (63%). *R_{f}* 0.60 (2:1 EtOAc-cyclohexane). [α]_{D} +37.46 (c 1.0 in DCM). ¹H NMR (300 MHz, CDCl₃): δ = 7.77-7.20 (m, 12 H, CH-arom), 6.30 (t, 1 H, *J*_{NH,CH2} = 5.6 Hz, N*H*CH₂), 5.78 (d. 1 H, *J*_{NH,CH} = 8.0 Hz, NHFmoc), 5.48 (m, 2 H, H-1, H-3), 4.94 (m, 1 H, H-4), 4.92 (dd, 1 H, *J*_{2,3} = 6.2 Hz, *J*_{1,2} = 4.6 Hz, H-2), 4.44 (m, 4 H, H-6a, OCH₂C*H*, C*H₂*Fmoc), 4.25 (m, 2 H, H-5, CHFmoc), 3.98 (m, 1 H, H-6b), 3.78 (m, 2 H, OC*H₂*CH), 3.27 (m, 2 H, N*H*CH₂), 2.09, 2.05, 2.04 (3 s, 9 H, CH₃CO), 1.55-0.89 (m, 25 H, CH₂, CH₂C*H₃*). ¹³C NMR (75.5 MHz, CDCl₃): δ = 169.9, 169.7, 169.0 (CO), 155.7 (CO carbamate), 143.6-120.0 (C-arom),79.5 (C-1), 72.4 (O*C*H₂CH), 70.2 (C-4), 69.1 (C-2), 68.9 (C-3), 67.3 (C-6), 67.0 (*C*H₂Fmoc), 54.2 (O*C*H₂CH), 51.7 (CHFmoc), 47.0 (C-5), 39.9 (NHCH₂), 31.9-14.1 (*C*H₃CO, CH₂, CH₂C*H₃*). ESIMS: *m*/*z* = 830.38 [M + Na]⁺. HRMS (ESI) calcd for [C₄₃H₅₇O₁₂N₃Na]⁺ 830.3834. Found 830.3824.

In a subsequent step, to a stirred solution of **39** (100 mg, 0.131 mmol) in DMF (1.5 mL), piperidine (131 µL, 1 mL/mmol) was added. After 1 h the mixture was evaporated, the crude product was dissolved in DMF (1.8 mL) and dodecanoyl chloride (47 µL, 0.196 mmol) and Et₃N (37 µL, 0.262 mmol) were added. The mixture was stirred for 16 h and concentrated. The resulting residue was purified by column chromatography (1:1 EtOAc- cyclohexane) to give the target compound **11.** Yield: 75 mg (65%, two steps). *R_{f}* 0.40 (1:1 EtOAc- cyclohexane). [α]_{D} +27.98 (c 1.0 in DCM). ¹H NMR (300 MHz, CDCl₃): δ = 6.36 (d, 1 H, *J*_{NH,CH} = 7.8 Hz, NHCH), 6.29 (bt, 1 H, *J*_{NH,CH} = 5.6 Hz, N*H*CH₂), 5.46 (d, 1 H, *J*_{1,2} = 4.7 Hz, H-1), 5.45 (m, 1 H, H-3), 4.94 (t, 1 H, *J*_{4,5} = 9.4 Hz, H-4), 4.91 (bdd, 1 H, *J*_{2,3} = 9.9 Hz, H-2), 4.60 (m, 1 H, OCH₂C*H*) , 4.49 (t, 1 H, *J*_{5,6a} = *J*_{5,6b} = 8.5 Hz, H-6a), 4.27 (bdd, 1 H, H-6b), 3.99 (m, 1 H, H-5), 3.76 (m, 2 H, OC*H₂*CH), 3.28 (m, 2 H, N*H*CH₂), 2.24 (m, 2 H, COCH₂), 2.11, 2.06, 2.04 (3 s, 9 H, CH₃CO), 1.64-0.89 (m, 48 H, CH₂, CH₂C*H₃*). ¹³C NMR (75.5 MHz, CDCl₃): δ = 173.3, 169.9, 169.6, 169.2 (CO), 155.8 (CO carbamate), 79.6 (C-1), 72.5 (C-4), 70.2 (C-2), 68.9 (C-3), 68.8 (O*C*H₂CH), 67.1 (C-6), 52.3 (O*C*H₂CH), 51.8 (C-5), 39.8 (NHCH₂), 36.5 (CO*C*H₂), 31.9-14.1 (*C*H₃CO, CH₂, CH₂C*H₃*). ESIMS: *m*/*z =* 768.50 [M + H]⁺. HRMS (ESI) calcd for [C₄₀H₆₉O₁₁N₃Na]⁺ 790.4824. Found 790.4810.

### EXAMPLE 12. Synthesis of compound 12.

Compound **12** was obtained by treatment of a solution of **11** in MeOH (2.6 mL) with 1 M NaOMe in MeOH (42 µL), followed by neutralization with Amberlite^{®} IR120 hydrogen form, filtration, evaporation and freeze drying. Yield: 60 mg (quant.). *R_{f}* 0.6 (70:10:1 DCM-MeOH-H₂O). [α]_{D} +14.52 (c 1.0 in DCM:MeOH). ¹H NMR (500 MHz, 1:1 CD₃OD -CDCl₃): δ = 5.00 (d, 1 H, *J*_{1,2} = 4.0 Hz, H-1), 4.44 (m, 1 H, H-6a), 4.15 (dd, 1 H, *J*_{5,6b} = 9.5 Hz, *J*_{6a,6b} = 6.3 Hz, H-6b), 3.64 (m, 1 H, H-5), 3.59 (m, 3 H, H-4, OC*H₂*CH), 3.49 (bt, 1 H, *J*_{2,3} = 10.7 Hz, H-3), 3.36 (dd, 1 H, *J*_{2,3} = 9.9 Hz, H-2), 3.25 (m, 1 H, OCH*₂*C*H*), 3.11 (m, 2 H, NHC*H₂*), 2.15 (t, 2 H, *J*_{H,H} = 7.0 Hz, COCH₂), 1.53-0.79 (m, 48 H, CH₂, CH₂C*H₃*). ¹³C NMR (125.7 MHz, 1:1 CD₃OD-CDCl₃): δ = 174.9, 170.5 (CO), 157.4 (CO carbamate), 82.7 (C-1), 74.4 (C-3), 73.5 (O*C*H₂CH), 71.9 (C-2), 68.5 (C-5), 67.5 (C-6), 53.6 (C-4), 52.7 (OCH₂*C*H), 39.9 (NH*C*H₂), 36.3 (CO*C*H₂), 31.9-14.1 (CH₂, CH₂C*H₃*). ESIMS: *m*/*z* = 664.45 [M + Na]⁺. HRMS (ESI) calcd for [C₃₄H₆₃O₈N₃Na]⁺ 664.4507. Found 664.4511. Anal. calcd. for C₃₄H₆₃N₃O₈: C 63.62, H 9.89, N 6.55. Found C 63.41, H 9.74, N 6.29.

### EXAMPLE 13. Synthesis of compound 13.

To a stirred solution of **38** (100 mg, 0.131 mmol) in DMF (1.5 mL), piperidine (131 µL, 1 mL/mmol) was added. After 1 h the mixture was evaporated, the crude product was dissolved in DMF (1.8 mL) and octyl isothiocyanate (38 µL, 0.196 mmol) and Et₃N (37 µL, 0.262 mmol) were added. The mixture was stirred for 16 h and concentrated. The resulting residue was purified by column chromatography (1:1 EtOAc-cyclohexane) to afford **13.** Yield: 59 mg (59%, two steps). *R_{f}* 0.50 (1:1 EtOAc-cyclohexane). [α]_{D} +18.24 (c 1.0 in DCM). ¹H NMR (500 MHz, CDCl₃): δ = 6.63 (bs, 2 H, NH), 5.39 (m, 2 H, H-1, NH), 4.88 (t, 1 H, *J*_{2,3} = *J*_{3,4} =9.7 Hz, H-3), 4.86 (t, 1 H, *J*_{4,5} = 9.4 Hz, H-4), 4.40 (t, 1 H, *J*_{5,6a} = *J*_{5,6b} = 8.6 Hz, H-6a), 4.18 (bdd, 1 H, H-6b), 3.94 (bdd, 1 H, H-5), 3.85 (m, 1 H, CH), 3.73 (dd, 1 H, *J*_{1,2} = 6.0 Hz, H-2), 3.18 (m, 2 H, NHC*H*₂), 2.04, 1.98, 1.95 (3 s, 9 H, CH₃CO), 1.57-0.81 (m, 38 H, CH₂, CH₂C*H₃*). ¹³C NMR (125.7 MHz, CDCl₃): δ = 181.4 (CS), 169.9, 169.7, 169.6 (CO ester), 156.0 (CO carbamate), 79.8 (C-1), 72.4 (C-4), 70.2 (C-2), 69.7 (C-3), 68.7 (CH), 67.1 (C-6), 51.9 (C-5), 39.9 (NH*C*H₂), 31.9-14.1 (*C*H₃CO, CH₂, CH₂*C*H₃. HRMS (ESI) calcd for [C₃₇H₆₄N₄O₁₀SNa]⁺ 779.4235. Found 779.4229.

### EXAMPLE 14. Synthesis of compound 14.

Compound **14** was obtained by treatment of a solution of **13** in MeOH (2.6 mL) with 1 M NaOMe in MeOH (42 µL), followed by neutralization with Amberlite^{®} IR120 hydrogen form, filtration, evaporation and freeze drying. Yield: 49 mg (quant.). *R_{f}* 0.6 (70:10:1 DCM-MeOH-H₂O). [α]_{D} +18.25 (c 1.0 in MeOH). ¹H NMR (500 MHz, CD₃OD): δ = 5.02 (d, 1 H, *J*_{1,2} = 4.0 Hz, H-1), 4.44 (m, 1 H, *J*_{5,6a} = 9.2 Hz, *J*_{6a,6b} = 8.6 Hz H-6a), 4.15 (dd, 1 H, *J*_{5,6b} = 6.1 Hz, H-6b), 3.72 (m, 2 H, H-5, CH), 3.61 (m, 2 H, CH₂), 3.51 (t, 1 H, *J*_{3,4} = 10.4 Hz, H-4), 3.36 (dd, 1 H, *J*_{2,3} = 9.6 Hz, H-2), 3.24 (m, 1 H, H-3), 3.14 (m, 2 H, N*H*CH₂), 1.47-0.82 (m, 38 H, CH₂, CH₂C*H₃*). ¹³C NMR (125.7 MHz, CD₃OD): δ = 170.9 (CO), 157.2 (CO carbamate), 82.4 (C-1), 74.1 (C-3), 73.0 (C-4), 71.6 (C-2), 68.2 (C-5), 67.1 (C-6), 53.4 (CH), 39.2 (NH*C*H₂), 31.6-13.0 (CH₂, CH₂C*H₃*). ESIMS: *m*/*z*= 631.25 [M + H]+. Anal. calcd. for C₃₁H₅₈N₄O₇S: C 59.02, H 9.27, N 8.88, S 5.08. Found C 58.75, H 8.98, N 8.54, S 4.72.

### EXAMPLE 15. Synthesis of compound 15.

Compound 15 was obtained by conjugation of glycosyl donor 32 and the thiol acceptor 40. Acceptor 40 was prepared from commercial N-Fmoc-S-trityl-L-cysteine through the procedure described hereinafter.

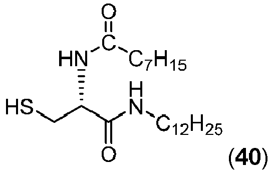

To a stirred solution of *N*-Fmoc-*S*-trityl-L-cysteine (10 g, 17 mmol) in THF (130 mL) were added HOBt (2.3 g, 17 mmol), DCC (3.5 g, 17 mmol) and dodecylamine (3.1 g, 17 mmol) at rt. The mixture was stirred overnight, filtered and concentrated. The crude mixture was dissolved in THF (132 mL) and piperidine (20 mL) was added. The reaction was stirred for 20 min. Then, the solvent was removed under reduced pressure and co-evaporated with MeOH (3 x 20 mL) to remove piperidine traces. The free amine (500 mg, 0.94 mmol) was dissolved in DCM (10 mL) and Et₃N (200 µL, 1.41 mmol) and octanoyl chloride (1.5 eq) were added. The mixture was then stirred overnight. After evaporation of the solvent, the crude product was dissolved in 1:1 DCM-TFA (4 mL) and triisopropylsilane (320 µL) was added. The reaction mixture was stirred for 1 h, the solvent was evaporated under reduced pressure and coevaporated with toluene (3 x 20 mL) to remove TFA traces. The resulting residue was purified by column chromatography (1:4 EtOAc-cyclohexane) to afford 40. Yield: 160 mg (75%). *R_{f}* 0.40 (1:4 EtOAc-cyclohexane). ¹H NMR (300 MHz, CDCl₃): δ = 6.47 (m, 2 H, NH), 4.50 (m, 1 H, CH), 3.17 (m, 2 H, CH₂N), 2.92, 2.66 (m, 2 H, SCH₂), 2.17 (t, 2 H, CH₂CO), 1.61-0.78 (m, 36 H, CH₂, CH₂C*H₃*). ¹³C NMR (75.5 MHz, CDCl₃): δ = 173.4, 169.7 (CO), 54.1 (CH), 39.7 (CH₂N), 36.5 (CH₂CO), 31.9-14.0 (CH₂, CH₂C*H₃*). HRMS (ESI) calcd for [C₂₃H₄₆O₂N₂SNa]⁺ 437.3172; found 437.3164.

In a subsequent step, to a solution of **32** (20 mg, 0.05 mmol) and **40** (0.10 mmol) in DCM (1.2 mL), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford the target compound **15**. Yield: 35 mg (65%). *R_{f}* 0.50 (1:1 EtOAc-cyclohexane). [α]_{D} +3.51 (c 1.0 in DCM). ¹H NMR (500 MHz, CDCl₃): δ = 6.38 (bt, 2 H, N*H*CH₂), 5.74 (d, 1 H, *J*_{1,2} = 5.9 Hz, H-1), 5.28 (t, 1 H, *J*_{2,3} = *J*_{3,4} =9.7 Hz, H-3), 4.87 (t, 1 H, *J*_{4,5} = 9.4 Hz, H-4), 4.86 (dd, 1 H, H-2), 4.61 (t, 1 H, *J*_{6a,6b} = *J*_{5,6a} = 8.5 Hz, H-6a), 4.43 (bt, 1 H, H-6b), 4.27 (m, 1 H, CH), 4.15 (bdd, 1 H, H-5), 3.12 (m, 2 H, NHC*H₂*), 2.94 (dd, 1 H, *J*_{H,H} = 13.0 Hz, *J*_{H,H} = 3.3 Hz, SCH₂), 2.73 (dd, 1 H, *J*_{H,H} = 15.0 Hz, *J*_{H,H} = 10.0 Hz, SCH₂), 2.18 (m, 2 H, COCH₂), 2.01, 1.99, 1.96 (3 s, 9 H, CH₃CO), 1.61-0.81 (m, 36 H, CH₂, CH₂C*H₃*). ¹³C NMR (125.7 MHz, CDCl₃): δ = 173.8, 170.0, 169.7, 169.6 169.4 (CO ester), 156.8 (CO carbamate), 72.6 (C-4), 70.3 (C-2), 69.5 (C-3), 67.2 (C-6), 60.2 (C-1), 53.1 (CH), 51.2 (C-5), 39.7 (NH*C*H₂), 36.7 (CO*C*H₂), 31.9-14.1 (*C*H₃CO, CH₂, CH₂C*H₃*). HRMS (ESI) calcd for [C₃₆H₆₁N₃O₁₀SNa]⁺ 750.3970. Found 750.3964.

### EXAMPLE 16. Synthesis of compound 16.

Compound **16** was obtained by treatment of a solution of **15** in MeOH (2.6 mL) with 1 M NaOMe in MeOH (42 µL), followed by neutralization with Amberlite^{®} IR120 hydrogen form, filtration, evaporation and freeze drying. Yield: 18 mg (quant.). *R_{f}* 0.5 (100:10:1 DCM-MeOH-H₂O). [α]_{D} +16.91 (c 1.0 in 5:1 DCM:MeOH). ¹H NMR (500 MHz, 5:1 CD₃OD-CDCl₃): δ = 5.33 (s, 1 H, NH), 5.20 (d, 1 H, *J*_{1,2} = 5.6 Hz, H-1), 4.51 (t, 1 H, *J*_{6a,6b} = 9.5 Hz, *J*_{5,6a} = 8.6 Hz, H-6a), 4.39 (dd, 1 H, *J*_{5,6b} = 6.2 Hz, H-6b), 4.18 (dd, 1 H, *J*_{3,4} = 9.5 Hz, *J*_{4,5} = 5.5 Hz, H-4), 3.79 (m, 1 H, CH), 3.58 (m, 1 H, H-5), 3.39 (t, 1 H, *J*_{2,3} = 9.3 Hz, H-3), 3.24 (m, 1 H, H-2), 3.08 (m, 2 H, NHC*H₂*), 2.86, 2.78 (dd, 2 H, SCH₂), 2.16 (t, 2 H, *J*_{H,H} = 7.0 Hz, COCH₂), 1.54-0.79 (m, 36 H, CH₂, CH₂C*H₃*). ¹³C NMR (125.7 MHz, 5:1 CD₃OD-CDCl₃): δ = 174.6, 170.7 (CO), 157.1 (CO carbamate), 74.0 (C-1), 73.7 (C-3), 71.0 (C-2), 67.0 (C-5), 62.5 (C-6), 53.2 (C-4), 53.1 (CH), 39.2 (NH*C*H₂), 35.6 (CO*C*H₂), 32.6-13.2 (CH₂, CH₂*C*H*₃*). ESIMS: *m*/*z*= 602.50 [M + H]⁺ Anal. calcd. for C₃₀H₅₅N₃O₇S: C 59.87, H 9.21, N 6.98, S 5.33. Found C 59.64, H 9.07, N 6.70, S 4.99.

### EXAMPLE 17. Synthesis of compound 17.

Compound **17** was obtained by conjugation of glycosyl donor 32 and the thiol acceptor **41.** Acceptor **41** was prepared from commercial N-Fmoc-S-trityl-L-cysteine through the procedure described hereinafter.

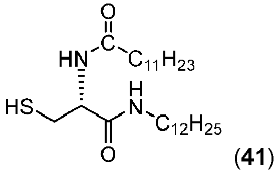

To a stirred solution of *N*-Fmoc-*S*-trityl-L-cysteine (10 g, 17 mmol) in THF (130 mL) were added HOBt (2.3 g, 17 mmol), DCC (3.5 g, 17 mmol) and dodecylamine (3.1 g, 17 mmol) at rt. The crude mixture was dissolved in THF (132 mL) and piperidine (20 mL) was added. The reaction was stirred for 20 min. Then, the solvent was removed under reduced pressure and co-evaporated with MeOH (3 x 20 mL) to remove piperidine traces. The free amine (500 mg, 0.94 mmol) was dissolved in DCM (10 mL) and Et₃N (200 µL, 1.41 mmol) and dodecyl chloride (1.5 eq) were added. The mixture was then stirred overnight. After evaporation of the solvent, the crude product was dissolved in 1:1 DCM-TFA (4 mL) and triisopropylsilane (320 µL) was added. The reaction mixture was stirred for 1 h, the solvent was evaporated under reduced pressure and co-evaporated with toluene (3 x 20 mL) to remove TFA traces. The resulting residue was purified by column chromatography (1:4 EtOAc-cyclohexane) to afford **41**. Yield: 230 mg (70%). *R_{f}* 0.40 (1:4 EtOAc-cyclohexane). ¹H NMR (300 MHz, CDCl₃): δ = 6.45 (m, 2 H, NH), 4.50 (m, 1 H, CH), 3.17 (m, 2 H, CH₂N), 2.94, 2.65 (m, 2 H, SCH₂), 2.16 (t, 2 H, CH₂CO), 1.61-0.81 (m, 44 H, CH₂, CH₂C*H₃*). ¹³C NMR (75.5 MHz, CDCl₃): δ = 173.4, 169.6 (CO), 54.1 (CH), 39.7 (CH₂N), 36.5 (CH₂CO), 31.9-14.0 (CH₂, CH₂C*H₃*). HRMS (ESI) calcd for [C₂₇H₅₄O₂N₂SNa]⁺ 493.3798; found 493.3793.

In a subsequent step, to a solution of **32** (20 mg, 0.05 mmol) and **41** (0.10 mmol), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:1 EtOAc-cyclohexane) to afford the target compound **17.** Yield: 27 mg (70 %). *R_{f}* 0.50 (1:1 EtOAc-cyclohexane). [α]_{D} +16.24 (c 1.0 in DCM). ¹H NMR (500 MHz, CDCl₃): δ = 6.39 (d, 1 H, *J*_{NH,CH} = 9.0 Hz, NHCH), 6.38 (bt, 1 H, *J*_{NH,CH} = 5.6 Hz, N*H*CH₂), 5.73 (d, 1 H, *J*_{1,2} = 5.9 Hz, H-1), 5.28 (t, 1 H, *J*_{2,3} = *J*_{3,4} =10.4 Hz, H-3), 4.86 (t, 1 H, *J*_{4,5} = 9.4 Hz, H-4), 4.85 (dd, 1 H , H-2), 4.61 (t, 1 H, *J*_{5,6a} = *J*_{5,6b} = 8.6 Hz, H-6a), 4.42 (m, 1 H, CH), 4.30 (bdd, 1 H, H-5), 4.24 (m, 1 H, H-6b), 3.11 (m, 2 H, NHC*H₂*), 2.93 (dd, 1 H, *J*_{H,H} = 15.0 Hz, *J*_{H,H} = 3.3 Hz, SCH₂), 2.72 (dd, 1 H, *J*_{H,H} = 15.0 Hz, *J*_{H,H} = 9.9 Hz, SCH₂), 2.18 (m, 2 H, COCH₂), 2.01, 1.99, 1.96 (3 s, 9 H, CH₃CO), 1.57-0.81 (m, 48 H, CH₂, CH₂C*H₃*). ¹³C NMR (125.7 MHz, CDCl₃): δ = 173.8, 170.0, 169.7, 169.4 (CO ester), 156.8 (CO carbamate), 72.6 (C-4), 70.3 (C-2), 69.5 (C-3), 67.3 (C-6), 60.2 (C-1), 53.1 (CH), 51.2 (C-5), 39.7 (NH*C*H₂), 36.7 (CO*C*H₂), 31.9-14.1 (*C*H₃CO, CH₂, CH₂C*H₃*). HRMS (ESI) calcd for [C₄₀H₆₉N₃O₁₀SNa]⁺ 806.4596; found 806.4588.

### EXAMPLE 18. Synthesis of compound 18.

Compound **18** was obtained by treatment of a solution of **17** in MeOH (2.6 mL) with 1 M NaOMe in MeOH (42 µL), followed by neutralization with Amberlite^{®} IR120 hydrogen form, filtration, evaporation and freeze drying. Yield: 20 mg (quant.). *R_{f}* 0.5 (70:10:1 DCM-MeOH-H₂O). [α]_{D} +16.23 (c 1.0 in 5:1 DCM:MeOH). ¹H NMR (500 MHz, 5:1 CD₃OD-CDCl₃): δ = 5.20 (d, 1 H, *J*_{1,2} = 5.7 Hz, H-1), 4.51 (bt, 1 H, *J*_{5,6a} = 9.5 Hz, *J*_{6a,6b} = 8.6 Hz, H-6a), 4.40 (dd, 1 H, *J*_{5,6b} = 6.3 Hz, H-6b), 4.18 (m, 1 H, H-5), 3.79 (m, 1 H, CH), 3.58 (dd, 1 H, *J*_{3,4} = 9.5 Hz, *J*_{4,5} = 5.5 Hz, H-4), 3.40 (t, 1 H, *J*_{2,3} = 9.3 Hz, H-3), 3.22 (m, 1 H, H-2), 3.09 (m, 2 H, NHC*H₂*), 2.86, 2.78 (dd, 2 H, SCH₂), 2.16 (t, 2 H, *J*_{H,H} = 7.0 Hz, COCH₂), 1.53-0.79 (m, 48 H, CH₂, CH₂C*H₃*). ¹³C NMR (125.7 MHz, 4:1 CD₃OD-CDCl₃): δ = 174.7, 170.7 (CO), 157.1 (CO carbamate), 74.1 (C-1), 73.8 (C-3), 71.1 (C-2), 67.1 (C-5), 62.6 (C-6), 53.3 (C-4), 53.2 (CH), 39.3 (NH*C*H₂), 35.8 (CO*C*H₂), 32.7-13.3 (CH₂, CH₂C*H₃*). ESIMS: *m*/*z* = 658.33 [M + H]⁺ Anal. calcd. for C₁₃H₁₆N₄O₈: C 43.82, H 4.53, N 15.73. Found C 43.89, H 4.60, N 15.57.

### EXAMPLE 19. Synthesis of compound 19.

Compound **19** was obtained by conjugation of glycosyl donor 46 and the thiol acceptor **40**. The glycosyl donor **46** was prepared from 5-azido-3-O-benzyl-5-deoxy-1,2-*O-*isopropylidene-α-D-glucofuranose (**42**) in four steps according to the following Scheme 8, through the procedure described hereinafter.

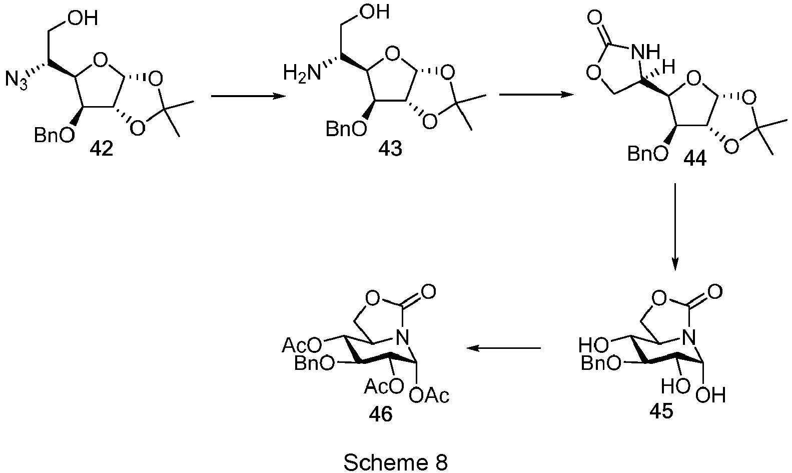

**Step 1:** To a stirred solution of **42** (5.7 g, 17.0 mmol) in dioxane/MeOH (5:1) (230 mL), Ph₃P (8.9 g, 34.0 mmol) was added and the reaction mixture was stirred at room temperature for 4 h. NH₄OH (32 mL) was added and the mixture was stirred overnight. Then, the solvent was removed under reduced pressure and the residue was purified by column chromatography (EtOAc, 45:5:1 → 45:5:3 EtOAc-EtOH-H₂O) to afford 5-amino-3-*O*-benzyl-5-deoxy-1,2-*O*-isopropylidene-α-D-glucofuranose (**43**). Yield: 7.1 g (90%). R*_{f}* 0.41 (45:5:3 EtOAc-EtOH-H₂O). [α]_{D} -68.2 (c 0.8 in DCM). ¹H NMR (400 MHz, CDCl₃) δ 7.45-7.30 (m, 5 H, CH-arom), 5.94 (d, 1 H, *J*_{1,2} = 3.8 Hz, H-1), 4.76 (d, 1 H, ²*J*_{H,H} = 11.8 Hz, O*CH₂*Ph), 4.64 (d, 1 H, H-2), 4.51 (d, 1 H, O*CH₂*Ph), 4.03 (d, 1 H, *J*_{3,4} = 3.0 Hz, H-3), 3.99 (dd, 1 H, *J*_{4,5} = 8.4 Hz, H-4), 3.79 (dd, 1 H, *J*_{6a,6b} = 10.8 Hz, *J*_{5,6a} = 4.0 Hz, H-6a), 3.58 (dd, 1 H, *J*_{5,6b} = 6.0 Hz, H-6b), 3.29 (m, 1 H, H-5), 2.00 (bs, 1 H, OH), 1.50, 1.34 (2 s, 6 H, CMe₂). ¹³C NMR (100 MHz, CDCl₃) δ 137.2-128.0 (C-arom), 111.6 (*C*Me₂), 105.1 (C-1), 81.7 (C-2), 81.6 (C-3, C-4), 71.7 (O*CH₂*Ph), 64.3 (C-6), 50.9 (C-5), 26.7, 26.2 (C*Me₂).* ESIMS: *m*/*z* 310.1 [M + H]⁺. Anal. Calcd for C₁₆H₂₃NO₅: C 62.12, H 7.49, N 4.53. Found: C 61.88, H 7.27, N 4.44.

**Step 2:** To a stirred solution of the amine **43** (7.3 g, 23 mmol) in DCM (162 mL) were added DIPEA (39 mL, 236 mmol) and triphosgene (10.5 g, 35.4 mmol) at 0 °C. After 30 min, the solvent was removed under reduced pressure, and the residue was purified by column chromatography using EtOAc as eluent to give 5-amino-5-deoxy-1,2-*O*-isopropylidene-3-*O*-benzyl-α-D-glucofuranose 5,6-(cyclic carbamate) (**44**). Yield: 5.4 g (69%). R*_{f}* 0.84 (EtOAc). [α]_{D} -91.7 (*c* 0.96 in CHCl₃). ¹H NMR (400 MHz, CDCl₃) δ 7.50-7.30 (m, 5 H, CH-arom), 5.95 (d, 1 H, *J*_{1,2} = 3.8 Hz, H-1), 5.38 (bs, 1 H, NH), 4.74 (d, 1 H, ²*J*_{H,H} = 11.8 Hz, O*CH₂*Ph), 4.68 (d, 1 H, H-2), 4.51 (d, 1 H, O*CH₂*Ph), 4.51-4.46 (m, 1 H, H-6a), 4.43 (dd, 1 H, *J*_{6b,6a} = 8.8 Hz, *J*_{5,6b} = 4.8 Hz, H-6b), 4.20 (dd, 1 H, *J*_{4,5} = 7.8 Hz, *J*_{3,4} = 3.4 Hz, H-4), 4.12-4.05 (m, 1 H, H-5), 4.02 (d, 1 H, H-3), 1.52, 1.35 (2 s, 6 H, CMe₂). ¹³C NMR (100 MHz, CDCl₃) δ 160.0 (CO), 137.1-128.0 (C-arom), 112.2 (*C*Me₂), 105.5 (C-1), 82.0 (C-2), 81.6 (C-4), 81.3 (C-3), 71.8 (O*CH₂*Ph), 68.1 (C-6), 50.8 (C-5), 26.9, 26.3 (C*Me₂).* ESIMS: *m*/*z* 358.1 [M + Na]⁺. Anal. Calcd for C₁₇H₂₁NO₆: C 60.89, H 6.31, N 4.18. Found: C 60.93, H 6.24, N 4.09.

**Step 3:** A solution of **44** (2.1 g, 6.2 mmol) in 90% TFA-H₂O (9.8 mL) was stirred at room temperature for 30 min. The solvent was eliminated under reduced pressure, coevaporated several times with water, treated with NaOH 0.1 N until pH 8 and concentrated. The resulting residue was purified by column chromatography using EtOAc as eluent and the product was freeze-dried from a water solution to afford 3-*O*-benzyl-5*N*,6*O*-oxomethylidenenojirimycin **(45).** Yield: 1.6 g (90%). *R_{f}* 0.60 (EtOAc). [α]_{D} -2.7 (c 1.0 in H₂O). ¹H NMR (400 MHz, D₂O) δ 7.50-7.30 (m, 5 H, CH-arom), 5.33 (d, 1 H, *J*_{1,2} = 4.0 Hz, H-1), 4.84 (d, 1 H, ²*J*_{H,H} = 10.8 Hz, O*CH₂*Ph), 4.80 (d, 1 H, O*CH₂*Ph), 4.57 (t, 1 H, *J*_{6a,6b} = *J*_{5,6a} = 8.8 Hz, H-6a), 4.25 (dd, 1 H, *J*_{5,6b} = 6.8 Hz, H-6b), 3.98-3.90 (m, 1 H, H-5), 3.70 (t, 1 H, *J*_{2,3} = *J*_{3,4} = 9.4 Hz, H-3), 3.63 (dd, 1 H, H-2), 3.56 (t, 1 H, *J*_{4,5} = 9.4 Hz, H-4). ¹³C NMR (100 MHz, D₂O) δ 157.6 (CO), 137.6-128.4 (C-arom), 80.9 (C-3), 75.3 (O*CH₂*Ph), 74.6 (C-1), 73.3 (C-4), 71.1 (C-2), 67.6 (C-6), 53.2 (C-5). ESIMS: *m*/*z* 318.1 [M + Na]⁺. Anal. Calcd for C₁₄H₁₇NO₆: C 56.94, H 5.80, N 4.74. Found: C 56.75, H 5.63, N 4.57.

**Step 4:** To a stirred solution of **45** (1.6 g, 5.4 mmol) in pyridine (7 mL) AC₂O (7 mL, 74 mmol) was added, and the mixture was stirred at room temperature overnight. The reaction was dropped into ice-water (100 mL), extracted with DCM (3 x 20 mL) and the organic phase was washed with HCI 1 M (3 x 20 mL), brine (20 mL), dried with MgSO₄, filtered and concentrated. The resulting residue was purified by column chromatography using 1:1 EtOAc-cyclohexane as eluent to give (1R)-1,4-di-O-acetyl-3-*O*-benzyl-5*N*,6*O*-oxomethylidenenojirimycin **(46).** Yield: 2 g (87%). R*_{f}* 0.42 (1:1 EtOAc-cyclohexane). [α]_{D} +13.5 (c 1.0 in DCM). ¹H NMR (300 MHz, CDCl₃) δ 7.30-7.16 (m, 5 H, CH-arom), 6.62 (d, 1 H, *J*_{1,2} = 3.8 Hz, H-1), 5.00 (dd, 1 H, *J*_{2,3} = 10.0 Hz, H-2), 4.86 (t, 1 H, *J*_{3,4} = *J*_{4,5} = 9.4 Hz), 4.62 (d, 2 H, ²*J*_{H,H} = 10.8 Hz, O*CH₂*Ph), 4.32 (dd, 1 H, *J*_{6a,6b} = *J*_{5,6a} = 8.2 Hz, H-6a), 4.21 (dd, 1 H, *J*_{5,6b} = 6.8 Hz, H-6b), 3.89 (m, 2 H, H-3, H-5), 2.99, 1.93, 1.91 (3 s, 3 H, COOCH₃).¹³C NMR (75.5 MHz, CDCl₃) δ 170.0, 169.2, 168.5 (CO ester), 154.3 (CO carbamate), 137.7-127.4 (C-arom), 76.7 (C-3), 75.5 (O*CH₂*Ph), 73.8 (C-4), 72.7 (C-1), 71.0 (C-2), 67.2 (C-6), 53.2 (C-5). HRMS (ESI) calcd for [C₂₀H₂₃NO₉Na]⁺ 444.1276; found 444.1261.

In a subsequent step, to a solution of **46** (150 mg, 0.35 mmol) and **40** (0.53 mmol), BF₃·OEt₂ (215 µL, 1.78 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:1 EtOAc-cyclohexane) to afford the target compound **19.** Yield: 200 mg (68%). *R_{f}* 0.65 (1:1 EtOAc-cyclohexane). [α]_{D} +31.50 (c 1.0 in DCM). ¹H NMR (500 MHz, CDCl₃): δ 7.28-7.16 (m, 5 H, CH-arom), 6.64 (d, 1 H, *J*_{NH,CH} = 8.5 Hz, NHCH), 6.54 (bt, 1 H, *J*_{NH,CH} = 5.6 Hz, N*H*CH₂), 5.72 (d, 1 H, *J*_{1,2} = 5.8 Hz, H-1), 4.85 (t, 1 H, *J*_{2,3} = *J*_{3,4} =7.5 Hz, H-3), 4.83 (t, 1 H, *J*_{4,5} = 9.5 Hz, H-4), 4.67 (d, 1 H, ²*J*_{H,H} = 11.5 Hz, O*CH₂*Ph), 4.56 (d, 1 H, ²*J*_{H,H} = 11.8 Hz, O*CH₂*Ph), 4.54 (bdd, 1 H , H-2), 4.46 (m, 1 H, CH), 4.28 (t, 1 H, *J*_{6a,6b} = *J*_{5,6a} = 8.6 Hz, H-6a), 4.19 (m, 1 H, H-5), 3.75 (t, 1 H, H-6b), 3.11 (m, 2 H, NHC*H₂*), 2.95 (dd, 1 H, *J*_{H,H} = 15.0 Hz, *J*_{H,H} = 3.3 Hz, SCH₂), 2.73 (dd, 1 H, *J*_{H,H} = 15.0 Hz, *J*_{H,H} = 9.9 Hz, SCH₂), 2.18 (m, 2 H, COCH₂), 1.99, 1.91 (2 s, 6 H, CH₃CO), 1.59-0.80 (m, 48 H, CH₂, CH₂C*H₃*). ¹³C NMR (125.7 MHz, CDCl₃): δ = 174.0, 170.1, 169.8, 169.6 (CO), 157.0 (CO carbamate), 137.7-127.4 (C-arom), 77.4 (CH₂Ph), 75.6 (C-4), 74.1 (C-2), 72.4 (C-3), 67.6 (C-6), 60.1 (C-1), 53.4 (CH), 51.8 (C-5), 39.7 (NH*C*H₂), 36.6 (CO*C*H₂), 31.9-14.1 (*C*H₃CO, CH₂, CH₂C*H₃*). HRMS (ESI) calcd for [C₄₅H₇₄N₃O₉SNa]⁺ 832.5140; found 832.5140.

### EXAMPLE 20. Synthesis of compound 20.

Compound 20 was obtained by treatment of a solution of **19** in MeOH (3.6 mL) with 1 M NaOMe in MeOH (25 µL), followed by neutralization with Amberlite^{®} IR120 hydrogen form, filtration, evaporation and freeze drying. Yield: 54 mg (quant.). *R_{f}* 0.5 (2:1 EtOAc-cyclohexane). [α]_{D} +43.06 (c 1.0 in DCM). ¹H NMR (500 MHz, 5:1 CD₃OD-CDCl₃): δ = 7.33-7.14 (m, 5 H, CH-arom), 5.20 (d, 1 H, *J*_{1,2} = 5.7 Hz, H-1), 4.76 (d, 1 H, ²*J*_{H,H} = 11.2 Hz, O*CH₂*Ph), 4.51 (t, 1 H, *J*_{6a,6b} = 9.5 Hz, *J*_{5,6a} = 8.5 Hz, H-6a), 4.40 □dd, 1 H, *J*_{5,6b} = 6.4 Hz, H-6b), 4.16 (dd, 1 H, *J*_{3,4} = 9.1 Hz, *J*_{4,5}= 5.9 Hz, H-4), 3.81 (m, 1 H, H-5), 3.74 (m, 1 H, CH), 3.37 (m, 2 H, H-2, H-3), 3.09 (m, 2 H, NHC*H₂*), 2.87, 2.80 (dd, 2 H, SCH₂), 2.16 (t, 2 H, *J*_{H,H} = 7.0 Hz, COCH₂), 1.53-0.79 (m, 48 H, CH₂, CH₂C*H₃*). ¹³C NMR (125.7 MHz, 4:1 CD₃OD-CDCl₃): δ = 174.7, 170.7 (CO), 157.0 (CO carbamate), 138.6-127.3 (C-arom), 82.4 (CH₂Ph), 75.3 (C-4), 73.9 (C-2), 71.3 (C-3), 67.1 (C-6), 62.8 (C-1), 53.5 (CH), 53.2 (C-5), 39.3 (NH*C*H₂), 35.7 (CO*C*H₂), 32.6-13.3 (CH₂, CH₂C*H₃*). HRMS (ESI) calcd for [C₄₁H₆₉N₃O₇SNa]⁺ 770.4748; found 770.4741.

### EXAMPLE 21. Synthesis of compound 21.

Compound **21** was obtained by conjugation of glycosyl donor **32** and the thiol acceptor **47.** Acceptor **47** was prepared from commercial N-Fmoc-S-trityl-L-cysteine through the procedure described hereinafter.

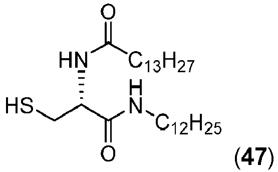

To a stirred solution of *N*-Fmoc-*S*-trityl-L-cysteine (10 g, 17 mmol) in THF (130 mL) were added HOBt (2.3 g, 17 mmol), DCC (3.5 g, 17 mmol) and dodecylamine (3.1 g, 17 mmol) at rt. The mixture was stirred overnight, filtered and concentrated. The crude mixture was dissolved in THF (132 mL) and piperidine (20 mL) was added. The reaction mixture was stirred for 20 min. Then, the solvent was removed under reduced pressure and co-evaporated with MeOH (3 x 20 mL) to remove piperidine traces. The free amine (500 mg, 0.94 mmol) was dissolved in DCM (10 mL) and Et₃N (200 µL, 1.41 mmol) and tetradecanoyl chloride (1.5 eq) were added. The mixture was then stirred overnight. After evaporation of the solvent, the crude product was dissolved in 1:1 DCM-TFA (4 mL) and triisopropylsilane (320 µL) was added. The reaction mixture was stirred for 1 h, the solvent was evaporated under reduced pressure and coevaporated with toluene (3 x 20 mL) to remove TFA traces. The resulting residue was purified by column chromatography (1:4 EtOAc-cyclohexane) to afford **47.** Yield: 250 mg (53%, two steps). *R_{f}* 0.50 (1:4 EtOAc-cyclohexane). [α]_{D} -18.96 (c 1.0 in DCM). Column chromatography 1:4 EtOAc-cyclohexane. Yield: 250 mg (53%, two steps). *R_{f}* 0.50 (1:4 EtOAc-cyclohexane). [α]_{D} -18.96 (c 1.0 in DCM). ¹H NMR (300 MHz, CDCl₃): δ = 6.46 (m, 2 H, NH), 4.50 (m, 1 H, CH), 3.17 (m, 2 H, CH₂N), 2.94, 2.65 (m, 2 H, SCH₂), 2.17 (t, 2 H, CH₂CO), 1.57-0.81 (m, 48 H, CH₂, CH₂C*H₃*). ¹³C NMR (75.5 MHz, CDCl₃): δ = 173.5, 169.7 (CO), 54.1 (CH), 39.7 (CH₂N), 36.5 (CH₂CO), 31.9-14.0 (CH₂, CH₂C*H₃*). HRMS (ESI) calcd for [C₂₈H₅₉O₅NS]⁺ 521.4108; found 521.4107.

In a subsequent step, to a solution of **32** (50 mg, 0.13 mmol) and **47** (0.19 mmol), BF₃·OEt₂ (80 µL, 0.65 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford the target compound 21. Yield: 92 mg (76%). *R_{f}* 0.60 (1:1 EtOAc-cyclohexane). [α]_{D} +15.40 (c 1.0 in DCM). ¹H NMR (500 MHz, CDCl₃): δ = 6.42 (bt, 2 H, N*H*CH₂), 5.72 (d, 1 H, *J*_{1,2} = 5.9 Hz, H-1), 5.27 (t, 1 H, *J*_{2,3} = *J*_{3,4} =9.8 Hz, H-3), 4.86 (t, 1 H, *J*_{4,5} = 9.3 Hz, H-4), 4.85 (dd, 1 H , H-2), 4.59 (t, 1 H, *J*_{6a,6b} = *J*_{5,6a} = 8.5 Hz, H-6a), 4.41 (m, 1 H, CH), 4.28 (bdd, 1 H, H-5), 4.23 (bt, 1 H, H-6b), 3.10 (m, 2 H, NHC*H₂*), 2.92 (dd, 1 H, *J*_{H,H} = 13.0 Hz, *J*_{H,H} = 3.3 Hz, SCH₂), 2.71 (dd, 1 H, *J*_{H,H} = 15.0 Hz, *J*_{H,H} = 10.0 Hz, SCH₂), 2.17 (m, 2 H, COCH₂), 2.00, 1.98, 1.95 (3 s, 9 H, CH₃CO), 1.58-0.79 (m, 46 H, CH₂, CH₂C*H₃*). ¹³C NMR (125.7 MHz, CDCl₃): δ = 173.8, 170.1, 169.7, 169.6, 169.4 (CO ester), 156.8 (CO carbamate), 72.6 (C-4), 70.2 (C-2), 69.5 (C-3), 67.2 (C-6), 60.0 (C-1), 53.1 (CH), 51.2 (C-5), 47.0 (NH*C*H₂), 39.7 (CO*C*H₂), 31.9-14.1 (*C*H₃CO, CH₂, CH₂C*H₃*). HRMS (ESI) calcd for [C₃₆H₆₁N₃O₁₀SNa]⁺ 750.3970; found 750.3964.

### EXAMPLE 22. Synthesis of compound 22.

Compound **22** was obtained by treatment of a solution of **21** in MeOH (2.6 mL) with 1 M NaOMe in MeOH (42 µL), followed by neutralization with Amberlite^{®} IR120 hydrogen form, filtration, evaporation and freeze drying. Yield: 80 mg (quant.). *R_{f}* 0.5 (100:10:1 DCM-MeOH-H₂O). [α]_{D} +16.23 (c 1.0 in 1:1 DCM:MeOH). ¹H NMR (500 MHz, 5:1 CD₃OD-CDCl₃): δ = d, 1 H, *J*_{1,2} = 5.7 Hz, H-1), 4.51 (bt, 1 H, *J*_{6a,6b} = 9.5 Hz, *J*_{5,6a} = 8.6 Hz, H-6a), 4.40 (dd, 1 H, *J*_{5,6b} = 6.3 Hz, H-6b), 4.18 (m, 1 H, H-5), 3.79 (m, 1 H, CH), 3.58 (dd, 1 H, *J*_{3,4} = 9.5 Hz, *J*_{4,5} = 5.5 Hz, H-4), 3.40 (t, 1 H, *J*_{2,3} = 9.3 Hz, H-3), 3.22 (m, 1 H, H-2), 3.09 (m, 2 H, NHC*H₂*), 2.86, 2.78 (dd, 2 H, SCH₂), 2.16 (t, 2 H, *J*_{H,H} = 7.0 Hz, COCH₂), 1.53-0.79 (m, 48 H, CH₂, CH₂C*H₃*). ¹³C NMR (125.7 MHz, 4:1 CD₃OD-CDCl₃): δ = 174.7, 170.7 (CO), 157.1 (CO carbamate), 74.1 (C-1), 73.8 (C-3), 71.1 (C-2), 67.1 (C-5), 62.6 (C-6), 53.3 (C-4), 53.2 (CH), 39.3 (NH*C*H₂), 35.8 (CO*C*H₂), 32.7-13.3 (CH₂, CH₂C*H₃*). ESIMS: *m*/*z* = 686.60 [M + H]⁺. Anal. calcd. for C₃₆H₆₇N₇O₇S: C 63.03, H 9.84, N 6.13, S 4.67. Found C, 62.85, H 9.52, N 5.83, S 4.30.

### EXAMPLE 23. Synthesis of compound 23.

Compound **23** was obtained by conjugation of glycosyl donor **32** and the thiol acceptor **48.** Acceptor **48** was prepared from commercial N-Fmoc-S-trityl-L-cysteine through the procedure described hereinafter.

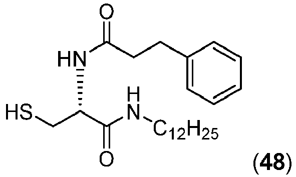

To a stirred solution of *N*-Fmoc-*S*-trityl-L-cysteine (10 g, 17 mmol) in THF (130 mL) were added HOBt (2.3 g, 17 mmol), DCC (3.5 g, 17 mmol) and dodecylamine (3.1 g, 17 mmol) at rt. The mixture was stirred overnight, filtered and concentrated. The crude mixture was dissolved in THF (132 mL) and piperidine (20 mL) was added. The reaction mixture was stirred for 20 min. Then, the solvent was removed under reduced pressure and co-evaporated with MeOH (3 x 20 mL) to remove piperidine traces. The free amine (500 mg, 0.94 mmol) was dissolved in DCM (10 mL) and Et₃N (200 µL, 1.41 mmol) and hydrocinnamoyl chloride (1.5 eq) were added. The mixture was then stirred overnight. After evaporation of the solvent, the crude product was dissolved in 1:1 DCM-TFA (4 mL) and triisopropylsilane (320 µL) was added. The reaction mixture was stirred for 1 h, the solvent was evaporated under reduced pressure and coevaporated with toluene (3 x 20 mL) to remove TFA traces. The resulting residue was purified by column chromatography (1:4 EtOAc-cyclohexane) to give **48.** Yield: 114 mg (72%). *R_{f}* 0.40 (1:4 EtOAc-cyclohexane). ¹H NMR (300 MHz, CDCl₃): δ = 7.24-7.11 (m, 5 H, CH-arom), 6.33. (m, 2 H, NH), 4.46 (m, 1 H, CH), 3.12 (m, 2 H, CH₂N), 2.90, 2.49 (m, 2 H, SCH₂), 2.17 (m, 2 H, CH₂CO), 1.47-0.78 (m, 25 H, CH₂, CH₂C*H₃*). ¹³C NMR (75.5 MHz, CDCl₃): δ = 172.2, 169.4 (CO), 140.3-126.4 (C-arom), 54.1 (CH), 39.7 (CH₂N), 36.5 (CH₂CO), 31.9-14.0 (CH₂, CH₂C*H₃*). HRMS (ESI) calcd for [C₂₄H₄₀O₂N₂SNa]⁺ 443.2703; found 443.2703.

In a subsequent step, to a solution of **32** (50 mg, 0.13 mmol) and **48** (0.19 mmol), BF₃·OEt₂ (80 µL, 0.65 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting acetylated compound was dissolved in MeOH (1.3 mL) and treated with 1 M NaOMe in MeOH (20 µL), followed by neutralization with Amberlite^{®} IR120 hydrogen form, filtration, evaporation and freeze drying. The resulting residue was purified by column chromatography (100:10:1 DCM-MeOH-H₂O) to afford the target compound **23.** Yield: 22 mg (quant.). *R_{f}* 0.5 (100:10:1 DCM-MeOH-H₂O). [α]_{D} +28.98 (c 1.0 in 1:5 DCM:MeOH). ¹H NMR (500 MHz, 5:1 CD₃OD-CDCl₃): δ =7.12 (m, 5 H, CH-arom), 5.17 (d, 1 H, *J*_{1,2} = 5.6 Hz, H-1), 4.49 (t, 1 H, *J*_{6a,6b} = 9.5 Hz, *J*_{5,6a} = 8.6 Hz, H-6a), 4.38 (dd, 1 H, *J*_{5,6b} = 6.2 Hz, H-6b), 4.17 (dd, 1 H, *J*_{3,4} = 9.5 Hz, *J*_{4,5} = 5.5 Hz, H-4), 3.77 (m, 1 H, CH), 3.57 (m, 1 H, H-5), 3.39 (t, 1 H, *J*_{2,3} = 9.3 Hz, H-3), 3.22 (m, 1 H, H-2), 3.05 (m, 2 H, NHC*H₂*), 2.83, 2.82, 2.74 (dd, 4 H, SCH₂, CH₂Ph), 2.47 (t, 2 H, *J*_{H,H} = 7.0 Hz, COCH₂), 1.40-0.79 (m, 23 H, CH₂, CH₂C*H₃*). ¹³C NMR (125.7 MHz, 5:1 CD₃OD-CDCl₃): δ = 173.6, 170.6 (CO), 157.1 (CO carbamate), 140.6-125.9 (C-arom), 74.0 (C-1), 73.8 (C-3), 71.1 (C-2), 67.1 (C-5), 62.6 (C-6), 53.3 (C-4), 53.2 (CH), 39.3 (NH*C*H₂), 37.4 (CO*C*H₂), 32.6-13.2 (CH₂, CH₂C*H₃*). ESIMS: *m*/*z*= 608.44 [M + H]⁺. Anal. calcd. for C₃₁H₄₉N₃O₇S: C 61.26, H 8.13, N 6.91, S 5.27. Found C 60.97, H 7.88, N 6.71, S 4.96.

### EXAMPLE 24. Synthesis of compound 24.

Compound **24** was obtained by conjugation of glycosyl donor **32** and the thiol acceptor **49.** Acceptor **49** was prepared from commercial N-Fmoc-S-trityl-L-cysteine through the procedure described hereinafter.

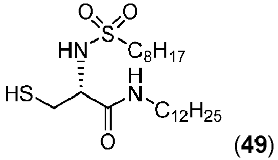

To a stirred solution of *N*-Fmoc-*S*-trityl-I-cysteine (10 g, 17 mmol) in THF (130 mL) were added HOBt (2.3 g, 17 mmol), DCC (3.5 g, 17 mmol) and dodecylamine (3.1 g, 17 mmol) at rt. The mixture was stirred overnight, filtered and concentrated. The crude mixture was dissolved in THF (132 mL) and piperidine (20 mL) was added. The reaction mixture was stirred for 20 min. Then, the solvent was removed under reduced pressure and co-evaporated with MeOH (3 x 20 mL) to remove piperidine traces. The free amine (500 mg, 0.94 mmol) was dissolved in DCM (10 mL) and Et₃N (200 µL, 1.41 mmol) and 1-octanesulfonyl chloride (1.5 eq) were added. The mixture was then stirred overnight. After evaporation of the solvent, the crude product was dissolved in 1:1 DCM-TFA (4 mL) and triisopropylsilane (320 µL) was added. The reaction mixture was stirred for 1 h, the solvent was evaporated under reduced pressure and coevaporated with toluene (3 x 20 mL) to remove TFA traces. The resulting residue was purified by column chromatography (1:4 EtOAc-cyclohexane) to afford **49.** Yield: 155 mg (80%). *R_{f}* 0.40 (1:4 EtOAc-cyclohexane). ¹H NMR (300 MHz, CDCl₃): δ = 6.43 (m, 2 H, NH), 3.98 (m, 1 H, CH), 3.10 (m, 2 H, CH₂S), 3.21 (m, 2 H, CH₂N), 2.97, 2.68 (m, 2 H, SCH₂), 1.91-0.81 (m, 38 H, CH₂, CH₂C*H₃*). ¹³C NMR (75.5 MHz, CDCl₃): δ = 168.8 (CO), 58.1 (CH), 53.7 (CH₂S), 40.0 (CH₂N), 31.9-14.0 (CH₂, CH₂C*H₃*). HRMS (ESI) calcd for [C₂₃H₄₉O₃N₂S₂Na]⁺ 465.3179; found 465.3174.

In a subsequent step, to a solution of **32** (50 mg, 0.13 mmol) and **49** (0.19 mmol), BF₃·OEt₂ (80 µL, 0.65 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford the target compound **24.** Yield: 70 mg (55%). *R_{f}* 0.50 (1:1 EtOAc-cyclohexane). [α]_{D} +14.94 (c 1.0 in DCM). ¹H NMR (500 MHz, CDCl₃): δ = 6.55 (bt, 1 H, *J*_{NH,CH} = 5.6 Hz, N*H*CH₂), 5.71 (d, 1 H, *J*_{1,2} = 5.9 Hz, H-1), 5.27 (t, 1 H, *J*_{2,3} = *J*_{3,4} =10.4 Hz, H-3), 4.89 (t, 1 H, *J*_{4,5} = 9.4 Hz, H-4), 4.86 (dd, 1 H , H-2), 4.55 (t, 1 H, *J*_{6a,6b} = *J*_{5,6a} = 8.6 Hz, H-6a), 4.35 (m, 1 H, H-6b), 4.23 (bdd, 1 H, H-5), 3.71 (m, 1 H, CH), 3.15 (m, 2 H, NHC*H₂*), 2.93-2.73 (m, 4 H, SCH₂), 2.02, 1.99, 1.97 (3 s, 9 H, CH₃CO), 1.72-0.81 (m, 38 H, CH₂, CH₂C*H₃*). ¹³C NMR (125.7 MHz, CDCl₃): δ = 170.1, 169.8, 169.2, 169.1 (CO ester), 156.9 (CO carbamate), 72.5 (C-4), 70.2 (C-2), 69.4 (C-3), 67.4 (C-6), 61.0 (C-1), 57.3 (CH), 53.7 (C-5), 51.5 (NH*C*H₂), 40.0 (CO*C*H₂), 31.9-14.1 (*C*H₃CO, CH₂, CH₂C*H₃*). HRMS (ESI) calcd for [C₃₆H₆₃N₃O₁₁S₂Na]⁺ 800.3796; found 800.3790.

### EXAMPLE 25. Synthesis of compound 25.

Compound **25** was obtained by treatment of a solution of **24** in MeOH (2.6 mL) with 1 M NaOMe in MeOH (42 µL), followed by neutralization with Amberlite^{®} IR120 hydrogen form, filtration, evaporation and freeze drying. Yield: 50 mg (quant.). *R_{f}* 0.5 (100:10:1 DCM-MeOH-H₂O). [α]_{D} +34.60 (c 1.0 in DCM:MeOH). ¹H NMR (500 MHz, 5:1 CD₃OD-CDCl₃): δ =5.31 (d, 1 H, *J*_{1,2} = 5.5 Hz, H-1), 4.59 (bt, 1 H, *J*_{6a,6b} = 9.5 Hz, *J*_{5,6a} = 8.6 Hz, H-6a), 4.26 (dd, 1 H, *J*_{5,6b} = 5.9 Hz, H-6b), 3.96 (m, 1 H, H-5), 3.92 (m, 1 H, CH), 3.67 (dd, 1 H, *J*_{3,4} = 9.8 Hz, *J*_{4,5} = 5.6 Hz, H-4), 3.49 (t, 1 H, *J*_{2,3} = 9.2 Hz, H-3), 3.30 (m, 1 H, H-2), 3.20 (m, 2 H, NHC*H₂*), 3.00, 2.88 (m, 2 H, SCH₂), 1.75-0.88 (m, 38 H, CH₂, CH₂C*H₃*). ¹³C NMR (125.7 MHz, 5:1 CD₃OD-CDCl₃): δ = 170.7 (CO), 157.2 (CO carbamate), 74.0 (C-1), 73.7 (C-3), 71.0 (C-2), 67.1 (C-5), 62.6 (C-6), 56.6 (C-4), 53.3 (CH), 53.1 (NH*C*H₂), 39.4-13.3 (CH₂, CH₂C*H₃*). ESIMS: *m*/*z =* 652.46 [M + H]⁺. Anal. calcd. for C₃₀H₅₇N₃O₈S₂: C 55.27, H 8.81, N 6.45, S 9.84. Found C 54.98, H 8.63, N 6.21, S 9.55.

### EXAMPLE 26. Synthesis of compound 26.

The synthesis of compound **26** comprised a first conjugation between glycosyl donor **33** and acceptor **36** to give intermediate **50.**

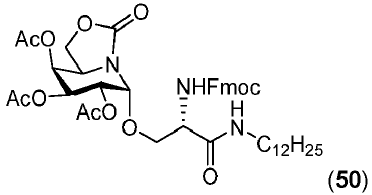

To a solution of 33 (20 mg, 0.05 mmol) in dry DCM (1.2 mL), the L-serine derivative 36 (0.10 mmol) and BF₃·OEt₂ (32 µL, 0.25 mmol) were added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:2 EtOAc-cyclohexane) to afford 50. Yield: 106 mg (60%). *R_{f}* 0.60 (2:1 EtOAc-cyclohexane). [α]_{D} +33.71 (c 1.0 in DCM). ¹H NMR (300 MHz, CDCl₃): δ = 7.95-7.28 (m, 12 H, CH-arom), 6.21 (bt, 1 H, *J*_{NH,CH2} = 5.6 Hz, N*H*CH₂), 5.65 (d. 1 H, *J*_{NH,CH} = 7.8 Hz, NHFmoc), 5.51 (d, 1 H, *J*_{1,2} = 4.0 Hz, H-1), 5.42 (bt, 1 H, *J*_{3,4} = 2.6 Hz, *J*_{4,5} = 2.2 Hz, H-4), 5.28 (dd, 1 H, *J*_{2,3} = 10.9 Hz, H-3), 5.13 (dd, 1 H, H-2), 4.07 (m, 4 H, H-6a, OCH₂C*H*, C*H₂*Fmoc), 4.23 (m, 2 H, H-5, C*H*Fmoc), 4.00 (dd, 1 H, *J*_{5,6a} = *J*_{5,6b} = 8.6 Hz, *J*_{6a,6b} = 5.9 Hz, H-6b), 3.79 (d, 2 H, OC*H₂*CH), 3.25 (m, 2 H, N*H*CH₂), 2.19, 2.10, 2.01 (3 s, 9 H, CH₃CO), 1.73-0.89 (m, 25 H, CH₂, CH₂C*H₃*). ¹³C NMR (75.5 MHz, CDCl₃): δ = 170.2, 169.8 (CO), 155.9 (CO carbamate), 143.6-120.0 (C-arom), 80.1 (C-1), 69.2 (O*C*H₂CH), 67.8 (C-4, C-3), 67.2 (C-2, C*H₂*Fmoc), 63.0 (C-6), 54.4 (O*C*H₂CH), 50.6 (CHFmoc), 47.0 (C-5), 39.8 (NH*C*H₂), 31.9-14.1 (*C*H₃CO, CH₂, CH₂C*H₃*). ESIMS: *m*/*z*= 830.38 [M + Na]⁺. HRMS (ESI) calcd for [C₄₃H₅₇O₁₂N₃Na]⁺ 830.3834; found 830.3825.

In a subsequent step, to a stirred solution of **50** (100 mg, 0.131 mmol) in DMF (1.5 mL), piperidine (131 µL, 1 mL/mmol) was added. After 1 h the mixture was concentrated, the crude product was dissolved in DMF (1.8 mL) and dodecanoyl chloride (47 µL, 0.196 mmol) and Et₃N (37 µL, 0.262 mmol) were added. The mixture was stirred for 16 h and concentrated. The resulting residue was purified by column chromatography (1:1 EtOAc-cyclohexane) to afford the target compound **26.** Yield: 65 mg (85%). *R_{f}* 0.30 (1:1 EtOAc-cyclohexane). [α]_{D} +40.83 (*c* 1.0 in DCM). ¹H NMR (300 MHz, CDCl₃): δ = 6.39 (d, 1 H, *J*_{NH,CH} = 8.2 Hz, NHCH), 6.34 (bt, 1 H, *J*_{NH,CH} = 5.6 Hz, N*H*CH₂), 5.51 (d, 1 H, *J*_{1,2} = 4.0 Hz, H-1), 5.42 (bt, 1 H, *J*_{3,4} = 2.6 Hz, *J*_{4,5} = 2.2 Hz, H-4), 5.28 (dd, 1 H, *J*_{2,3} = 10.7 Hz, H-3), 5.13 (dd, 1 H, H-2), 4.61 (m, 1 H, OCH₂C*H*) , 4.46 (t, 1 H, *J*_{5,6a} = *J*_{5,6b} = 8.8 Hz, H-6a), 4.29 (m, 1 H, H-5), 4.02 (dd, 1 H, *J*_{5,6b} = 6.0 Hz, H-6b), 2.00 (d, 2 H, ²*J*_{H,H} = 6.6 Hz, OC*H₂*CH), 3.49 (m, 2 H, COCH₂), 3.25 (m, 2 H, N*H*CH₂), 2.19, 2.12, 2.01 (3 s, 9 H, CH₃CO), 1.64-0.89 (m, 48 H, CH₂, CH₂C*H₃*). ¹³C NMR (75.5 MHz, CDCl₃): δ = 173.4, 170.2, 169.8, 169.1 (CO), 156.1 (CO carbamate), 80.1 (C-1), 68.8 (C-4), 67.8 (C-3, OC*H₂*CH), 67.2 (C-2), 63.1 (C-6), 52.3 (OCH₂*C*H), 50.6 (C-5), 49.1 (CO*C*H₂), 39.8 (NH*C*H₂), 36.5-14.1 (*C*H₃CO, CH₂, CH₂C*H₃*). ESIMS: *m*/*z* = 790.48 [M + Na]⁺. HRMS (ESI) calcd for [C₄₀H₆₉O₁₁N₃Na]⁺ 790.4824; found 790.4816.

### EXAMPLE 27. Synthesis of compound 27.

Compound **27** was obtained by treatment of a solution of **26** in MeOH (2.6 mL) with 1 M NaOMe in MeOH (42 µL), followed by neutralization with Amberlite^{®} IR120 hydrogen form, filtration, evaporation and freeze drying. Yield: 33 mg (quant.). *R_{f}* 0.6 (70:10:1 DCM-MeOH-H₂O). [α]_{D} +15.61 (c 1.0 in DCM-MeOH). ¹H NMR (500 MHz, 1:1 MeOD-CDCl₃): δ = 5.13 (d, 1 H, *J*_{1,2} = 4.2 Hz, H-1), 4.55 (dd, 1 H, *J*_{4,5} = 7.5 Hz, *J*_{4,5} = 5.9 Hz, H-4), 4.43 (m, 1 H, OC*H₂*CH), 3.97 (m, 2 H, OC*H₂*CH), 3.82 (m, 2 H, H-2, H-6a), 3.73 (m, 2 H, H-3, H-6b), 3.65 (m, 1 H, H-5), 3.19 (m, 2 H, NHC*H₂*), 2.24 (m, 2 H, COCH₂), 1.87-0.88 (m, 48 H, CH₂, CH₂C*H₃*). ¹³C NMR (125.7 MHz, 1:1 CD₃OD-CDCl₃): δ = 174.6, 170.3 (CO ester), 157.7 (CO carbamate), 82.5 (C-1), 70.0 (C-2), 68.7 (C-3), 67.8 (C-6), 67.7(C-5), 63.7 (O*C*H₂CH), 52.8 (C-4), 52.6 (OCH₂*C*H), 39.5 (NH*C*H₂), 35.8 (CO*C*H₂), 31.7-13.5 (CH₂, CH₂C*H₃*). ESIMS: *m*/*z =* 664.45 [M + Na]⁺. HRMS (ESI) calcd for [C₃₄H₆₃O₈N₃Na]⁺ 664.4507; found 664.4503.

### EXAMPLE 28. Synthesis of compound 28.

To a solution of **33** (20 mg, 0.05 mmol) and **40** (0.10 mmol), BF₃·OEt₂ (32 µL, 0.25 mmol) was added under Ar atmosphere at 0 °C. After 1 h the mixture was diluted with DCM (5 mL), washed with saturated NaHCO₃ (3 x 10 mL), dried with MgSO₄, filtered and concentrated. The resulting residue was purified by column chromatography (1:1 EtOAc-cyclohexane) to afford the target compound **28.** Yield: 27 mg (70%). *R_{f}* 0.40 (1:1 EtOAc-cyclohexane). [α]_{D} +31.24 (c 1.0 in DCM). ¹H NMR (500 MHz, CDCl₃): δ = 6.39 (m, 2 H, NH), 5.86 (d, 1 H, *J*_{1,2} = 3.9 Hz, H-1), 5.35 (bs, 1 H, H-4), 5.08 (bd, 2 H, H-2, H-3), 4.60 (dd, 1 H, *J*_{5,6a} = *J*_{5,6b} = 8.6 Hz, H-6a), 4.57 (m, 1 H, H-5), 4.42 (m, 1 H, CH), 3.98 (dd, 1 H, *J*_{5,6b} = 5.1 Hz, H-6b), 3.12 (m, 2 H, NHC*H₂*), 2.95 (dd, 1 H, *J*_{H,H} = 15.0 Hz, *J*_{H,H} = 1.8 Hz, SCH₂), 2.68 (dd, 1 H, *J*_{H,H} = 15.0 Hz, *J*_{H,H} = 10.4 Hz, SCH₂), 2.18 (m, 2 H, CH₂CO), 2.10, 2.02, 1.93 (3 s, 9 H, CH₃CO), 1.58-0.81 (m, 48 H, CH₂, CH₂C*H₃*). ¹³C NMR (125.7 MHz, CDCl₃): δ = 173.8, 170.1, 169.9, 169.7 (CO ester), 157.0 (CO carbamate), 68.5 (C-1), 67.4 (C-4), 67.2 (C-3), 63.5 (C-2), 60.6 (C-6), 52.9 (CH), 50.3 (C-5), 39.7 (NH*C*H₂), 36.7-14.1 (*C*H₃CO, CH₂, CH₂C*H₃*). HRMS (ESI) calcd for [C₄₀H₆₉O₁₀N₃SNa]⁺ 806.4596; found 806.4590.

### EXAMPLE 29. Synthesis of compound 29.

Compound **29** was obtained by treatment of a solution of **28** in MeOH (2.6 mL) with 1 M NaOMe in MeOH (42 µL), followed by neutralization with Amberlite^{®} IR120 hydrogen form, filtration, evaporation and freeze drying. Yield: 22 mg (quant.). *R_{f}* 0.6 (70:10:1 DCM-MeOH-H₂O). [α]_{D} +20.12 (c 1.0 in DCM:MeOH). ¹H NMR (500 MHz, 5:1 CD₃OD-CDCl₃): δ = 5.30 (d, 1 H, *J*_{1,2} = 5.8 Hz, H-1), 4.39 (m, 3 H, H-6a, H-4, H-2), 4.08 (m, 1 H, CH), 3.98 (m, 1 H, *J*_{6a,6b} = 9.6 Hz, *J*_{5,6b} = 5.7 Hz, H-6b), 3.75 (bt, 1 H, H-3), 3.54 (dd, 1 H, *J*_{5,6} = 10.0 Hz, *J*_{4,5} = 2.6 Hz H-5), 3.09 (m, 2 H, N*H*CH₂), 2.84, 2.75 (dd, 2 H, SCH₂), 2.16 (t, 2 H, COCH₂), 1.53-0.79 (m, 48 H, CH₂, CH₂C*H₃*). ¹³C NMR (125.7 MHz, 5:1 CD₃OD-CDCl₃): δ = 174.7, 170.7 (CO), 157.8 (CO carbamate), 70.7 (C-1), 68.4 (C-2), 67.1 (C-3), 63.6 (C-5), 62.8 (C-6), 53.2 (CH), 52.7 (C-4), 39.4 (NH*C*H₂), 35.9 (CO*C*H₂), 31.7-13.5 (CH₂, CH₂C*H₃*). ESIMS: *m*/*z* = 658.34 [M + H]⁺. Anal. calcd. for C₃₄H₆₃N₃O₇S: C 62.07, H 9.65, N 6.39, S 4.87. Found C 61.88, H 9.61, N 6.19, S 4.65.

### EXAMPLE 30. Synthesis of compound 30.

The synthesis of compound **30** comprised a first conjugation between glycosyl donor **46** and acceptor **36** to give intermediate **51.**

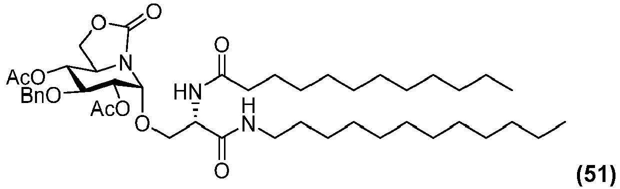

To a solution of **46** (200 mg, 0.47 mmol) and **36** (0.71 mmol), BF₃·OEt₂ (286 µL, 2.37 mmol) was added under Ar atmosphere at 0 °C. After 1 h the mixture was diluted with DCM (10 mL), washed with saturated NaHCO₃ (3 x 10 mL), dried with MgSO₄, filtered and concentrated. The resulting residue was purified by column chromatography using 1:2 EtOAc-cyclohexane as eluent. Yield: 250 mg (65%). *R_{f}* 0.60 (1:1 EtOAc-cyclohexane) to afford **51**. [α]_{D} +30.53 (c 1.0 in DCM). ¹H NMR (500 MHz, CDCl₃): δ = 7.60-7.13 (m, 12 H, CH-arom), 6.20 (t, 1 H, *J*_{NH,CH2} = 5.6 Hz, N*H*CH₂), 5.73 (d. 1 H, *J*_{NH,CH} = 5.8 Hz, NHFmoc), 4.80 (m, 2 H, H-1, H-3), 4.62 (d, 1 H, ²*J*_{H,H} = 11.5 Hz, O*CH₂*Ph), 4.52 (d, 1 H, ²*J*_{H,H} = 11.8 Hz, O*CH₂*Ph), 4.33 (dd, 3 H, *J*_{6a,6b} = *J*_{5,6a} = 8.2 Hz, H-6a, OCH₂, C*H₂*Fmoc), 4.20 (t, 1 H, *J*_{3,4} = *J*_{4,5}= 8.6 Hz, H-4), 4.15 □bt, 1 H, H-2), 3.85 (m, 2 H, H-6b, CH), 3.40 (m, 1 H, H-5), 3.16 (m, 2 H, NHC*H₂*), 1.97, 1.85 (2 s, 6 H, CH₃CO), 1.63-0.80 (m, 25 H, CH₂, CH₂C*H₃*). ¹³C NMR (125.7 MHz, CDCl₃): δ = 170.0, 169.8, 168.7 (CO), 156.9 (CO carbamate), 143.7-120.0 (C-arom), 79.9 (C-1), 76.8 (O*C*H₂CH), 75.5 (C-4), 74.1 (C-2), 72.4 (C-3), 69.8 (C-6), 67.4 (*C*H₂Fmoc), 52.3 (OCH₂*C*H), 49.4 (CHFmoc), 47.1 (C-5), 8 39.8 (NHCH₂), 33.7-14.1 (*C*H₃CO, CH₂, CH₂C*H₃*). HRMS (ESI) calcd for [C₄₈H₆₁O₁₁N₃Na]⁺ 878.4198; found 878.4191.

In a subsequent step, to a solution of **51** (418 mg, 0.488 mmol) in THF (4.8 mL), piperidine (964 µL, 20 % volume) was added and the reaction was stirred for 15 min. After that, the solvent was evaporated and coevaporated with MeOH to remove piperidine traces. The crude product was dissolved in DCM (4.8 mL) and dodecanoyl chloride (170 µL, 0.732 mmol) and Et₃N (137 µL, 0.976 mmol) were added. The mixture was stirred for 16 h and concentrated. The resulting residue was purified by column chromatography 1:1 EtOAc-cyclohexane to afford the target compound **30.** Yield: 200 mg (50 %, two steps). *R_{f}* 0.50 (1:1 EtOAc- cyclohexane). [α]_{D} +22.40 (c 1.0 in DCM). ¹H NMR (500 MHz, CDCl₃): δ = 7.27-7.15 (m, 5 H, CH-arom), 6.49 (d, 1 H, *J*_{NH,CH} = 8.0 Hz, NHCH), 6.33 (t, 1 H, *J*_{NH,CH} = 5.7 Hz, N*H*CH₂), 5.34 (d, 1 H, *J*_{1,2} = 3.9 Hz, H-1), 4.82 (t, 1 H, *J*_{4,5} = 9.5 Hz, H-4), 4.80 (dd, 1 H, *J*_{2,3} = 10.0 Hz, H-2), 4.65 (d, 1 H, ²*J*_{H,H} = 11.6 Hz, O*CH₂*Ph), 4.57 (d, 1 H, ²*J*_{H,H} = 11.7 Hz, O*CH₂*Ph), 4.53 (m, 1 H, OC*H₂*CH), 4.37 (t, 1 H, *J*_{5,6a} = *J*_{6a,6b} = 8.9 Hz, H-6a), 4.21 (bdd, 1 H, H-6b), 3.86 (m, 1 H, H-3), 3.71 (m, 1 H, H-5), 3.41 (m, 2 H, OC*H₂*CH), 3.15 (m, 2 H, NHC*H₂*), 2.16 (t, 2 H, COCH₂), 2.00, 1.90 (2 s, 6 H, CH₃CO), 1.64-0.80 (m, 48 H, CH₂, CH₂C*H₃*). ¹³C NMR (125.7 MHz, CDCl₃): δ = 174.3, 170.8, 170.5, 169.9 (CO), 157.2 (CO carbamate), 138.5-128.1 (C-arom), 80.5 (C-1), 76.2 (C-4), 74.9 (C-2), 73.2 (C-3), 69.9 (O*C*H₂CH), 68.2 (C-6), 53.5 (O*C*H₂CH), 53.0 (C-5), 40.4 (NH*C*H₂), 37.2 (CO*C*H₂), 34.6-14.8 (*C*H₃CO, CH₂, CH₂C*H₃*). HRMS (ESI) calcd for [C₄₅H₇₃O₁₀N₃Na]⁺ 838.5188; found 838.5181.

### EXAMPLE 31. Synthesis of compound 31.

Compound **31** was obtained by treatment of a solution of **30** in MeOH (10 mL) with 1 M NaOMe in MeOH (155 µL), followed by neutralization with Amberlite^{®} IR120 hydrogen form, filtration, evaporation and freeze drying Column chromatography 2:1 EtOAc-cyclohexane. Yield: 130 mg (quant.). *R_{f}* 0.40 (2:1 EtOAc-cyclohexane). [α]_{D} +21.90 (c 1.0 in DCM). ¹H NMR (500 MHz, CDCl₃): δ = 7.34-7.16 (m, 5 H, CH-arom), 4.98 □d, 1 H, *J*_{1,2} = 3.7 Hz, H-1), 4.78 (dd, 1 H, ²*J*_{H,H} = 11.0 Hz, O*CH₂*Ph), 4.48 (dd, 1 H, *J*_{3,4} = 7.0 Hz, *J*_{4,5} = 5.9 Hz, H-4), 4.45 (t, 1 H, *J*_{6a,6b} = *J*_{5,6a} = 8.8 Hz, H-6a H-6a), 4.13 (dd, 1 H, *J*_{5,6b} = 6.1 Hz, H-6b), 3.66 (m, 1 H, H-5), 3.60 (m, 2 H, O*CH₂*CH), 3.51 (dd, 1 H, *J*_{2,3} = 9.3 Hz, H-2), 3.47 (bt, 1 H, H-3), 3.35 (m, 1 H, OCH₂C*H*), 3.10 (m, 2 H, NHC*H*₂), 2.16 (t, 2 H, COCH₂), 1.78-0.79 (m, 48 H, CH₂, CH₂C*H*₃). ¹³C NMR (125.7 MHz, CDCl₃): δ = 174.8, 170.4, 157.1 (CO carbamate), 138.6-127.3 (C-arom), 82.5 (C-1), 81.7 (C-3), 75.4 (O*C*H₂CH), 74.1 (C-2), 71.7 (C-5), 67.7 (C-6), 67.2 (C-4), 53.5 (OCH₂*C*H), 52.7 (CH₂Ph), 39.4 (NH*C*H₂), 35.6 (CO*C*H₂), 33.4-13.3 (*C*H₃CO, CH₂, CH₂*C*H₃). HRMS (ESI) calcd for [C₄₁H₆₉O₈N₃Na]⁺ 754.4977; found 754.4970.

### EXAMPLE 32. Stability of the glycolipid mimetics of the invention to the action of glcosidases

The stability of the glyosidic linkage in compounds **2, 4, 6, 8, 10, 12, 14, 16, 18, 20, 22, 23, 25, 27** and **29,** selected as representative examples, upon incubation with human fibroblast lysates for 4 h was checked. For such purpose, cells were scraped into ice-cold H₂O (10⁶ cells mL⁻¹) and lysed by sonication. Insoluble materials were removed by centrifugation at 15,000 rpm for 5 min and protein concentrations were determined with Protein Assay Rapid Kit (WAKO, Tokyo, Japan). 10 µL of the lysates in 0.1% Triton X-100 in distilled water were incubated at 37 °C with 20 µL of the compound solution (200 µM) in 0.1 M citrate buffer, pH 4.5. In control experiments, the activity of several lysosomas glycosidases was confirmed using fluorescent substrates. The substrates were 4-methylumbelliferone (4-MU)-conjugated β-D-glucopyranoside (for GCase), α-D-glucopyranoside (for α-glucosidase), α-D-galactopyranoside (for α-Galase) and β-D-galactopyranoside (for β-galactosidase). The liberated 4-methylumbelliferone was measured in the black-well plate with a Perkin Elmer Luminescence Spectrometer (excitation wavelength: 340 nm; emission: 460 nm). No traces of the free polysubstituted 2-oxa-3-oxoindolizidine, which would result from hydrolysis of the glycolipid mimetics was detected by gas chromatography upon trimethylsilylation, confirming the stability of the compounds to the action of the glycosidases

### EXAMPLE 33. Suppression of LPS-induced inflammation (Splenocytes in vitro).

Splenocytes were treated concurrently with the compounds of the invention and LPS to assess the suppressive function of compounds on LPS-induced inflammation. Selected results are presented in **Figure 1****.** Several of the compounds, particularly compounds **8, 10, 12, 14, 18, 20** in **Table 1,** showed prominent inflammation-suppressing function.

### EXAMPLE 34. Suppression on LPS-induced inflammation (BMDM in vitro).

Since macrophage is the major responder to LPS-induced inflammation, we assessed the capability of selected compounds to suppress LPS-induced inflammation in bone marrow-derived macrophages (BMDM). Data shown in **Figure 2** demonstrate that compound **12** is particularly efficient in suppressing LPS-induced inflammation.

### EXAMPLE 35. Suppression of LPS-induced inflammation in vivo.

To determine the anti-inflammatory function *in vivo,* we assessed the capability of compound **12** in suppressing LPS-induced inflammation in B6 mice. In **Figure 3****,** the level of IL-6 and TNFα in sera was shown to be significantly suppressed when injected twice in a half hour time after LPS-treatment, confirming that compound **12** is efficient in suppressing LPS-induced inflammation.

### EXAMPLE 36. ERK and p38 of the MAPK family and STAT3 are suppressed by the new compounds.

Using the multiplex bead array, we examined how the new compounds might exert their suppressive effect in term of signal transduction. In **Figure 4****,** as a representative example, we found that phosphorylation of ERK and p38 of the MAPK family, but not JNK, was significantly reduced by compound **12** comparing to LPS treatment alone. Hence, the new compounds may exercise their anti-inflammation function through suppressing phosphorylation of ERK and p38. We also observed the significant reduction of phosphorylation in STAT3.

### EXAMPLE 37. Suppression of aGalCer-induced inflammation (Splenocytes in vitro).

Splenocytes were treated concurrently with the compounds of the invention and aGalCer to assess their ability to suppress aGalCer-induced inflammation. Selected results are presented in **Figure 5****.** Several of the compounds, particularly compounds **2, 6,** and **12** in **Table 1,** showed prominent inflammation-suppressing function.

### EXAMPLE 38. Immune-stimulating function of compounds (Splenocytes in vitro).

Splenocytes were treated with the compounds of the invention alone to assess the immune-stimulating function of the compounds by measuring their ability to promote the secretions of IFN-γ and IL-4. In **Figure 6****,** as a representative example, we found that compounds **12, 18, 22, 27,** and **29** were effective in promoting the productions of IFNγ, but not IL-4, supporting those compounds **12, 18, 22, 27,** and **29** are Th1-biased. Conversely, as another representative example, compound **8** was also proficient at inducing IL-4 secretion, but not IFNγ, supporting that compound 8 is Th2-biased.

### EXAMPLE 39. Suppression of OVA-induced airway hyperreactivity (AHR) and inflammation in vivo.

Using the Th2 OVA-induced AHR mouse model, we assessed the capability of selected compounds **8, 12, 18,** and **27** in suppressing Th2 inflammation. We found that compounds **8, 12,** and **18** can suppress AHR onset **(****Figure 7****).** Moreover, we observed that compound **12** could significantly suppress the increase of the major instigators of AHR, IL-13 and IL-4, in BALF **(****Figure 8****)** and in lungs (protein in **Figure 9** and mRNA in **Figure 10****).**

### EXAMPLE 40. Evaluating the adjuvancy function of compounds in vivo.

Besides assessing the ability of the new compounds to suppress inflammation, we also looked into their function to act as adjuvants, i.e. their ability to promote desirable immunological trends in the development of vaccines. We measured the *in vivo* adjuvancy capability of selected compounds **8, 12, 18, 27,** and **29** in inducing IFNγ and IL-4 in B6 mice **(****Figure 11****).** Compounds **8, 12, 27,** and **29** showed significant capability in inducing both IFNγ and IL-4 2 hours after injection and still having a significant detectable level of both cytokines at 18 hours after injection. Compound 18 only showed significant serum IFNγ level at both time points. This result strongly supports the usage of the compounds of the invention as the adjuvant in vaccine formulation.

### EXAMPLE 41. Compound 12 promotes DC maturation in vivo.

The capability of selected compounds **8, 12, 18, 27,** and **29** in promoting DC maturation *in vivo was* assessed by the mRNA level of DC-maturation marker CD86 in the spleen of mice injected with the compounds of the invention. The injection of compound **12** into mice was shown to induce a significant increase of CD86 mRNA expression in the spleens of mice **(****Figure 12****).** This result strongly supports the usage of compound **12** in vaccine formulation, as DC maturation is essential to the development of a robust immune response.

### EXAMPLE 42. Compound 12 promotes Th1 immune response in vivo.

Compounds **12** and **27** were injected into OT-1 mice (transgenic mice that express a TCR that is specific for an ovalbumin OVA peptide) to validate the Th1-biased immunomodulation property. In **Figure 13****,** compound **12** was observed to induce significant CD8⁺ T cell proliferation, indicating compound **12** can promote Th1 immune response.

### EXAMPLE 43. Compound 12 adjuvancy is MyD88-dependent and Th1-biased.

To determine whether the adjuvancy function of the representative compound **12** is MyD88-dependent or not, we performed experiments with immune cells isolated from MyD88 knocked-out (KO) mice. Foremost, we found that the ability of compound **12** to induce IFNγ in splenocytes isolated from MyD88 KO mice was completely abrogated **(****Figure 14****).** The result also confirmed the Th1-biased nature of compound **12,** as no IL-4 induction was observed. Secondly, we found that the ability of bone marrow-derived dendritic cells (BMDC) from MyD88 KO mice to produce IL-12p40 in response to compound **12** was severely curtailed as compared with BMDC from wildtype mice **(****Figure 15****).** Data are dose-dependent and differences become more pronounced at the higher concentrations. Lastly, we found that the maturation of BMDC induced by compound **12** treatment was impeded in BMDC from MyD88 deficient mice **(****Figure 16****).** Taken together, the results support that compound **12** adjuvancy function is MyD88-dependent.

## Claims

1. A compound of formula I: 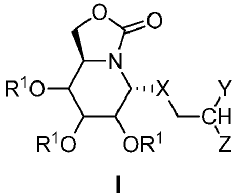 wherein:
each R¹ is independently -H, -CH₂Ph (Bn), -COPh, -CO-C₁-C₁₂ alkyl, C₁-C₁₂ alkyl,
wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is O, S, NH or CH₂;
Y is a group NH-C(=O)-Y', NH-C(=O)-NHY', NH-C(=S)-NHY', NH-SO₂-Y'or a group of formula **la:** 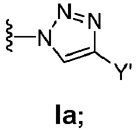
Y' is C₁-C₂₅ alkyl optionally substituted by one or more groups R⁴;
R⁴ is -OH, -OCH₂Ph (-OBn), -OCH₂Ph (-OBz), C₁-C₁₂ alkyl, -O-C₁-C₁₂ alkyl, -S-C₁-C₁₂ alkyl, -N(R³)₂, -N₃, halogen or Cy₁;
C_{y1} is a 5-membered to 14-membered ring which can be saturated, partially unsaturated or aromatic, and which optionally contains from 1 to 4 identical or different heteroatoms in total selected from N, O, S or P, wherein C_{y1} can be optionally fused to a 5- or 6-membered carbocycle or heterocycle which can be saturated, partially unsaturated or aromatic, and wherein C_{y1} is optionally substituted by one or more groups selected from phenyl, halogen, C₁-C₄ alkyl, halo-C₁-C₄ alkyl, -O-C₁-C₄ alkyl,-OCOO-C₁-C₄ alkyl, -CO-C₁-C₄ alkyl, S- C₁-C₄ alky or N₃-C₁-C₄ alkyl;
Z is -CO-O-C₄-C₂₅ alkyl, -CO-NH-C₄-C₂₅ alkyl or C₄-C₂₅ alkyl, wherein Z is optionally substituted by one or more groups R⁵; and
each R⁵ independently is -OH, -OC(=O)Ph (-OBz), C₁-C₁₂ alkyl, -O-C₁-C₁₂ alkyl, -S-C₁-C₁₂ alkyl, -N(R³)₂, -N₃, halogen or Cy₁.

2. The compound of formula **I** according to claim 1, wherein R¹ is independently selected from -H, -CH₂Ph (Bn), -CO-C₁-C₁₂ alkyl, wherein each R¹ is independently optionally substituted by one or more group R².

3. The compound of formula **I** according to any of claims 1 or 2, wherein X is O or S.

4. The compound of formula **I** according to any of claims 1 to 3, wherein Y is a group NH-C(=O)-Y', NH-C(=S)-NHY', NH-SO₂-Y'or a group of formula **la**.

5. The compound of formula I according to any of claims 1 to 4, wherein:
Y is a group NH-C(=O)-Y' or NH-C(=S)-NHY', NH-SO₂-Y';
Y' is C₁-C₂₅ alkyl optionally substituted by one or more groups R⁴;
R⁴ is -OH, -OCH₂Ph (-OBn), C₁-C₁₂ alkyl, -O-C₁-C₁₂ alkyl, -S-C₁-C₁₂ alkyl, -N(R³)₂,N₃, halogen or Cy₁.

6. The compound of formula I according to any of claims 1 to 4, wherein:
Y is a group of formula **la;** and
Y' is C₁-C₂₅ alkyl optionally substituted by one or more groups R⁴,

7. The compound of formula I according to any of claims 1 to 5, wherein Z is -CO-NH-C8-C20 lineal alkyl.

8. The compound of formula I according to any of claims 1 to 4 and 6, wherein:
Z is C₄-C₂₅ alkyl, wherein Z is substituted by two groups R⁵; and
each R⁵ independently is -OH or -OC(=O)Ph (-OBz).

9. The compound of formula I according to claim 1, selected from:
**1** 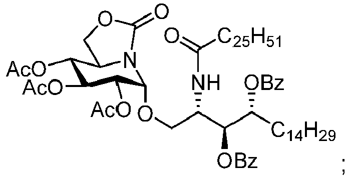
2 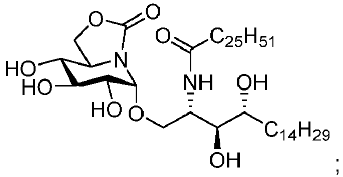
3 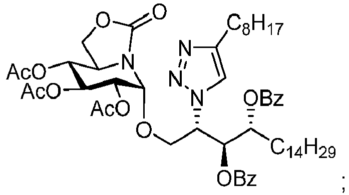
4 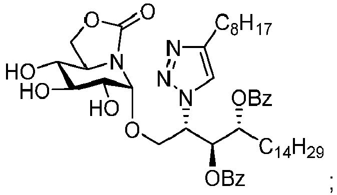
5 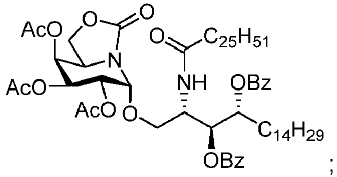
6 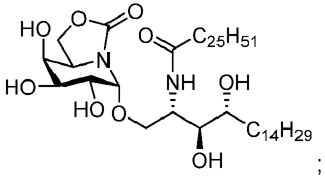
7 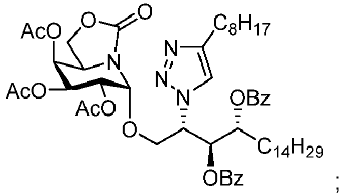
8 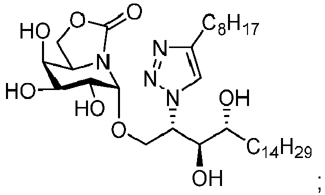
9 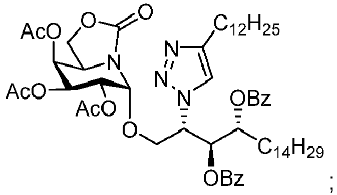
10 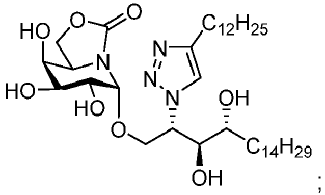
11 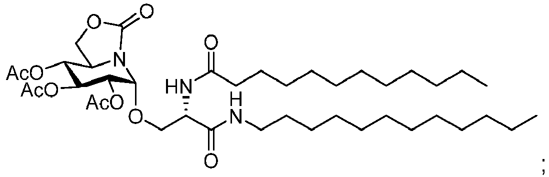
12 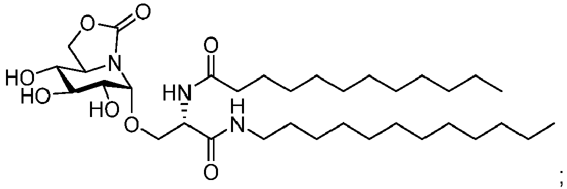
13 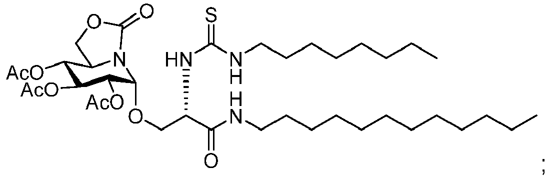
14 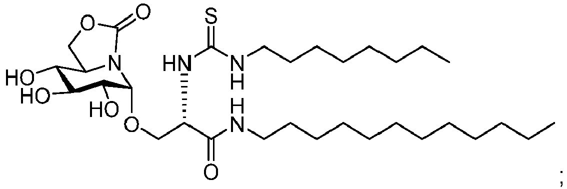
15 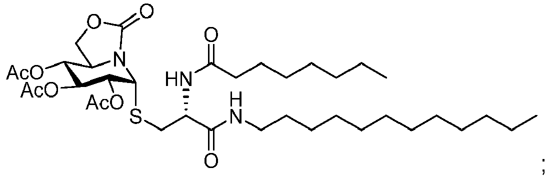
16 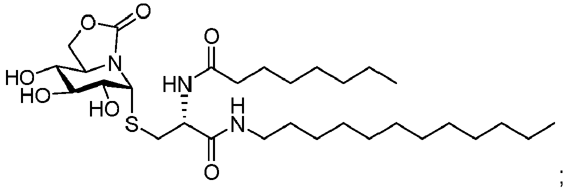
17 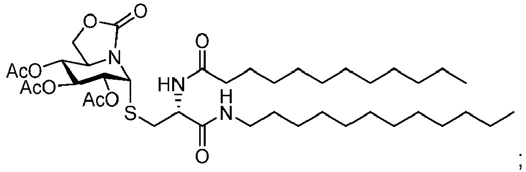
18 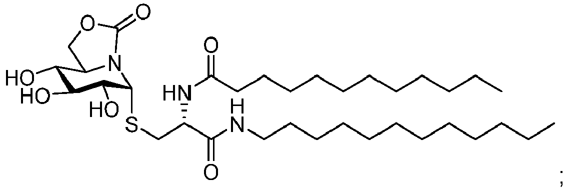
19 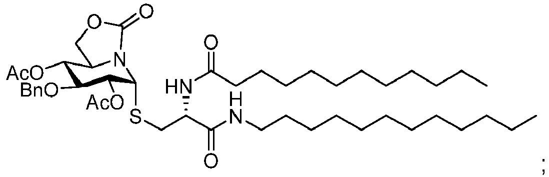
20 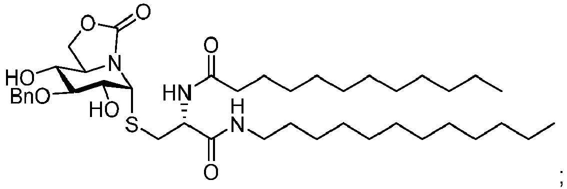
21 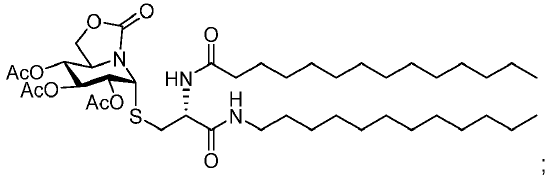
22 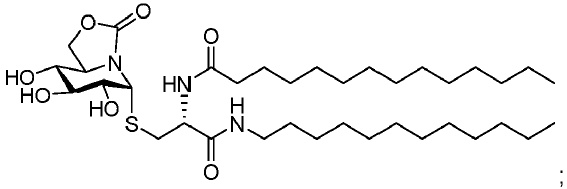
**23** 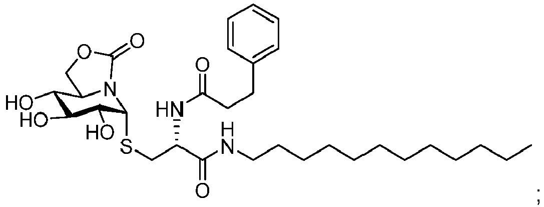
**24** 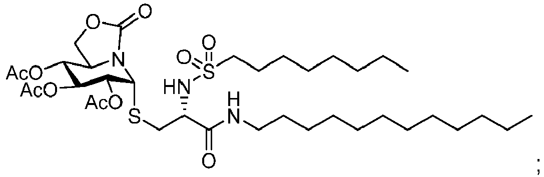
**25** 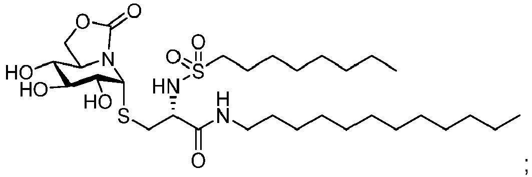
**26** 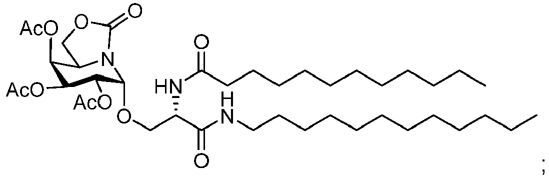
**27** 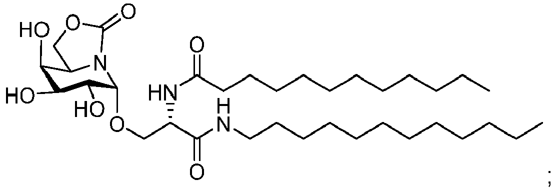
**28** 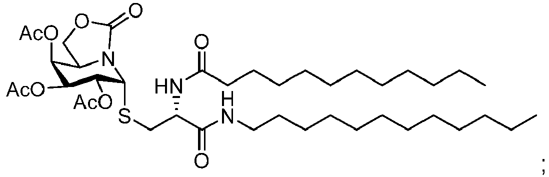
**29** 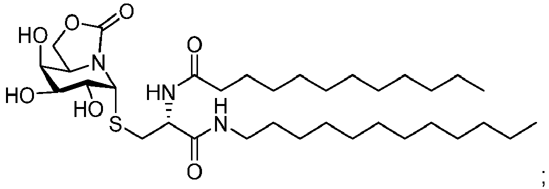
**30** 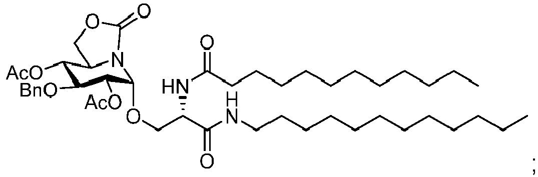 and
**31**

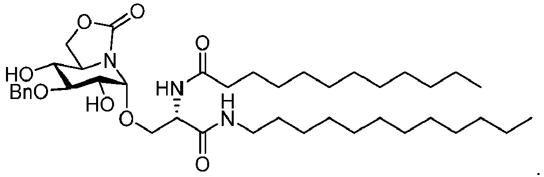

10. A pharmaceutical composition which comprises at least one compound of formula I according to any of claims 1 to 9 or any mixtures or combinations thereof.

11. A compound of formula **I** according to any of claims 1 to 9 or a pharmaceutical composition thereof, for use as a medicament.

12. A compound of formula I according to any of claims 1 to 9 or a pharmaceutical composition thereof, for use in the treatment and/or prevention of an immune disease cursed by Th1/Th2 imbalance.

13. The compound of formula I for the use according to claim 12, for the treatment and/or prevention of a disease selected from metabolic syndrome, non-alcoholic fatty liver disease (NAFLD), non-alcoholic steatohepatitis (NASH), immune mediated hepatitis, an autoimmune disease, graft rejection pathology, atherosclerosis, airway hyperactivity, asthma, allergic rhinitis and a neurodegenerative disorder.

14. A vaccine which comprises an effective amount of a compound of formula I according to any of claims 1 to 9 and a vaccine agent.

15. The vaccine according to claim 14, wherein the vaccine agent is selected from a killed microorganism, a live attenuated microorganism, a toxoid and a fragment of an inactivated or attenuated microorganism, a synthetic peptide or glycopeptide construct, a ribonucleic acid (RNA)-based vaccine or a deoxyribonucleic acid (DNA)-based vaccine.
